# EUROPEAN PATENT APPLICATION

(11) **EP 1 692 939 A1**
(43) Date of publication of application: **23.08.2006**
(21) Application number: 05003623.5
(22) Date of filing: 19.02.2005
(51) Int. Cl.: A01N 43/40, A01N 43/42, A01N 43/54, A01N 43/58, A01N 43/60, A01N 43/90

(54) **Pesticidal substituted piperidines**

(71) Applicant: Bayer CropScience S.A., 69009 Lyon (FR)
(72) Inventor: The designation of the inventor has not yet been filed
(74) Representative: Lutze, Oliver

(57) **Abstract**

The invention relates to the use of piperidine derivatives encompassed from the general formula (I) for the control of pests, including arthropods and helminths, and a method for the control of pests.

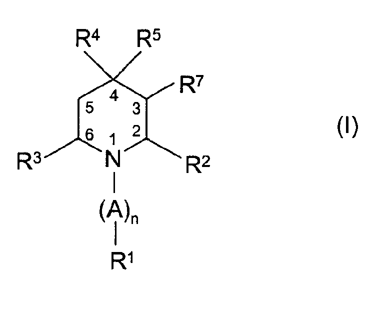

## Description

The invention relates to the use of piperidine derivatives for the control of pests, including arthropods and helminths, and a method for the control of pests.

Some of the concerned piperidine derivatives are known as synthesis intermediates from
Faul, Margaret M.; Kobierski, Michael E.; Kopach, Michael E., Journal of Organic Chemistry (2003), 68(14), 5739-5741. CODEN: JOCEAH ISSN: 0022-3263; Gooding, Owen W.; Beard, Colin C.; HTCYAM; Heterocycles; EN; 32; 9; 1991; 1777-1780;
Massa, S.; Mai, A.; Artico, M, Synthetic Communications (1990), 20(22), 3537-45;

Some piperidine derivatives are known synthesis intermediates for the preparation of herbicidal isonipecotin acid derivatives (DE 25 10 831) or for tert.-amino-substituted 1,5-imino-cycloalkanes (US 2,836,598, US 2,845,427). In addition several substituted piperidines are already known as compounds with pharmacological properties from CH 469 736, WO 04/026873 and from DE 29 19 553.

The control of insects, arachnids and helminths with some substituted piperidine compounds has been described in WO 9637494 A1 (3-aryl-3-cyano-8-azabicyclo[3.2.1]octanes), GB 2319524 A1 (nortropane derivatives), DE 10 2004 010 086 A1 (substituted 4-methylene-piperidine derivates). However, the described pesticidal substituted piperidines are not structurally related to the piperidine derivatives described as pesticides in the present application.

Since modern pesticides must meet a wide range of demands, for example regarding level, duration and spectrum of action, use spectrum, toxicity, combination with other active substances, combination with formulation auxiliaries or synthesis, and since the occurrence of resistances is possible, the development of such substances can never be regarded as concluded, and there is constantly a high demand for novel pesticidal compounds which are advantageous over the known compounds, at least as far as some aspects are concerned.

It is an object of the present invention to provide new pesticides which may be used as ectoparasiticides in stock animals or in domestic companion animals. Another object of the invention is to provide new pesticides which may be used in lower dose than existing pesticides. Another object of the invention is to provide new pesticides which do not have the same biochemical mode of action as known pesticides and are active against pests that have developed resistance against commercial pesticides. A further object of the invention is to provide new pesticides which are safer to the user and the environment. These objects are met in whole or in part by the present invention.

The present invention relates to the use of compounds which are substituted piperidine derivatives of formula (I) or a pesticidally acceptable salt thereof, for the control of pests: 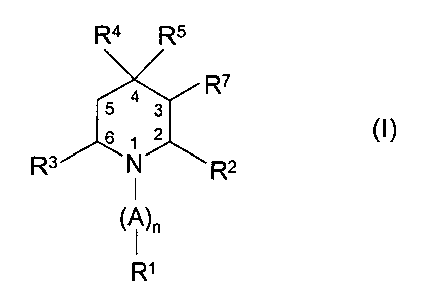 wherein:
- A: is a straight or branched (C₁-C₃)-alkylene or (C₁-C₃)-haloalkylene
- R¹: is (C₆-C₁₄)-aryl, unsubstituted or substituted
by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or is (C₁-C₆)-alkyl, (C₃-C₇)-cycloalkyl, (C₃-C₇)-cycloalkenyl unsubstituted or substituted by one or more radicals selected from the group consisting of halogen, (C₁-C₆)-alkoxy, (C₁-C₆)-haloalkoxy, CN, NO₂, S(O)ₚR¹², NR¹³R¹⁴, OH and oxo;
or a heterocyclyl or heteroaryl, unsubstituted or substituted
by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
- R² and R³: are each independently H or (C₁-C₃)-alkyl;
or wherein R² and R³ may form together a (C₁-C₆)-alkylene bridge ;
- R⁴ and R⁵: are each independently H, (C₁-C₆)-alkyl, (C₂-C₆)-alkenyl or (C₁-C₆)-alkoxy; wherein in case R⁴ and R⁵ are each independently (C₁-C₆)-alkyl, (C₂-C₆)-alkenyl or (C₁-C₆)-alkoxy both groups together may form a 4-7 membered ring with the carbon atom in position 4 (C-4) of the piperidine ring; and
wherein the residues may be unsubstituted or substituted by one or more radicals selected from the group consisting of halogen, (C₁₋C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴;
or in case R⁴ is H or (C₁-C₆)-alkyl, R⁵ may be also OH, OCOR⁸, OCOOR⁹, OCO-COO(C₁-C₄)-alkyl, COO(C₁-C₄)-alkyl , COOH, CH₂Phenyl,
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴;
or in case R⁵ is a binding electron pair, R⁴ may form together with R⁵ a residue X selected from the group consisting of O, S, N-OH, N-O-(C₁-C₆)-alkyl, N-O-CO-R⁸, N-O-COOR⁹
- R⁷: is H, (C₁-C₃)-alkyl, (C₂-C₄)-alkenyl ;
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or wherein the residues
- R⁷ and R⁵: and the carbon atoms in position 3 (C-3) and in position 4 (C-4) of the piperidin ring form a (C₃-C₇)-cycloalkyl, (C₃-C₇)-cycloalkenyl or a (C₆-C₁₄) -aryl residue,
either unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
- R⁸: is H, (C₂-C₆)-alkenyl, (C₂-C₆)-alkynyl, (C₃-C₇)-cycloalkyl or (C₁-C₆)-alkyl, unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴ ;
- R⁹: is H, (C₃-C₆)-alkenyl, (C₃-C₆)-alkynyl, (C₃-C₇)-cycloalkyl or (C₁-C₆)-alkyl, unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴
- R¹⁰: is (C₆-C₁₄)-aryl, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴
- R¹¹: is a saturated or unsaturated heteroaromatic or heterocyclyl radical, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
- R¹²: is (C₁-C₆)-alkyl or (C₁-C₆)-haloalkyl
- R¹³ and R¹⁴: are each independently H, (C₁-C₆)-alkyl, (C₁-C₆)-haloalkyl, (C₂-C₆)-alkenyl, (C₂-C₆)-alkynyl, (C₃-C₇)-cycloalkyl or (C₃-C₇)-cycloalkyl-(C₁-C₆)-alkyl; or a group wherein R¹³ and R¹⁴ together with the N form a 4 to 8-membered heteroaryl or heterocyclyl ring that may contain one or two further hetroatoms;
and wherein
- n: is 0 or 1 and
- p: is 0, 1 or 2.

The invention also encompasses any stereoisomer, enantiomer or geometric isomer, and mixtures thereof.

By the term "pesticidally acceptable salts" is meant salts the anions or cations of which are known and accepted in the art for the formation of salts for pesticidal use. Suitable salts with acids, e.g. formed by compounds of formula (I) containing a basic nitrogen in the piperidine ring, include salts with inorganic acids, for example hydrochlorides, sulphates, phosphates and nitrates and salts with organic acids for example acetic acid, methanesulfonic acid.

The term "pests" encompass in particular arthropod pests, including insects and arachnids, and helminths pests, including nematodes. An preferred embodiment of the present invention is to provide pesticides which may be used as ectoparasiticides in stock animals or in domestic companion animals.

In the present specification, including the accompanying claims, the aforementioned substituents have the following meanings:
"Halogen atom" means fluorine, chlorine, bromine or iodine.
   The term "halo" before the name of a radical means that this radical is partially or completely halogenated, that is to say, substituted by F, Cl, Br, or I, in any combination, preferably by F or Cl.
"Alkyl" -groups and portions thereof (unless otherwise defined) may be straight- or branched-chain.
   The expression "(C₁-C₆)-alkyl" is to be understood as meaning an unbranched or branched hydrocarbon radical having 1, 2, 3, 4, 5 or 6 carbon atoms, such as, for example a methyl, ethyl, propyl, isopropyl, 1-butyl, 2-butyl, 2-methylpropyl or tert.-butyl radical.
   Alkyl radicals and also in composite groups, unless otherwise defined, preferably have 1 to 4 carbon atoms.
"(C₁-C₃)-alkylene" is to be understood as meaning an unbranched or branched alkanediyl group having 1 to 3 carbon atoms, e.g. -CH₂-, -CH₂-CH₂-, -CH₂-CH₂-CH₂₋or -CH₂-CH(CH₃)-. The expression "(C₁-C₃)- haloalkene" means an (C₁-C₃)-alkylene group, in which one or more hydrogen atoms are replaced by the same number of identical or different halogen atoms.
"(C₁-C₆)-haloalkyl" means an alkyl group mentioned under the expression "(C₁-C₆)-alkyl" in which one or more hydrogen atoms are replaced by the same number of identical or different halogen atoms, such as monohaloalkyl, perhaloalkyl, CF₃, CHF₂, CH₂F, CHFCH₃, CF₃CH₂, CF₃CF₂, CHF₂CF₂, CH₂FCHCl, CH₂Cl, CCl₃, CHCl₂ or CH₂CH₂Cl.
"(C₁-C₆)-alkoxy" means an alkoxy group whose carbon chain has the meaning given under the expression "(C₁-C₆)-alkyl". "Haloalkoxy" is, for example, OCF₃, OCHF₂, OCH₂F, CF₃CF₂O, OCH₂CF₃ or OCH₂CH₂Cl.
"(C₂-C₆)-alkenyl" means an unbranched or branched non-cyclic carbon chain having a number of carbon atoms which corresponds to this stated range and which contains at least one double bond which can be located in any position of the respective unsaturated radical. "(C₂-C₆)-alkenyl" accordingly denotes, e.g. the vinyl, allyl, 2-methyl-2-propenyl, 2-butenyl, pentenyl, 2-methylpentenyl or the hexenyl group.
"(C₂-C₆)-alkynyl" means an unbranched or branched non-cyclic carbon chain having a number of carbon atoms which corresponds to this stated range and which contains one triple bond which can be located in any position of the respective unsaturated radical. "(C₂-C₆)-alkynyl" accordingly denotes, for example, the propargyl, 1-methyl-2-propynyl, 2-butynyl or 3-butynyl group.
"Cycloalkyl" groups preferably have from three to seven carbon atoms in the ring and
are optionally substituted by halogen or alkyl.
   The expression "(C₃-C₇)-cycloalkyl-(C₁-C₆)-alkyl" means a (C₁-C₆)-alkyl group which is substituted by a (C₃-C₇)-cycloalkyl ring.
   In compounds of formula (I) the following examples of radicals are provided:
   An example of alkyl substituted by cycloalkyl is cyclopropylmethyl; and
   an example of alkyl substituted by alkoxy is methoxymethyl (CH₂OCH₃);
"Aryl-(C₁-C₆)-alkyl" means a (C₁-C₆)-alkyl radical which is substituted by an aryl radical.
"Aryl" denotes a mono-, bi- or polycyclic aromatic system, for example phenyl, naphthyl, tetrahydronaphthyl, indenyl, indanyl, pentalenyl, fluorenyl and the like, preferably phenyl. Aryl groups may be unsubstituted or substituted by one or more radicals, preferably 1, 2 or 3 radicals.
A "heterocyclyl" radical preferably contains one or more, in particular 1, 2 or 3, hetero atoms in the heterocyclic ring, preferably selected from the group consisting of N, O, S and P (S atoms being optionally in the SO or SO₂ oxidation state); it is preferably an aliphatic heterocyclyl radical having 3 to 7 ring atoms. The saturated or unsaturated "heterocyclyl" radical can be, for example, oxiranyl, oxetanyl, oxolanyl (= tetrahydrofuryl), oxanyl, pyrrolidyl, piperidyl, piperazinyl, dioxolanyl, oxazolinyl, isoxazolinyl, oxazolidinyl, isoxazolidinyl or morpholinyl. The "heterocyclyl" radical may be unsubstituted or substituted, preferably by one or more radicals, most preferably by 1, 2 or 3 radicals.
A "heteroaryl" radical preferably contains one or more, in particular 1, 2 or 3, hetero atoms in the heteroaromatic ring, preferably selected from the group consisting of N, O and S; it is preferably a heteroaromatic radical having 5 to 7 ring atoms. The heteroaromatic ring can be for example, a mono-, bi- or polycyclic aromatic system in which at least 1 ring contains one or more hetero atoms, for example pyridyl, pyrimidinyl, pyridazinyl, pyrazinyl, triazinyl, thienyl, thiazolyl, thiadiazolyl, oxazolyl, isoxazolyl, furyl, pyrrolyl, pyrazolyl, imidazolyl and triazolyl. The "heteroaryl" radical may be unsubstituted or substituted, preferably by one or more radicals, most preferably by 1, 2 or 3 radicals.

The present invention provides the use of preferred compounds which are piperidin derivatives of formula (Ia), (Ib) and (Ic): 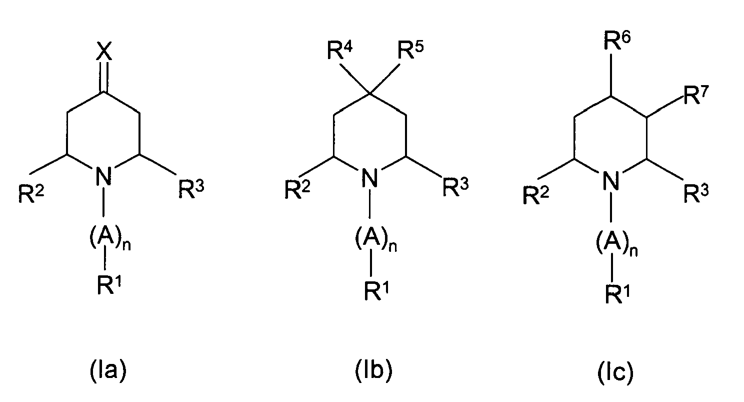 wherein:
- A: is an unbranched or branched (C₁-C₃)-alkylene and/or
- R¹: is phenyl, unsubstituted or substituted
by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or is (C₁-C₆)-alkyl, (C₁-C₆)-haloalkyl, (C₃-C₇)-cycloalkyl, (C₃-C₇)-cycloalkylenyl;
or is heteroaryl e.g. pyridine, pyrimidine, pyrazine and pyridazine, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
and/or
- R², R³: are each independently H, (C₁-C₃)-alkyl; wherein R² and R³ may form together a (C₁-C₃)-alkylene bridge, in particular a -C₂H₄- group so that a tropan ring system is generated;
and/or in formula (Ib)
- R⁴, R⁵: are each independently H, (C₁-C₆)-alkyl, (C₁-C₆)-alkoxy; or in case R⁴ and R⁵ are (C₁-C₆)-alkyl or (C₁-C₆)-alkoxy both groups together with C-4 of the piperidine ring may form a 4-7 membered ring;
and/or in formula (Ic)
- R⁶: is OH, OCOR⁸, OCOOR⁹, OCO-COO(C₁-C₄)-alkyl, COO(C₁-C₄)-alkyl , COOH or CH₂Phenyl, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴
and/or
- R⁷: is H, (C₁-C₃)-alkyl,
or wherein
- R⁷ and R⁶: and the carbon atoms in position 3 (C-3) and 4 (C-4) of the piperidin ring form together a 5 to 7 -membered cycloalkyl or cycloalkenyl ring or a 6 to 14 membered aromatic ring, either unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
and/or in formula (Ia)
- X: is O, S, N-OH, N-O-(C₁-C₆)-alkyl, N-O-CO-R⁸, N-O-COOR⁹
and wherein
- R⁸: is H, (C₂-C₆)-alkenyl, (C₂-C₆)-haloalkenyl, (C₂-C₆)-alkynyl, (C₂-C₆)-haloalkynyl, (C₃-C₇)-cycloalkyl or (C₁-C₆)-alkyl which last mentioned group is unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴ ;
- R⁹: is H, (C₃-C₆)-alkenyl, (C₃-C₆)-haloalkenyl, (C₃-C₆)-alkynyl, (C₃-C₆)-haloalkynyl, (C₃-C₇)-cycloalkyl, or (C₁-C₆)-alkyl which last mentioned group is unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴ ;
- R¹⁰: is phenyl, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
- R¹¹: is a saturated, unsaturated or heteroaromatic heterocyclyl radical unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
- R¹²: is (C₁-C₆)-alkyl or (C₁-C₆)-haloalkyl
- R¹³ and R¹⁴: are each independently H (C₁-C₆)-alkyl, (C₁-C₆)-haloalkyl, (C₂-C₆)-alkenyl, (C₂-C₆)-alkynyl, (C₃-C₇)-cycloalkyl or (C₃-C₇)-cycloalkyl-(C₁-C₆)-alkyl; or R¹³ and R¹⁴ together with the N form a 4 to 8-membered heteroaryl or heterocyclyl ring that may contain one or two further hetroatoms selected from the group consisting ot N, O, S ;
and wherein
- n: is 0 or 1,
- p: is 0, 1 or 2,
or a pesticidally acceptable salt thereof, for the control of pests.

More preferred compounds for the control of pests are a class of compounds of formual (I) in which:
- A: is a straight chain or branched (C₁-C₃)-alkylene and
- R¹: is phenyl
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or is (C₁-C₆)-alkyl, (C₁-C₆)-haloalkyl, (C₃-C₇)-cycloalkyl, (C₃-C₇)-cycloalkylenyl;
or is pyridine, pyrimidine, pyrazine and pyridazine,
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo; and
- R² and R³: are each independently H or CH₃;
or in case R² and R³ are both CH₃ the methyl groups may be connected to form a -C₂H₄- group;
and wherein in formula (Ib) preferrably
- R⁴ and R⁵: are each independently H, (C₁-C₆)-alkyl, (C₁-C₆)-alkoxy;
or in case R⁴ and R⁵ are (C₁-C₆)-alkyl or (C₁-C₆)-alkoxy both groups together
with C-4 of the piperidine ring may form a 4-7 membered ring;
and wherein in formula (lc) preferrably
- R⁶: is OH, OCOR⁸, OCOOR⁹, COO(C₁-C₄)-alkyl ; and
- R⁷: is H or
- R⁷: together with R⁶ and C-3 and C-4 of the piperidin ring forms a saturated or unsaturated 6-membered ring, either unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ to generate formula (IIa) and (IIb) and (IIc); 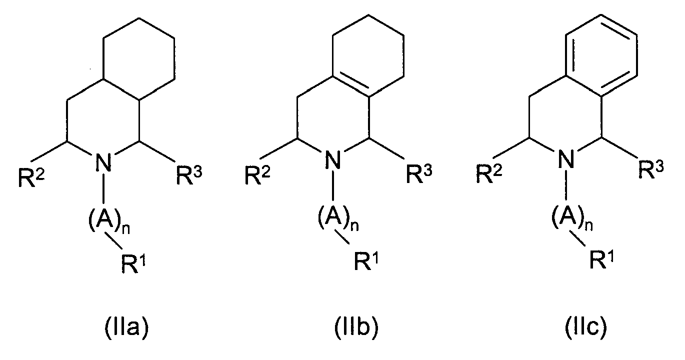
and wherein in formula (Ic) preferably
X is O;
and wherein R⁸, R⁹, R¹⁰, R¹¹, R¹², R¹³ and R¹⁴ are as defined above, and wherein n is 0 or 1 and p is 0, 1 or 2.
or an pesticidally acceptable salt thereof.

Concerning R⁸ radicals selected from the group consisting of H, (C₃-C₇)-cycloalkyl, or (C₁-C₆)-alkyl which last mentioned group is unsubstituted or substituted by one or more radicals selected from the group consisting of halogen, (C₃-C₇)-cycloalkyl, (C₁₋C₆)-alkoxy, (C₁-C₆)-alkylthio, OH, CN, NO₂, R¹⁰, R¹¹, S(O)ₚR¹² and NR¹³R¹⁴ are preferred;

Concerning R⁹ radicals selected from the group consisting of H, (C₃-C₇)-cycloalkyl, or (C₁-C₆)-alkyl which last mentioned group is unsubstituted or substituted by one or more radicals selected from the group consisting of halogen, (C₃-C₇)-cycloalkyl, (C₁₋C₆)-alkoxy, (C₁-C₆)-alkylthio, OH, CN, NO₂, R¹⁰, R¹¹, S(O)ₚR¹² and NR¹³R¹⁴ are preferred.

Concerning R¹⁰ phenyl radical, unsubstituted or substituted by one or more radicals selected from the group consisting of halogen, (C₁-C₃)-alkyl, (C₁-C₃)-haloalkyl, (C₁₋C₃)-alkoxy, (C₁-C₃)-haloalkoxy, CN, NO₂, S(O)ₚR¹² and NR¹³R¹⁴ are preferred.

R¹¹ is a saturated, unsaturated or heteroaromatic heterocyclyl radical unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;

Concerning R¹² radicals selected from the group consisting of (C₁-C₃)-alkyl or (C₁₋C₃)-haloalkyl are preferred.

Concerning R¹³ and R¹⁴ radicals independently selected from the group consisting of H (C₁-C₃)-alkyl, (C₁-C₃)-haloalkyl, (C₂-C₄)-alkenyl, (C₂-C₄)-alkynyl, (C₃-C₇)-cycloalkyl or (C₃-C₇)-cycloalkyl-(C₁-C₆)-alkyl; or wherein R¹³ and R¹⁴ together with the N form a 4 to 8-membered ring that may contain one or two further N atoms or one further O or S atom; are preferred.

Further compounds of formula (I) are preferred wherein n is 1.

### General process for the preparation of compounds of formula (I):

In the description when symbols appearing in formulae are not specifically defined, it is understood that they are "as defined above" in accordance with the first definition of each symbol in the specification.

The compounds of general formula (I) can be prepared by the application, adaptation and combination of known methods, e.g. as described in
Baraldi, Pier Giovanni; Romagnoli, Romeo; Pavani, Maria Giovanna; Nunez, Maria del Carmen; Tabrizi, Mojgan Aghazadeh; Shryock, John C.; Leung, Edward; Moorman, Allan R.; Uluoglu, Canan; Iannotta, Valeria; Merighi, Stefania; Borea, Pier Andrea; JMCMAR; J.Med.Chem.; EN; 46; 5; 2003; 794 - 809;
Gooding, Owen W.; Beard, Colin C.; HTCYAM; Heterocycles; EN; 32; 9; 1991; 1777-1780;
Klein, Pieter A. M van der; Kourounakis, Angeliki P.; IJzerman, Ad P.; JMCMAR; J.Med.Chem.; EN; 42; 18; 1999; 3629 - 3635;
Gong, Leyi; Hogg, J. Heather; Collier, James; Wilhelm, Robert S.; Soderberg, Carol; BMCLE8; Bioorg.Med.Chem.Lett.; EN; 13; 20; 2003; 3597 - 3600;
Faul, Margaret M.; Kobierski, Michael E.; Kopach, Michael E., Journal of Organic Chemistry (2003), 68(14), 5739-5741.

It is known from prior art that compounds of formula (I) or (Ia) wherein R¹, R², R³, (A)ₙ are as defined above, and R⁴ and R⁵ are X, wherein X is O may be prepared by the reaction of piperidones of formula (IIIa) with alkylating agents of formula (IV): 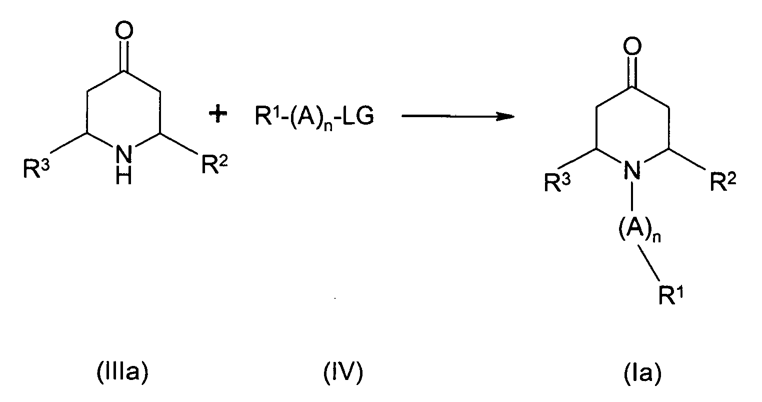 wherein LG is halogen or a sulfonate group RSO₂O.
Compounds of formula (IV) R¹-(A)ₙ-LG may be halides or sulfonates.

The addition reaction may be performed in presence of bases like alkali hydroxides, alkali alcoholates, alkali carbonates, organic amines (e.g. triethylamine, diisopropylethylamine) in organic solvents.

It is also known that compounds of formula (I), (Ib) or (Ic) wherein R¹ , R², R³ , (A)ₙ , R⁴, R⁵ (wherein R⁴, R⁵ are not X), R⁶, R⁷ are as defined above, may be prepared by the reaction of piperidines of formula (IIIb) or (IIIc) with alkylating agents of formula (IV):

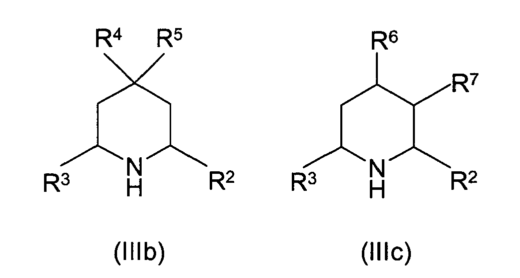

The addition reaction may be performed in presence of bases like alkali hydroxides, alkali alcoholates, alkali carbonates, organic amines (e.g. triethylamine, diisopropylethylamine) in organic solvents.

Compounds of formula (lb) may be prepared as described e.g. in:
Zhang, Wei; Curran, Dennis P.; Chen, Christine Hiu-Tung; TETRAB; Tetrahedron; EN; 58; 20; 2002; 3871 - 3876
J.R.Geigy A.-G.; CH 469736; 1966; Chem.Abstr.; EN; 71; 113003n; 1969;

Compounds of formula (Ic) may be prepared as described e.g. in:
Buettelmann, Bernd; Alanine, Alexander; Bourson, Anne; Gill, Ramanjit; Heitz, Marie-Paule; Mutel, Vincent; Pinard, Emmanuel; Trube, Gerhard; Wyler, Rene; BMCLE8; Bioorg.Med.Chem.Lett.; EN; 13; 5; 2003; 829 - 832 ;
Synthelabo S.A.; DE 2919553; 1979; Chem.Abstr.; EN; 92; 94264;

It is further known from prior art that compounds of formula (Ia) wherein R¹, R², R³, (A)ₙ are as defined above, and X is O may be prepared by the reaction of ketals of formula (Ib) wherein R¹, R², R³ , (A)ₙ are as defined above and R⁴, R⁵ are alkoxy or alkylendioxy, with acids in aqueous mixtures. This hydrolysis reaction is performed in water or mixtures of water with organic solvents in presence of acids like hydrochloric acid or sulfuric acid at temperatures from 20 to 120°C.

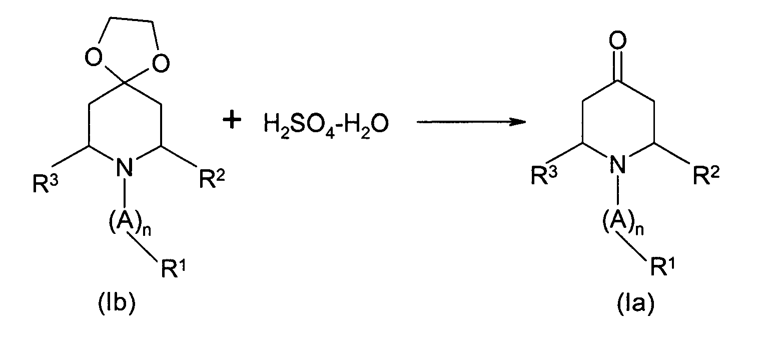

The reaction may be performed as described e.g. in:
Novelli, Federica; Sparatore, Fabio; FRMCE8; Farmaco; EN; 57; 11; 2002; 871 - 882 Janssens, Frans; Torremans, Joseph; Janssen, Marcel; Stokbroekx, Raymond A.; Luyckx, Marcel; Janssen, Paul A. J.; JMCMAR; J.Med.Chem.; EN; 28; 12; 1985; 1925-1933
Faul, Margaret M.; Kobierski, Michael E.; Kopach, Michael E.; JOCEAH; J.Org.Chem.; EN; 68; 14; 2003; 5739 - 5741
Faja, Montserrat; Reese, Colin B.; Song, Quanlai; Zhang, Pei-Zhuo; JCPRB4; J.Chem.Soc.Perkin Trans.1; EN; 3; 1997; 191-194
J.R.Geigy A.-G.; CH 469736; 1966; Chem.Abstr.; EN; 71; 113003n; 1969

It is known that compounds of formula (Ia) wherein R¹, (A)ₙ are as defined above, X is O and R², R³ together form a C₂H₄ bridge may be prepared by the reaction of amines R¹-(A)ₙ-NH₂ (V) with succinaldehyde and 3-oxo-pentanedioic acid or its esters to generate 8-substituted tropanones (Ia) (R², R³ together form C₂H₄). 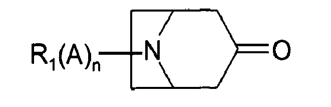 (Ia), wherein R², R³ together form C₂H₄

The reaction may be performed as described e.g. in:
Sterling Drug Inc.; US 2845427; 1955; Sterling Drug Inc.; US 2836598; 1954.
Maag, Hans; Locher, Rita; Daly, John J.; Kompis, Ivan; HCACAV; Helv.Chim.Acta; EN; 69; 1986; 887-897
Ridder, Dirk J. A. De; Goubitz, Kees; Schenk, Henk; Krijnen, Bert; Verhoven, Jan W.; HCACAV; Helv.Chim.Acta; EN; 86; 2003; 812 - 826

### Biological Scope:

According to a further feature of the present invention there is provided a method for the control of pests at a locus which comprises applying thereto an effective amount of a compound of formula (I) or a salt thereof. For this purpose, the said compound is normally used in the form of a pesticidal composition (i.e. in association with compatible diluents or carriers and/or surface active agents suitable for use in pesticidal compositions), for example as hereinafter described.

The term "compound of the invention" as used hereinafter embraces a thioether derivative of formula (I) as defined above and a pesticidally acceptable salt thereof.

One aspect of the present invention as defined above is a method for the control of pests at a locus. The locus includes, for example, the pest itself, the place (plant, field, forest, orchard, waterway, soil, plant product, or the like) where the pest resides or feeds, or a place susceptible to future infestation by the pest. The compound of the invention may therefore be applied directly to the pest, to the place where the pest resides or feeds, or to the place susceptible to future infestation by the pest.
As is evident from the foregoing pesticidal uses, the present invention provides pesticidally active compounds and methods of use of said compounds for the control of a number of pest species which includes: arthropods, especially insects or mites, or plant nematodes. The compound of the invention may thus be advantageously employed in practical uses, for example, in agricultural or horticultural crops, in forestry, in veterinary medicine or livestock husbandry, or in public health.
The compounds of the invention may be used for example in the following applications and on the following pests:

For the control of soil insects, such as corn rootworm, termites (especially for protection of structures), root maggots, wireworms, root weevils, stalkborers, cutworms, root aphids, or grubs. They may also be used to provide activity against plant pathogenic nematodes, such as root-knot, cyst, dagger, lesion, or stem or bulb nematodes, or against mites. For the control of soil pests, for example corn rootworm, the compounds are advantageously applied to or incorporated at an effective rate into the soil in which crops are planted or to be planted or to the seeds or growing plant roots.
In the area of public health, the compounds are especially useful in the control of many insects, especially filth flies or other Dipteran pests, such as houseflies, stableflies, soldierflies, hornflies, deerflies, horseflies, midges, punkies, blackflies, or mosquitoes.
In the protection of stored products, for example cereals, including grain or flour, groundnuts, animal feedstuffs, timber or household goods, e.g. carpets and textiles, compounds of the invention are useful against attack by arthropods, more especially beetles, including weevils, moths or mites, for example Ephestia spp. (flour moths), Anthrenus spp. (carpet beetles), Tribolium spp. (flour beetles), Sitophilus spp. (grain weevils) or Acarus spp. (mites).
In the control of cockroaches, ants or termites or similar arthropod pests in infested domestic or industrial premises or in the control of mosquito larvae in waterways, wells, reservoirs or other running or standing water.
For the treatment of foundations, structures or soil in the prevention of the attack on building by termites, for example, Reticulitermes spp., Heterotermes spp., Coptotermes spp..
In agriculture against adults, larvae and eggs of Lepidoptera (butterflies and moths), e.g. Heliothis spp. such as Heliothis virescens (tobacco budworm), Heliothis armigera and Heliothis zea. Against adults and larvae of Coleoptera (beetles) e.g. Anthonomus spp. e.g. grandis (cotton boll weevil), Leptinotarsa decemlineata (Colorado potato beetle), Diabrotica spp. (corn rootworms). Against Heteroptera (Hemiptera and
Homoptera) e.g. Psylla spp., Bemisia spp., Trialeurodes spp., Aphis spp., Myzus spp., Megoura viciae, Phylloxera spp., Nephotettix spp. (rice leaf hoppers), Nilaparvata spp..
Against Diptera e.g. Musca spp.. Against Thysanoptera such as Thrips tabaci. Against Orthoptera such as Locusta and Schistocerca spp., (locusts and crickets) e.g. Gryllus spp., and Acheta spp. for example, Blatta orientalis, Periplaneta americana, Blatella germanica, Locusta migratoria migratorioides, and Schistocerca gregaria. Against Collembola e.g. Periplaneta spp. and Blatella spp. (roaches). Against arthropods of agricultural significance such as Acari (mites) e.g. Tetranychus spp., and Panonychus spp..
Against nematodes which attack plants or trees of importance to agriculture, forestry or horticulture either directly or by spreading bacterial, viral, mycoplasma or fungal diseases of the plants. For example root-knot nematodes such as Meloidogyne spp. (e.g. M. incognita).

In the field of veterinary medicine or livestock husbandry or in the maintenance of public health against arthropods which are parasitic internally or externally upon vertebrates, particularly warm-blooded vertebrates, for example domestic animals, e.g. cattle, sheep, goats, equines, swine, poultry, dogs or cats, for example Acarina, including ticks (e.g. soft-bodied ticks including Argasidae spp. e.g. Argas spp. and Ornithodorus spp. (e.g. Ornithodorus moubata); hard-bodied ticks including Ixodidae spp., e.g. Boophilus spp. e.g. Boophilus microplus, Rhipicephalus spp. e.g. Rhipicephalus appendiculatus and Rhipicephalus sanguineus; mites (e.g. Damalinia spp.); fleas (e.g. Ctenocephalides spp. e.g. Ctenocephalides felis (cat flea) and Ctenocephalides canis (dog flea)); lice e.g. Menopon spp.; Diptera (e.g. Aedes spp., Anopheles spp., Musca spp., Hypoderma spp.); Hemiptera.; Dictyoptera (e.g. Periplaneta spp., Blatella spp.); Hymenoptera; for example against infections of the gastro-intestinal tract caused by parasitic nematode worms, for example members of the family Trichostrongylidae.

In a preferred aspect of the invention the compounds of formula (I) are used for the control of parasites of animals. Preferably the animal to be treated is a domestic companion animal such as a dog or a cat.

In a further aspect of the invention the compounds of formula (I) or salts or compositions thereof are used for the preparation of a veterinary medicament.

A further feature of the invention thus relates to the use of a compound of formula (I) or a salt thereof, or of a composition thereof, for the control of pests.

In practical use for the control of arthropods, especially insects or mites, or helminths, especially nematode pests of plants, a method, for example, comprises applying to the plants or to the medium in which they grow an effective amount of a compound of the invention. For such a method, the compound of the invention is generally applied to the locus in which the arthropod or nematode infestation is to be controlled at an effective rate in the range of about 2g to about 1 kg of the active compound per hectare of locus treated. Under ideal conditions, depending on the pest to be controlled, a lower rate may offer adequate protection. On the other hand, adverse weather conditions, resistance of the pest or other factors may require that the active ingredient be used at higher rates. The optimum rate depends usually upon a number of factors, for example, the type of pest being controlled, the type or the growth stage of the infested plant, the row spacing or also the method of application. Preferably an effective rate range of the active compound is from about 10g/ha to about 400g/ha, more preferably from about 50g/ha to about 200 g/ha.
When a pest is soil-borne, the active compound generally in a formulated composition, is distributed evenly over the area to be treated (ie, for example broadcast or band treatment) in any convenient manner and is applied at rates from about 10g/ha to about 400g ai/ha, preferably from about 50g/ha to about 200 g ai/ha. When applied as a root dip to seedlings or drip irrigation to plants the liquid solution or suspension contains from about 0.075 to about 1000 mg ai/l, preferably from about 25 to about 200 mg ai/l. Application may be made, if desired, to the field or crop-growing area generally or in close proximity to the seed or plant to be protected from attack. The compound of the invention can be washed into the soil by spraying with water over the area or can be left to the natural action of rainfall.
During or after application, the formulated compound can, if desired, be distributed mechanically in the soil, for example by ploughing, disking, or use of drag chains. Application can be prior to planting, at planting, after planting but before sprouting has taken place, or after sprouting.
The compound of the invention and methods of control of pests therewith are of particular value in the protection of field, forage, plantation, glasshouse, orchard or vineyard crops, of ornamentals, or of plantation or forest trees, for example: cereals (such as wheat or rice), cotton, vegetables (such as peppers), field crops (such as sugar beets, soybeans or oil seed rape), grassland or forage crops (such as maize or sorghum), orchards or groves (such as of stone or pit fruit or citrus), ornamental plants, flowers or vegetables or shrubs under glass or in gardens or parks, or forest trees (both deciduous and evergreen) in forests, plantations or nurseries.
They are also valuable in the protection of timber (standing, felled, converted, stored or structural) from attack, for example, by sawflies or beetles or termites.
They have applications in the protection of stored products such as grains, fruits, nuts, spices or tobacco, whether whole, milled or compounded into products, from moth, beetle, mite or grain weevil attack. Also protected are stored animal products such as skins, hair, wool or feathers in natural or converted form (e.g. as carpets or textiles) from moth or beetle attack as well as stored meat, fish or grains from beetle, mite or fly attack.

Additionally, the compound of the invention and methods of use thereof are of particular value in the control of arthropods or helminths which are injurious to, or spread or act as vectors of diseases domestic animals, for example those hereinbefore mentioned, and more especially in the control of ticks, mites, lice, fleas, midges, or biting, nuisance or myiasis flies. The compounds of the invention are particularly useful in controlling arthropods or helminths which are present inside domestic host animals or which feed in or on the skin or suck the blood of the animal, for which purpose they may be administered orally, parenterally, percutaneously or topically.
The compositions hereinafter described for application to growing crops or crop growing loci or as a seed dressing may, in general, alternatively be employed in the protection of stored products, household goods, property or areas of the general environment. Suitable means of applying the compounds of the invention include: to growing crops as foliar sprays (for example as an in-furrow spray), dusts, granules, fogs or foams or also as suspensions of finely divided or encapsulated compositions as soil or root treatments by liquid drenches, dusts, granules, smokes or foams; to seeds of crops via application as seed dressings, e.g. by liquid slurries or dusts; to animals infested by or exposed to infestation by arthropods or helminths, by parenteral, oral or topical application of compositions in which the active ingredient exhibits an immediate and/or prolonged action over a period of time against the arthropods or helminths, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax smears or livestock self-treatment systems;
to the environment in general or to specific locations where pests may lurk, including stored products, timber, household goods, or domestic or industrial premises, as sprays, fogs, dusts, smokes, wax-smears, lacquers, granules or baits, or in tricklefeeds to waterways, wells, reservoirs or other running or standing water.

The compounds of formula (I) are particularly useful for the control of parasites of animals when applied orally, and in a further preferred aspect of the invention the compounds of formula (I) are used for the control of parasites of animals by oral application. The compounds of the formula (I) or salts thereof may be administered before, during or after meals. The compounds of the formula (I) or salts thereof may be mixed with a carrier and/or foodstuff.
The compound of the formula (I) or salt thereof is administered orally in a dose to the animal in a dose range generally from 0.1 to 500 mg/kg of the compound of the formula (I) or salt thereof per kilogram of animal body weight (mg/kg).
The frequency of treatment of the animal, preferably the domestic animal to be treated by the compound of the formula (I) or salt thereof is generally from about once per week to about once per year, preferably from about once every two weeks to once every three months.
The compounds of the invention may be administered most advantageously with another parasiticidally effective material, such as an endoparasiticide, and/or an ectoparasiticide, and/or an endectoparasiticide. For example, such compounds include macrocyclic lactones such as avermectins or milbemycins e.g., ivermectin, pyratel or an insect growth regulator such as lufenuron or methoprene.

The compounds of the formula (I) can also be employed for controlling harmful organisms in crops of known genetically engineered plants or genetically engineered plants yet to be developed. As a rule, the transgenic plants are distinguished by especially advantageous properties, for example by resistances to particular crop protection agents, resistances to plant diseases or pathogens of plant diseases, such as particular insects or microorganisms such as fungi, bacteria or viruses. Other particular properties concern, for example, the harvested material with regard to quantity, quality, storage properties, composition and specific constituents. Thus, transgenic plants are known where the starch content is increased, or the starch quality is altered, or where the harvested material has a different fatty acid composition.

The use in economically important transgenic crops of useful plants and ornamentals is preferred, for example of cereals such as wheat, barley, rye, oats, millet, rice, cassava and maize or else crops of sugar beet, cotton, soya, oilseed rape, potatoes, tomatoes, peas and other types of vegetables.

When used in transgenic crops, in particular those which have resistances to insects, effects are frequently observed, in addition to the effects against harmful organisms to be observed in other crops, which are specific for application in the transgenic crop in question, for example an altered or specifically widened spectrum of pests which can be controlled, or altered application rates which may be employed for application.

The invention therefore also relates to the use of compounds of the formula (I) for controlling harmful organisms in transgenic crop plants.

According to a further feature of the present invention there is provided a pesticidal composition comprising one or more compounds of the invention as defined above, in association with, and preferably homogeneously dispersed in one or more compatible pesticidally acceptable diluents or carriers and/or surface active agents [i.e. diluents or carriers and/or surface active agents of the type generally accepted in the art as being suitable for use in pesticidal compositions and which are compatible with compounds of the invention].
In practice, the compounds of the invention most frequently form parts of compositions. These compositions can be employed to control arthropods, especially insects, or plant nematodes or mites. The compositions may be of any type known in the art suitable for application to the desired pest in any premises or indoor or outdoor area. These compositions contain at least one compound of the invention as the active ingredient in combination or association with one or more other compatible components which are for example, solid or liquid carriers or diluents, adjuvants, surface-active-agents, or the like appropriate for the intended use and which are agronomically or medicinally acceptable. These compositions, which may be prepared by any manner known in the art, likewise form a part of this invention.

The compounds of the invention, in their commercially available formulations and in the use forms prepared from these formulations may be present in mixtures with other active substances such as insecticides, attractants, sterilants, acaricides, nematicides, fungicides, growth regulatory substances or herbicides.

The pesticides include, for example, phosphoric esters, carbamates, carboxylic esters, formamidines, tin compounds and materials produced by microorganisms.

Preferred components in mixtures are:
1. from the group of the phosphorus compounds
   acephate, azamethiphos, azinphos-ethyl, azinphos-methyl, bromophos, bromophosethyl, cadusafos (F-67825), chlorethoxyphos, chlorfenvinphos, chlormephos, chlorpyrifos, chlorpyrifos-methyl, demeton, demeton-S-methyl, demeton-S-methyl sulfone, dialifos, diazinon, dichlorvos, dicrotophos, dimethoate, disulfoton, EPN, ethion, ethoprophos, etrimfos, famphur, fenamiphos, fenitriothion, fensulfothion, fenthion, flupyrazofos, fonofos, formothion, fosthiazate, heptenophos, isazophos, isothioate, isoxathion, malathion, methacrifos, methamidophos, methidathion, salithion, mevinphos, monocrotophos, naled, omethoate, oxydemeton-methyl, parathion, parathion-methyl, phenthoate, phorate, phosalone, phosfolan, phosphocarb (BAS-301), phosmet, phosphamidon, phoxim, pirimiphos, pirimiphosethyl, pirimiphos-methyl, profenofos, propaphos, proetamphos, prothiofos, pyraclofos, pyridapenthion, quinalphos, sulprofos, temephos, terbufos, tebupirimfos, tetrachlorvinphos, thiometon, triazophos, trichlorphon, vamidothion;
2. from the group of the carbamates
   alanycarb (OK-135), aldicarb, 2-sec-butylphenyl methylcarbamate (BPMC), carbaryl, carbofuran, carbosulfan, cloethocarb, benfuracarb, ethiofencarb, furathiocarb, HCN-801, isoprocarb, methomyl, 5-methyl-m-cumenylbutyryl (methyl)carbamate, oxamyl, pirimicarb, propoxur, thiodicarb, thiofanox, 1-methylthio(ethylideneamino)-N-methyl-N-(morpholinothio)carbamate (UC 51717), triazamate;
3. from the group of the carboxylic esters
   acrinathrin, allethrin, alphametrin, 5-benzyl-3-furylmethyl (E)- (1 R)-cis-2,2-dimethyl-3-(2-oxothiolan-3-ylidenemethyl)cyclopropanecarboxylate, beta-cyfluthrin, alphacypermethrin, beta-cypermethrin, bioallethrin, bioallethrin ((S)-cyclopentylisomer), bioresmethrin, bifenthrin, (RS)-1-cyano-1-(6-phenoxy-2-pyridyl)methyl (1 RS)-trans-3-(4-tert-butylphenyl)-2,2-dimethylcyclopropanecarboxylate (NCl 85193), cycloprothrin, cyfluthrin, cyhalothrin, cythithrin, cypermethrin, cyphenothrin, deltamethrin, empenthrin, esfenvalerate, fenfluthrin, fenpropathrin, fenvalerate, flucythrinate, flumethrin, fluvalinate (D isomer), imiprothrin (S-41311), lambda-cyhalothrin, permethrin, phenothrin (® isomer), prallethrin, pyrethrins (natural products), resmethrin, tefluthrin, tetramethrin, theta-cypermethrin, tralomethrin, transfluthrin, zeta-cypermethrin (F-56701);
4. from the group of the amidines
   amitraz, chlordimeform;
5. from the group of the tin compounds
   cyhexatin, fenbutatin oxide;
6. others
   abamectin, ABG-9008, acetamiprid, acequinocyl, Anagrapha falcitera, AKD-1022, AKD-3059, ANS-118, azadirachtin, Bacillus thuringiensis, Beauveria bassianea, bensultap, bifenazate, binapacryl, BJL-932, bromopropylate, BTG-504, BTG-505, buprofezin, camphechlor, cartap, chlorobenzilate, chlorfenapyr, chlorfluazuron, 2-(4-chlorophenyl)-4,5-diphenylthiophene (UBI-T 930), chlorfentezine, chlorproxyfen, chromafenozide, clothianidine, 2-naphthylmethyl cyclopropanecarboxylate (Ro12-0470), cyromazin, diacloden (thiamethoxam), diafenthiuron, DBI-3204, ethyl 2-chloro-N-(3,5-dichloro-4-(1,1,2,3,3,3-hexafluoro-1-propyloxy)phenyl)carbamoyl)-2-carboximidate, DDT, dicofol, diflubenzuron, N-(2,3-dihydro-3-methyl-1,3-thiazol-2-ylidene)-2,4-xylidine, dihydroxymethyldihydroxypyrrolidine, dinobuton, dinocap, diofenolan, emamectin benzoate, endosulfan, ethiprole (sulfethiprole), ethofenprox, etoxazole, fenazaquin, fenoxycarb, fipronil, flonicamid (IKI-220), fluazuron, flumite (flufenzine, SZI-121), 2-fluoro-5-(4-(4-ethoxyphenyl)-4-methyl-1-pentyl)diphenyl ether (MTI 800), granulosis and nuclear polyhedrosis viruses, fenpyroximate, fenthiocarb, fluacrypyrim, flubenzimine, flubrocythrinate, flucycloxuron, flufenoxuron, flufenzine, flufenprox, fluproxyfen, gamma-HCH, halfenozide, halofenprox, hexaflumuron (DE_473), hexythiazox, HOI-9004, hydramethylnon (AC 217300), indoxacarb, ivermectin, L-14165, imidacloprid, indoxacarb (DPX-MP062), kanemite (AKD-2023), lufenuron, M-020, M-020, methoxyfenozide, milbemectin, NC-196, neemgard, nidinoterfuran, nitenpyram, 2-nitromethyl-4,5-dihydro-6H-thiazine (DS 52618), 2-nitromethyl-3,4-dihydrothiazole (SD 35651), 2-nitromethylene-1,2-thiazinan-3-ylcarbamaldehyde (WL 108477), novaluron, pirydaryl, propargite, protrifenbute, pymethrozine, pyridaben, pyrimidifen, pyriproxyfen, NC-196, NC-1111, NNI-9768, novaluron (MCW-275), OK-9701, OK-9601, OK-9602, OK-9802, R-195, RH-0345, RH-2485, RYI-210, S-1283, S-1833, SI-8601, silafluofen, silomadine (CG-177), spinosad, spirodiclofen, SU-9118, tebufenozide, tebufenpyrad, teflubenzuron, tetradifon, tetrasul, thiacloprid, thiocyclam, thiamethoxam, tolfenpyrad, triazamate, triethoxyspinosyn A, triflumuron, verbutin, vertalec (mykotal), YI-5301.

The abovementioned components for combinations are known active substances, many of which are described in Ch.R Worthing, S.B. Walker, The Pesticide Manual, 12^{th} Edition, British Crop Protection Council, Farnham 2000.

The effective use doses of the compounds employed in the invention can vary within wide limits, particularly depending on the nature of the pest to be eliminated or degree of infestation, for example, of crops with these pests. In general, the compositions according to the invention usually contain about 0.05 to about 95% (by weight) of one or more active ingredients according to the invention, about 1 to about 95% of one or more solid or liquid carriers and, optionally, about 0.1 to about 50% of one or more other compatible components, such as surface-active agents or the like. In the present account, the term "carrier" denotes an organic or inorganic ingredient, natural or synthetic, with which the active ingredient is combined to facilitate its application, for example, to the plant, to seeds or to the soil. This carrier is therefore generally inert and it must be acceptable (for example, agronomically acceptable, particularly to the treated plant).

The carrier may be a solid, for example, clays, natural or synthetic silicates, silica, resins, waxes, solid fertilizers (for example ammonium salts), ground natural minerals, such as kaolins, clays, talc, chalk, quartz, attapulgite, montmorillonite, bentonite or diatomaceous earth, or ground synthetic minerals, such as silica, alumina, or silicates especially aluminium or magnesium silicates. As solid carriers for granules the following are suitable: crushed or fractionated natural rocks such as calcite, marble, pumice, sepiolite and dolomite; synthetic granules of inorganic or organic meals; granules of organic material such as sawdust, coconut shells, corn cobs, corn husks or tobacco stalks; kieselguhr, tricalcium phosphate, powdered cork, or absorbent carbon black; water soluble polymers, resins, waxes; or solid fertilizers. Such solid compositions may, if desired, contain one or more compatible wetting, dispersing, emulsifying or colouring agents which, when solid, may also serve as a diluent.
The carrier may also be liquid, for example: water; alcohols, particularly butanol or glycol, as well as their ethers or esters, particularly methylglycol acetate; ketones, particularly acetone, cyclohexanone, methylethyl ketone, methylisobutylketone, or isophorone; petroleum fractions such as paraffinic or aromatic hydrocarbons, particularly xylenes or alkyl naphthalenes; mineral or vegetable oils; aliphatic chlorinated hydrocarbons, particularly trichloroethane or methylene chloride; aromatic chlorinated hydrocarbons, particularly chlorobenzenes; water-soluble or strongly polar solvents such as dimethylformamide, dimethyl sulphoxide, or N-methylpyrrolidone; liquefied gases; or the like or a mixture thereof.
The surface-active agent may be an emulsifying agent, dispersing agent or wetting agent of the ionic or non-ionic type or a mixture of such surface-active agents. Amongst these are e.g., salts of polyacrylic acids, salts of lignosulphonic acids, salts of phenolsulphonic or naphthalenesulphonic acids, polycondensates of ethylene oxide with fatty alcohols or fatty acids or fatty esters or fatty amines, substituted phenols (particularly alkylphenols or arylphenols), salts of sulphosuccinic acid esters, taurine derivatives (particularly alkyltaurates), phosphoric esters of alcohols or of polycondensates of ethylene oxide with phenols, esters of fatty acids with polyols, or sulphate, sulphonate or phosphate functional derivatives of the above compounds. The presence of at least one surface-active agent is generally essential when the active ingredient and/or the inert carrier are only slightly water soluble or are not water soluble and the carrier agent of the composition for application is water. Compositions of the invention may further contain other additives such as adhesives or colorants. Adhesives such as carboxymethylcellulose or natural or synthetic polymers in the form of powders, granules or lattices, such as arabic gum, polyvinyl alcohol or polyvinyl acetate, natural phospholipids, such as cephalins or lecithins, or synthetic phospholipids can be used in the formulations. It is possible to use colorants such as inorganic pigments, for example: iron oxides, titanium oxides or Prussian Blue; organic dyestuffs, such as alizarin dyestuffs, azo dyestuffs or metal phthalocyanine dyestuffs; or trace nutrients such as salts of iron, manganese, boron, copper, cobalt, molybdenum or zinc.
For their agricultural application, the compounds of the invention are therefore generally in the form of compositions, which are in various solid or liquid forms.
Solid forms of compositions which can be used are dusting powders (with a content of the compound of the invention, ranging up to 80%), wettable powders or granules (including water dispersible granules), particularly those obtained by extrusion, compacting, impregnation of a granular carrier, or granulation starting from a powder (the content of the compound of the invention, in these wettable powders or granules being between about 0.5 and about 80%). Solid homogenous or heterogenous compositions containing one or more compounds of the invention, for example granules, pellets, briquettes or capsules, may be used to treat standing or running water over a period of time. A similar effect may be achieved using trickle or intermittent feeds of water dispersible concentrates as described herein.
Liquid compositions, for example, include aqueous or non-aqueous solutions or suspensions (such as emulsifiable concentrates, emulsions, flowables, dispersions, or solutions) or aerosols. Liquid compositions also include, in particular, emulsifiable concentrates, dispersions, emulsions, flowables, aerosols, wettable powders (or powder for spraying), dry flowables or pastes as forms of compositions which are liquid or intended to form liquid compositions when applied, for example as aqueous sprays (including low and ultra-low volume) or as fogs or aerosols.
Liquid compositions, for example, in the form of emulsifiable or soluble concentrates most frequently comprise about 5 to about 80% by weight of the active ingredient, while the emulsions or solutions which are ready for application contain, in their case, about 0.01 to about 20% of the active ingredient. Besides the solvent, the emulsifiable or soluble concentrates may contain, when required, about 2 to about 50% of suitable additives, such as stabilizers, surface-active agents, penetrating agents, corrosion inhibitors, colorants or adhesives. Emulsions of any required concentration, which are particularly suitable for application, for example, to plants, may be obtained from these concentrates by dilution with water. These compositions are included within the scope of the compositions which may be employed in the present invention. The emulsions may be in the form of water-in-oil or oil-in-water type and they may have a thick consistency.
The liquid compositions of this invention may, in addition to normal agricultural use applications be used for example to treat substrates or sites infested or liable to infestation by arthropods (or other pests controlled by compounds of this invention) including premises, outdoor or indoor storage or processing areas, containers or equipment or standing or running water.
All these aqueous dispersions or emulsions or spraying mixtures can be applied, for example, to crops by any suitable means, chiefly by spraying, at rates which are generally of the order of about 100 to about 1,200 liters of spraying mixture per hectare, but may be higher or lower (eg. low or ultra-low volume) depending upon the need or application technique. The compound or compositions according to the invention are conveniently applied to vegetation and in particular to roots or leaves having pests to be eliminated. Another method of application of the compounds or compositions according to the invention is by chemigation, that is to say, the addition of a formulation containing the active ingredient to irrigation water. This irrigation may be sprinkler irrigation for foliar pesticides or it can be ground irrigation or underground irrigation for soil or for systemic pesticides.

The concentrated suspensions, which can be applied by spraying, are prepared so as to produce a stable fluid product which does not settle (fine grinding) and usually contain from about 10 to about 75% by weight of active ingredient, from about 0.5 to about 30% of surface-active agents, from about 0.1 to about 10% of thixotropic agents, from about 0 to about 30% of suitable additives, such as anti-foaming agents, corrosion inhibitors, stabilizers, penetrating agents, adhesives and, as the carrier, water or an organic liquid in which the active ingredient is poorly soluble or insoluble Some organic solids or inorganic salts may be dissolved in the carrier to help prevent settling or as antifreezes for water.

The wettable powers (or powder for spraying) are usually prepared so that they contain from about 10 to about 80% by weight of active ingredient, from about 20 to about 90% of a solid carrier, from about 0 to about 5% of a wetting agent, from about 3 to about 10% of a dispersing agent and, when necessary, from about 0 to about 80% of one or more stabilizers and/or other additives, such as penetrating agents, adhesives, anti-caking agents, colorants, or the like. To obtain these wettable powders, the active ingredient is thoroughly mixed in a suitable blender with additional substances which may be impregnated on the porous filler and is ground using a mill or other suitable grinder. This produces wettable powders, the wettability and the suspendability of which are advantageous. They may be suspended in water to give any desired concentration and this suspension can be employed very advantageously in particular for application to plant foliage.
The "water dispersible granules (WG)" (granules which are readily dispersible in water) have compositions which are substantially close to that of the wettable powders.
They may be prepared by granulation of formulations described for the wettable powders, either by a wet route (contacting finely divided active ingredient with the inert filler and a little water, e.g. 1 to 20% by weight, or with an aqueous solution of a dispersing agent or binder, followed by drying and screening), or by a dry route (compacting followed by grinding and screening).
The rates and concentrations of the formulated compositions may vary according to the method of application or the nature of the compositions or use thereof. Generally speaking, the compositions for application to control arthropod or plant nematode pests usually contain from about 0.00001 % to about 95%, more particularly from about 0.0005% to about 50% by weight of one or more compounds of the invention, or of total active ingredients (that is to say the compounds of the invention, together with other substances toxic to arthropods or plant nematodes, synergists, trace elements or stabilizers). The actual compositions employed and their rate of application will be selected to achieve the desired effect(s) by the farmer, livestock producer, medical or veterinary practitioner, pest control operator or other person skilled in the art.
Solid or liquid compositions for application topically to animals, timber, stored products or household goods usually contain from about 0.00005% to about 90%, more particularly from about 0.001 % to about 10%, by weight of one or more compounds of the invention. For administration to animals orally or parenterally, including percutaneously solid or liquid compositions, these normally contain from about 0.1 % to about 90% by weight of one or more compounds of the invention. Medicated feedstuffs normally contain from about 0.001 % to about 3% by weight of one or more compounds of the invention. Concentrates or supplements for mixing with feedstuffs normally contain from about 5% to about 90%, preferably from about 5% to about 50%, by weight of one or more compounds of the invention. Mineral salt licks normally contain from about 0.1 % to about 10% by weight of one or more compounds of formula (I) or pesticidally acceptable salts thereof.
Dusts or liquid compositions for application to livestock, goods, premises or outdoor areas may contain from about 0.0001 % to about 15%, more especially from about 0.005% to about 2.0%, by weight, of one or more compounds of the invention. Suitable concentrations in treated waters are between about 0.0001 ppm and about 20 ppm, more particularly about 0.001 ppm to about 5.0 ppm. of one or more compounds of the invention, and may be used therapeutically in fish farming with appropriate exposure times. Edible baits may contain from about 0.01 % to about 5%, preferably from about 0.01 % to about 1.0%, by weight, of one or more compounds of the invention.
When administered to vertebrates parenterally, orally or by percutaneous or other means, the dosage of compounds of the invention, will depend upon the species, age, or health of the vertebrate and upon the nature and degree of its actual or potential infestation by arthropod or helminth pests. A single dose of about 0.1 to about 100 mg, preferably about 2.0 to about 20.0 mg, per kg body weight of the animal or doses of about 0.01 to about 20.0 mg, preferably about 0.1 to about 5.0 mg, per kg body weight of the animal per day, for sustained medication, are generally suitable by oral or parenteral administration. By use of sustained release formulations or devices, the daily doses required over a period of months may be combined and administered to animals on a single occasion.

The following composition EXAMPLES 2A - 2M illustrate compositions for use against arthropods, especially mites or insects, or plant nematodes, which comprise, as active ingredient, compounds of the invention, such as those described in preparative examples. The compositions described in EXAMPLES 2A - 2M can each be diluted to give a sprayable compositon at concentrations suitable for use in the field. Generic chemical descriptions of the ingredients (for which all of the following percentages are in weight percent), used in the composition EXAMPLES 2A - 2M exemplified below, are as follows:

| Trade Name | Chemical Description |
|---|---|
| Ethylan BCP | Nonylphenol ethylene oxide condensate |
| Soprophor BSU | Tristyrylphenol ethylene oxide condensate |
| Arylan CA | A 70% w/v solution of calcium dodecylbenzenesulfonate |
| Solvesso 150 | Light C₁₀ aromatic solvent |
| Arylan S | Sodium dodecylbenzenesulfonate |
| Darvan NO₂ | Sodium lignosulphonate |
| Celite PF | Synthetic magnesium silicate carrier |
| Sopropon T36 | Sodium salts of polycarboxylic acids |
| Rhodigel 23 | Polysaccharide xanthan gum |
| Bentone 38 | Organic derivative of magnesium montmorillonite |
| Aerosil | Microfine silicon dioxide |

### EXAMPLE 2A

A water soluble concentrate is prepared with the composition as follows:

| | |
|---|---|
| Active ingredient | 7% |
| Ethylan BCP | 10% |
| N-methylpyrrolidone | 83% |

To a solution of Ethylan BCP dissolved in a portion of N-methylpyrrolidone is added the active ingredient with heating and stirring until dissolved. The resulting solution is made up to volume with the remainder of the solvent.

### EXAMPLE 2B

An emulsifiable concentrate (EC) is prepared with the composition as follows:

| | |
|---|---|
| Active ingredient | 25%(max) |
| Soprophor BSU | 10% |
| Arylan CA | 5% |
| N-methylpyrrolidone | 50% |
| Solvesso 150 | 10% |

The first three components are dissolved in N-methylpyrrolidone and to this is then added the Solvesso 150 to give the final volume.

### EXAMPLE 2C

A wettable powder (WP) is prepared with the composition as follows:

| | |
|---|---|
| Active ingredient | 40% |
| Arylan S | 2% |
| Darvan NO₂ | 5% |
| Celite PF | 53% |

The ingredients are mixed and ground in a hammer-mill to a powder with a particle size of less than 50 microns.

### EXAMPLE 2D

An aqueous-flowable formulation is prepared with the composition as follows:

| | |
|---|---|
| Active ingredient | 40.00% |
| Ethylan BCP | 1.00% |
| Sopropon T360. | 0.20% |
| Ethylene glycol | 5.00% |
| Rhodigel 230. | 0.15% |
| Water | 53.65% |

The ingredients are intimately mixed and are ground in a bead mill until a mean particle size of less than 3 microns is obtained.

### EXAMPLE 2E

An emulsifiable suspension concentrate is prepared with the composition as follows:

| | |
|---|---|
| Active ingredient | 30.0% |
| Ethylan BCP | 10.0% |
| Bentone 38 | 0.5% |
| Solvesso 150 | 59.5% |

The ingredients are intimately mixed and ground in a beadmill until a mean particle size of less than 3 microns is obtained.

### EXAMPLE 2F

A water dispersible granule is prepared with the composition as follows:

| | |
|---|---|
| Active ingredient | 30% |
| Darvan No 2 | 15% |
| Arylan S | 8% |
| Celite PF | 47% |

The ingredients are mixed, micronized in a fluid-energy mill and then granulated in a rotating pelletizer by spraying with water (up to 10%). The resulting granules are dried in a fluid-bed drier to remove excess water.

### EXAMPLE 2G

A dusting powder is prepared with the composition as follows:

| | |
|---|---|
| Active ingredient | 1 to 10% |
| Talc powder-superfine | 99 to 90% |

The ingredients are intimately mixed and further ground as necessary to achieve a fine powder. This powder may be appplied to a locus of arthropod infestation, for example refuse dumps, stored products or household goods or animals infested by, or at risk of infestation by, arthropods to control the arthropods by oral ingestion. Suitable means for distributing the dusting powder to the locus of arthropod infestation include mechanical blowers, handshakers or livestock self treatment devices.

### EXAMPLE 2H

An edible bait is prepared with the composition as follows:

| | |
|---|---|
| Active ingredient | 0.1 to 1.0% |
| Wheat flour | 80% |
| Molasses | 19.9 to 19% |

The ingredients are intimately mixed and formed as required into a bait form. This edible bait may be distributed at a locus, for example domestic or industrial premises, e.g. kitchens, hospitals or stores, or outdoor areas, infested by arthropods, for example ants, locusts, cockroaches or flies, to control the arthropods by oral ingestion.

### EXAMPLE 21

A solution formulation is prepared with a composition as follows:

| | |
|---|---|
| Active ingredient | 15% |
| Dimethyl sulfoxide | 85% |

The active ingredient is dissolved in dimethyl sulfoxide with mixing and or heating as required. This solution may be applied percutaneously as a pour-on application to domestic animals infested by arthropods or, after sterilization by filtration through a polytetrafluoroethylene membrane (0.22 micrometer pore size), by parenteral injection, at a rate of application of from 1.2 to 12 ml of solution per 100 kg of animal body weight.

### EXAMPLE 2J

A wettable powder is prepared with the composition as follows:

| | |
|---|---|
| Active ingredient | 50% |
| Ethylan BCP | 5% |
| Aerosil | 5% |
| Celite PF | 40% |

The Ethylan BCP is absorbed onto the Aerosil which is then mixed with the other ingredients and ground in a hammer-mill to give a wettable powder, which may be diluted with water to a concentration of from 0.001 % to 2% by weight of the active compound and applied to a locus of infestation by arthropods, for example, dipterous larvae or plant nematodes, by spraying, or to domestic animals infested by, or at risk of infection by arthropods, by spraying or dipping, or by oral administration in drinking water, to control the arthropods.

### EXAMPLE 2K

A slow release bolus composition is formed from granules containing the following components in varying percentages(similar to those described for the previous compositions) depending upon need:
Active ingredient
Density agent
Slow-release agent
Binder
The intimately mixed ingredients are formed into granules which are compressed into a bolus with a specific gravity of 2 or more. This can be administered orally to ruminant domestic animals for retention within the reticulo-rumen to give a continual slow release of active compound over an extended period of time to control infestation of the ruminant domestic animals by arthropods.

### EXAMPLE 2L

A slow release composition in the form of granules, pellets, brickettes or the like can be prepared with compositions as follows:

| | |
|---|---|
| Active ingredient | 0.5 to 25% |
| Polyvinyl chloride | 75 to 99.5% |
| Dioctyl phthalate (plasticizer) | |

The components are blended and then formed into suitable shapes by melt-extrusion or molding. These composition are useful, for example, for addition to standing water or for fabrication into collars or eartags for attachment to domestic animals to control pests by slow release.

### EXAMPLE 2M

A water dispersible granule is prepared with the composition as follows:

| | |
|---|---|
| Active ingredient | 85%(max) |
| Polyvinylpyrrolidone | 5% |
| Attapulgite clay | 6% |
| Sodium lauryl sulfate | 2% |
| Glycerine | 2% |

The ingredients are mixed as a 45% slurry with water and wet milled to a particle size of 4 microns, then spray-dried to remove water.

The following non-limiting Examples illustrate the preparation of new compounds of formula (I).

### Chemical Examples:

NMR spectra were run in deuterochloroform unless stated otherwise, and shifts are given in ppm. In the Examples which follow, quantities (also percentages) are weight based, unless stated otherwise.

### Example 1

### 8-(4-Trifluoromethyl-benzyl)-1,4-dioxa-8-aza-spiro[4.5]decane (compound 11-22)

A mixture of 1,4-dioxa-8-aza-spiro[4.5]decane (0,5g 3,5 mmol), 4-bromomethylbenzotrifluoride (0,76g 3,2 mmol) and diisopropylethylamine (0,53g 4,1 mmol) in dioxane (5 mL) were heated to reflux for 6h. After extractive workup with heptane-ethylacetate 1:1, water and sodiumhydroxide (7 mmol) a light coloured oil was obtained, 0,90g, purity >95%, compound 11-22.
¹H-NMR (ppm): 1,74, 4H, CH₂C; 2,51, 4H, NCH₂; 3,56, 2H, NCH₂Ph; 3,94, 4H, OCH₂; 7,44, 2H, and 7,55, 2H + 2H, PhH;

### Example 2

### 1-(4-Trifluoromethyl-benzyl)-piperidin-4-one (compound 01-82)

A mixture of 8-(4-trifluoromethyl-benzyl)-1,4-dioxa-8-aza-spiro[4.5]decane (0,90g 3,0 mmol) and hydrochloric acid (0,89g 9,0 mmol HCl) in water (10 mL) were heated to reflux for 8h. After extractive workup with heptane-ethylacetate 1:1 and sodiumhydroxide (12 mmol as 6N NaOH, 2 mL) a light coloured oil was obtained, 0,63g, purity 95%, compound 01-82.
¹H-NMR (ppm): 2,46, 4H, CH₂CO; 2,75, 4H, NCH₂; 3,67, 2H, NCH₂Ph; 7,50 and 7,59, 2H + 2H, PhH;

### Example 3

### 1-(4-Trifluoromethyl-benzyl)-piperidin-4-ol (compound 14-82)

A mixture of 1-(4-Trifluoromethyl-benzyl)-piperidin-4-one (1,50g 5,8 mmol) and sodium borohydride (0,14g 3,5 mmol) in ethanol (20 mL) were heated to reflux for 5h. After extractive workup with heptane-ethylacetate 1:1 and water a light coloured solid was obtained, 1,17g, purity 91 %, compound 14-82.
¹H-NMR (ppm): 1,60, 2H; 1,76, 1H, OH; 1,89, 2H; 2,16, 2H; 2,74, 2H; 3,53, 2H, NCH₂Ph; 3,71, 1 H, CHOH; 7,44 and 7,56, 4H, PhH;

### Example 4

### Propionic acid 1-(4-Trifluoromethyl-benzyl)-piperidin-4-yl ester (compound 16-82)

A mixture of 1-(4-Trifluoromethyl-benzyl)-piperidin-4-ol (0,25g 1,0 mmol), DMAP (12mg) and propionic acid anhydride (0,19g 1,4 mmol) in dioxane (5 mL) were heated to reflux for 6 h. After extractive workup with heptane-ethylacetate 1:1 and water an oil was obtained, 0,27g, purity 90%, compound 16-82.
¹H-NMR (ppm): 1,13, 3H; 1,71, 2H; 1,89, 2H; 2,28, 2H; 2,31, 2H; 2,66, 2H; 3,53, 2H; 4,81, 1 H; 7,44 and 7,56, 4H;

### Example 5

### 1-(4-Chloro-benzyl)-4-methoxy-piperidin (compound 12-11)

A mixture of 1-(4-chlorobenzyl)-piperidin-4-ol (0,5g 2,2 mmol), sodium hydride (0,13g, 60%, 3,3 mmol) and methyliodide (0,38g 2,6 mmol) in DMF (5 mL) were stirred at 35°C for 20 h. After extractive workup with heptane-ethylacetate 1:1 and water and column chromatography (ethylacetate) an oil was obtained, 0,05g, purity 96%, compound 12-11.
¹H-NMR (ppm): 1,57, 2H; 1,87, 2H; 2,12, 2H; 2,68, 2H; 3,20, 1 H, CH-OMe; 3,32, 3H, OCH₃; 3,43, 2H, NCH2Ph; 7,24, 4H, PhH;

### Example 6

### 8-(4-Trifluoromethyl-benzyl)-8-aza-bicyclo[3.2.1]octan-3-one (compound 07-82)

A mixture of 2,5-dimethoxytetrahydrofuran (1,36g 10,3 mmol) and hydrochloric acid (2,25g, 37%, 22,8 mmol) in water 20 mL were stirred at 20°C for 1 h. Then 3-oxo-pentanedioic acid (1,50g 10,3 mmol), 4-trifluoromethyl-benzylamine (2,00g 11,4 mmol) and sodium acetate (2,81g 34,3 mmol) were added and heated to 60°C for 2 h. After extractive workup with heptane-ethylacetate 1:1 and water and column chromatography (heptane-ethylacetate 1:1) an oil was obtained, 1,53, purity >96%, compound 07-82.
¹H-NMR (ppm): 1,66, 2H; 2,13, 2H; 2,23, 2H; 2,68, 2H; 3,47, 2H; 3,80, 2H, NCH₂Ph; 7,55 and 7,60, 4H, PhH;

### Example 7

### 2-Benzyl-1,2,3,4-decahydro-isoquinoline (compound 19-01)

A mixture of Decahydroisochinoline (0,60g 4,3 mmol), benzylbromide (0,74g 4,3 mmol) and diisopropylethylamine (0,66g 5,1 mmol) in dioxane (5 mL) was heated to reflux for 7 h. After extractive workup an oil was obtained, 0,84 g, compound 19-01, 2 isomers (3,5:1).
¹H-NMR (ppm): 0,92 to 2,87, 16H; 3,43, 2H; 7,28, 5H;

### Example 8

### 2-(4-Trifluoromethyl-benzyl)-1,2,3,4-tetrahydro-isoquinoline (compound 21-82)

A mixture of 1,2,3,4-Tetrahydroisochinoline (0,27g 2,0 mmol), 4-bromomethylbenzotrifluoride (0,48g 2,0 mmol) and diisopropylethylamine (0,31g 2,4 mmol) in dioxane (5 mL) was heated to reflux for 7 h. After extractive workup and column chromatography an oil was obtained, 0,37 g, compound 21-82 .
¹H-NMR (ppm): 2,74, 2H; 2,89, 2H; 3,63, 2H, 3,71, 2H; 6,97, 1H; 7,10, 3H; 7,52, 2H; 7,58, 2H;

### Example 9

### 2-(6-Fluoropyridin-2-yl)-1,2,3,4-tetrahydro-isoquinoline (compound 23-05)

A mixture of 1,2,3,4-Tetrahydroisochinoline (0,30g 2,25 mmol), 2,6-difluoropyridine (0,26g 2,25 mmol) and diisopropylethylamine (0,38g 2,9 mmol) in dioxane (10 mL) was heated to reflux for 12 h. After extractive workup a solid was obtained, 0,26 g, compound 23-05 .
¹H-NMR (ppm): 2,95, 2H; 3,80, 2H; 4,66, 2H,NCH₂; 6,14, 1 H, 6,43, 1 H and 7,53, 1 H, Py-H; 7,18, 4H, PhH;

### Tables:

The following preferred compounds shown in Tables 1 to 24 also form part of the present invention, and were or may be prepared in accordance with, or analogously to, the above-mentioned Examples 1 to 9 or the above-described general methods.

Where subscripts are omitted they are intended, for example CH2 means CH₂. In the Tables Me means methyl, Et means ethyl, Pr means propyl, Bu means butyl, C5H11 means n-pentyl, C6H13 means n-hexyl, C2H4 means ethylene (-CH₂CH₂-), cC3H5 means cyclopropyl- moiety.
¹H-NMR spectra shift values are given in ppm.
"Cpd No" means Compound Number. Compound numbers are given for reference purposes only. "CA REG No" means Chemical Abstract Registration Number.

**Table 1: Compounds of Formula (Ia) in which the substituents have the following meanings: R¹ is Phenyl substituted by R; (A)n is CH2; R² and R³ are each H; X is O;**

| Compound number | | R | CA REG No | NMR |
|---|---|---|---|---|
| 01- | 01 | H | 3612-20-2 | |
| 01- | 02 | 2-F | 374926-47-3 | |
| 01- | 03 | 2-Cl | 135576-51-1 | |
| 01- | 04 | 2-Br | | |
| 01- | 05 | 2-I | | |
| 01- | 06 | 3-F | 155868-53-4 | |
| 01- | 07 | 3-Cl | 247206-81-1 | |
| 01- | 08 | 3-Br | | |
| 01- | 09 | 3-I | | |
| 01- | 10 | 4-F | 134348-51-9 | |
| 01- | 11 | 4-Cl | 21937-61-1 | |
| 01- | 12 | 4-Br | | 1 H: 2,44; 2,72; 3,55; 7,24; 7,45; |
| 01- | 13 | 4-I | | |
| 01- | 14 | 2-Me | 76167-41-4 | |
| 01- | 15 | 2-Et | | |
| 01- | 16 | 2-nPr | | |
| 01- | 17 | 2-iPr | | |
| 01- | 18 | 2-nBu | | |
| 01- | 19 | 2-sec-Bu | | |
| 01- | 20 | 2-iBu | | |
| 01- | 21 | 2-tBu | | |
| 01- | 22 | 2-CF3 | | |
| 01- | 23 | 2-MeO | 562840-43-1 | |
| 01- | 24 | 2-EtO | | |
| 01- | 25 | 2-nPrO | | |
| 01- | 26 | 2-nBuO | | |
| 01- | 27 | 2-OCHF2 | | |
| 01- | 28 | 2-OCF3 | | |
| 01- | 29 | 2-OCH2CF3 | | |
| 01- | 30 | 2-OC2F4H | | |
| 01- | 31 | 2-OC3F6H | | |
| 01- | 32 | 2-SMe | | |
| 01- | 33 | 2-SOMe | | |
| 01- | 34 | 2-SO2Me | | |
| 01- | 35 | 2-SEt | | |
| 01- | 36 | 2-SOEt | | |
| 01- | 37 | 2-SO2Et | | |
| 01- | 38 | 2-SCF3 | | |
| 01- | 39 | 2-SOCF3 | | |
| 01- | 40 | 2-SO2CF3 | | |
| 01- | 41 | 2-CN | | |
| 01- | 42 | 2-NO2 | | |
| 01- | 43 | 2-NMe2 | | |
| 01- | 44 | 3-Me | 343778-60-9 | |
| 01- | 45 | 3-Et | | |
| 01- | 46 | 3-nPr | | |
| 01- | 47 | 3-iPr | | |
| 01- | 48 | 3-nBu | | |
| 01- | 49 | 3-sec-Bu | | |
| 01- | 50 | 3-iBu | | |
| 01- | 51 | 3-tBu | | |
| 01- | 52 | 3-CF3 | 321601-36-9 | |
| 01- | 53 | 3-MeO | 163341-33-1 | |
| 01- | 54 | 3-EtO | | |
| 01- | 55 | 3-nPrO | | |
| 01- | 56 | 3-nBuO | | |
| 01- | 57 | 3-OCHF2 | | |
| 01- | 58 | 3-OCF3 | 662110-99-8 | |
| 01- | 59 | 3-OCH2CF3 | | |
| 01- | 60 | 3-OC2F4H | | |
| 01- | 61 | 3-OC3F6H | | |
| 01- | 62 | 3-SMe | | |
| 01- | 63 | 3-SOMe | | |
| 01- | 64 | 3-SO2Me | | |
| 01- | 65 | 3-SEt | | |
| 01- | 66 | 3-SOEt | | |
| 01- | 67 | 3-SO2Et | | |
| 01- | 68 | 3-SCF3 | | |
| 01- | 69 | 3-SOCF3 | | |
| 01- | 70 | 3-SO2CF3 | | |
| 01- | 71 | 3-CN | | |
| 01- | 72 | 3-NO2 | | |
| 01- | 73 | 3-NMe2 | | |
| 01- | 74 | 4-Me | 241495-46-5 | |
| 01- | 75 | 4-Et | | |
| 01- | 76 | 4-nPr | | |
| 01- | 77 | 4-iPr | | |
| 01- | 78 | 4-nBu | | |
| 01- | 79 | 4-sec-Bu | | |
| 01- | 80 | 4-iBu | | |
| 01- | 81 | 4-tBu | | |
| 01- | 82 | 4-CF3 | | 1 H: 2,46, 2,75, 3,67, 7,50, 7,59 |
| 01- | 83 | 4-MeO | | |
| 01- | 84 | 4-EtO | | |
| 01- | 85 | 4-nPrO | | |
| 01- | 86 | 4-nBuO | | |
| 01- | 87 | 4-OCHF2 | | |
| 01- | 88 | 4-OCF3 | | |
| 01- | 89 | 4-OCH2CF3 | | |
| 01- | 90 | 4-OC2F4H | | |
| 01- | 91 | 4-OC3F6H | | |
| 01- | 92 | 4-SMe | | |
| 01- | 93 | 4-SOMe | | |
| 01- | 94 | 4-SO2Me | | |
| 01- | 95 | 4-SEt | | |
| 01- | 96 | 4-SOEt | | |
| 01- | 97 | 4-SO2Et | | |
| 01- | 98 | 4-SCF3 | | |
| 01- | 99 | 4-SOCF3 | | |
| 01- | 100 | 4-SO2CF3 | | |
| 01- | 101 | 4-CN | | |
| 01- | 102 | 4-NO2 | | |
| 01- | 103 | 4-NMe2 | | |
| 01- | 104 | 2,3-F2 | | |
| 01- | 105 | 2,4-F2 | | 1 H: 2,43; 2,74; 3,64; 6,79; 6,84; 7,37; |
| 01- | 106 | 2,5-F2 | | |
| 01- | 107 | 2,6-F2 | | |
| 01- | 108 | 3,4-F2 | | |
| 01- | 109 | 3,5-F2 | | |
| 01- | 110 | 2,3-Cl2 | | |
| 01- | 111 | 2,4-Cl2 | | 1 H: 2,46; 2,80; 3,68; 7,25; 7,38; 7,47; |
| 01- | 112 | 2,5-Cl2 | | |
| 01- | 113 | 2,6-Cl2 | 439096-19-2 | |
| 01- | 114 | 3,4-Cl2 | 220772-52-1 | |
| 01- | 115 | 3,5-Cl2 | | |
| 01- | 116 | 2-Cl-4-CF3 | | |
| 01- | 117 | 2-Cl-4-F | | |
| 01- | 118 | 2,3-Me2 | | |
| 01- | 119 | 2,4-Me2 | | |
| 01- | 120 | 2,5-Me2 | | |
| 01- | 121 | 2,6-Me2 | | |
| 01- | 122 | 3,4-Me2 | 617714-65-5 | |
| 01- | 123 | 3,5-Me2 | | |
| 01- | 124 | 2,4,6-F3 | | |
| 01- | 125 | 3,4,5-F3 | | |
| 01- | 126 | 2,4,6-Cl3 | | |
| 01- | 127 | 2,3,4-Cl3 | | |
| 01- | 128 | 2,4,5-Cl3 | | |
| 01- | 129 | 3,4,5-Cl3 | | |
| 01- | 130 | 2,6-Cl2-4-CF3 | | |
| 01- | 131 | 2,4,6-Me3 | | |
| 01- | 132 | 2,4-Br2 | | |
| 01- | 133 | 3,4-Br2 | | |
| 01- | 134 | 2-Cl-4-Br | | |
| 01- | 135 | 2-Br-4-F | | |

**Table 2: Compounds of Formula (la) in which the substituents have the following meanings: R¹ is Phenyl substituted by R; (A)n is C2H4 or CH(CH3); R² and R³ are each H; X is O;**

| Compound number | | (A)n | R | CA REG No | NMR |
|---|---|---|---|---|---|
| 02- | 01 | C2H4 | H | 39742-60-4 | |
| 02- | 02 | C2H4 | 2-F | | |
| 02- | 03 | C2H4 | 2-Cl | 39742-61-5 | |
| 02- | 04 | C2H4 | 2-Br | | |
| 02- | 05 | C2H4 | 2-I | | |
| 02- | 06 | C2H4 | 3-F | | |
| 02- | 07 | C2H4 | 3-Cl | | |
| 02- | 08 | C2H4 | 3-Br | | |
| 02- | 09 | C2H4 | 3-I | | |
| 02- | 10 | C2H4 | 4-F | 23808-43-7 | |
| 02- | 11 | C2H4 | 4-Cl | 39742-62-6 | |
| 02- | 12 | C2H4 | 4-Br | | |
| 02- | 13 | C2H4 | 4-I | | |
| 02- | 14 | C2H4 | 4-Me | | |
| 02- | 15 | C2H4 | 4-Et | | |
| 02- | 16 | C2H4 | 4-nPr | | |
| 02- | 17 | C2H4 | 4-iPr | | |
| 02- | 18 | C2H4 | 4-nBu | | |
| 02- | 19 | C2H4 | 4-sec-Bu | | |
| 02- | 20 | C2H4 | 4-iBu | | |
| 02- | 21 | C2H4 | 4-tBu | | |
| 02- | 22 | C2H4 | 4-CF3 | | |
| 02- | 23 | C2H4 | 4-MeO | 39742-63-7 | |
| 02- | 24 | C2H4 | 4-EtO | | |
| 02- | 25 | C2H4 | 4-nPrO | | |
| 02- | 26 | C2H4 | 4-nBuO | | |
| 02- | 27 | C2H4 | 4-OCHF2 | | |
| 02- | 28 | C2H4 | 4-OCF3 | | |
| 02- | 29 | C2H4 | 4-OCH2CF3 | | |
| 02- | 30 | C2H4 | 4-OC2F4H | | |
| 02- | 31 | C2H4 | 4-OC3F6H | | |
| 02- | 32 | C2H4 | 4-SMe | | |
| 02- | 33 | C2H4 | 4-SOMe | | |
| 02- | 34 | C2H4 | 4-SO2Me | | |
| 02- | 35 | C2H4 | 4-SEt | | |
| 02- | 36 | C2H4 | 4-SOEt | | |
| 02- | 37 | C2H4 | 4-SO2Et | | |
| 02- | 38 | C2H4 | 4-SCF3 | | |
| 02- | 39 | C2H4 | 4-SOCF3 | | |
| 02- | 40 | C2H4 | 4-SO2CF3 | | |
| 02- | 41 | C2H4 | 4-CN | | |
| 02- | 42 | C2H4 | 4-NO2 | | |
| 02- | 43 | C2H4 | 4-NMe2 | | |
| 02- | 44 | C2H4 | 2,3-F2 | | |
| 02- | 45 | C2H4 | 2,4-F2 | | |
| 02- | 46 | C2H4 | 2,5-F2 | | |
| 02- | 47 | C2H4 | 2,6-F2 | | |
| 02- | 48 | C2H4 | 3,4-F2 | | |
| 02- | 49 | C2H4 | 3,5-F2 | | |
| 02- | 50 | C2H4 | 2,3-Cl2 | | |
| 02- | 51 | C2H4 | 2,4-Cl2 | | |
| 02- | 52 | C2H4 | 2,5-Cl2 | | |
| 02- | 53 | C2H4 | 2,6-Cl2 | | |
| 02- | 54 | C2H4 | 3,4-Cl2 | | |
| 02- | 55 | C2H4 | 3,5-Cl2 | | |
| 02- | 56 | C2H4 | 2-Cl-4-CF3 | | |
| 02- | 57 | C2H4 | 2-Cl-4-F | | |
| 02- | 58 | CH(CH3) | H | 36482-37-8 | |
| 02- | 59 | CH(CH3) | 2-F | | |
| 02- | 60 | CH(CH3) | 2-Cl | | |
| 02- | 61 | CH(CH3) | 2-Br | | |
| 02- | 62 | CH(CH3) | 2-I | | |
| 02- | 63 | CH(CH3) | 3-F | | |
| 02- | 64 | CH(CH3) | 3-Cl | | |
| 02- | 65 | CH(CH3) | 3-Br | | |
| 02- | 66 | CH(CH3) | 3-I | | |
| 02- | 67 | CH(CH3) | 4-F | | |
| 02- | 68 | CH(CH3) | 4-Cl | | |
| 02- | 69 | CH(CH3) | 4-Br | | |
| 02- | 70 | CH(CH3) | 4-I | | |
| 02- | 71 | CH(CH3) | 4-Me | | |
| 02- | 72 | CH(CH3) | 4-Et | | |
| 02- | 73 | CH(CH3) | 4-nPr | | |
| 02- | 74 | CH(CH3) | 4-iPr | | |
| 02- | 75 | CH(CH3) | 4-nBu | | |
| 02- | 76 | CH(CH3) | 4-sec-Bu | | |
| 02- | 77 | CH(CH3) | 4-iBu | | |
| 02- | 78 | CH(CH3) | 4-tBu | | |
| 02- | 79 | CH(CH3) | 4-CF3 | | |
| 02- | 80 | CH(CH3) | 4-MeO | | |
| 02- | 81 | CH(CH3) | 4-EtO | | |
| 02- | 82 | CH(CH3) | 4-nPrO | | |
| 02- | 83 | CH(CH3) | 4-nBuO | | |
| 02- | 84 | CH(CH3) | 4-OCHF2 | | |
| 02- | 85 | CH(CH3) | 4-OCF3 | | |
| 02- | 86 | CH(CH3) | 4-OCH2CF3 | | |
| 02- | 87 | CH(CH3) | 4-OC2F4H | | |
| 02- | 88 | CH(CH3) | 4-OC3F6H | | |
| 02- | 89 | CH(CH3) | 4-SMe | | |
| 02- | 90 | CH(CH3) | 4-SOMe | | |
| 02- | 91 | CH(CH3) | 4-SO2Me | | |
| 02- | 92 | CH(CH3) | 4-SEt | | |
| 02- | 93 | CH(CH3) | 4-SOEt | | |
| 02- | 94 | CH(CH3) | 4-SO2Et | | |
| 02- | 95 | CH(CH3) | 4-SCF3 | | |
| 02- | 96 | CH(CH3) | 4-SOCF3 | | |
| 02- | 97 | CH(CH3) | 4-SO2CF3 | | |
| 02- | 98 | CH(CH3) | 4-CN | | |
| 02- | 99 | CH(CH3) | 4-NO2 | | |
| 02- | 100 | CH(CH3) | 4-NMe2 | | |
| 02- | 101 | CH(CH3) | 2,3-F2 | | |
| 02- | 102 | CH(CH3) | 2,4-F2 | | |
| 02- | 103 | CH(CH3) | 2,5-F2 | | |
| 02- | 104 | CH(CH3) | 2,6-F2 | | |
| 02- | 105 | CH(CH3) | 3,4-F2 | | |
| 02- | 106 | CH(CH3) | 3,5-F2 | | |
| 02- | 107 | CH(CH3) | 2,3-Cl2 | | |
| 02- | 108 | CH(CH3) | 2,4-Cl2 | | |
| 02- | 109 | CH(CH3) | 2,5-Cl2 | | |
| 02- | 110 | CH(CH3) | 2,6-Cl2 | | |
| 02- | 111 | CH(CH3) | 3,4-Cl2 | | |
| 02- | 112 | CH(CH3) | 3,5-Cl2 | | |
| 02- | 113 | CH(CH3) | 2-Cl-4-CF3 | | |
| 02- | 114 | CH(CH3) | 2-Cl-4-F | | |
| 02- | 115 | CH(C2H5) | H | | |
| 02- | 116 | CH(C2H5) | 2-F | | |
| 02- | 117 | CH(C2H5) | 2-Cl | | |
| 02- | 118 | CH(C2H5) | 2-Br | | |
| 02- | 119 | CH(C2H5) | 2-I | | |
| 02- | 120 | CH(C2H5) | 3-F | | |
| 02- | 121 | CH(C2H5) | 3-Cl | | |
| 02- | 122 | CH(C2H5) | 3-Br | | |
| 02- | 123 | CH(C2H5) | 3-I | | |
| 02- | 124 | CH(C2H5) | 4-F | | |
| 02- | 125 | CH(C2H5) | 4-Cl | | |
| 02- | 126 | CH(C2H5) | 4-Br | | |
| 02- | 127 | CH(C2H5) | 4-I | | |

**Table 3: Compounds of Formula (Ia) in which the substituents have the following meanings: R¹ is Phenyl substituted by R; (A)n is C3H6 or CH(CH3)CH2; R² and R³ are each H; X isO ;**

| Compound number | | (A)n | R | CA REG No | NMR |
|---|---|---|---|---|---|
| 03- | 01 | C3H6 | H | 107100-64-1 | |
| 03- | 02 | C3H6 | 2-F | | |
| 03- | 03 | C3H6 | 2-Cl | | |
| 03- | 04 | C3H6 | 2-Br | | |
| 03- | 05 | C3H6 | 2-I | | |
| 03- | 06 | C3H6 | 3-F | | |
| 03- | 07 | C3H6 | 3-Cl | | |
| 03- | 08 | C3H6 | 3-Br | | |
| 03- | 09 | C3H6 | 3-I | | |
| 03- | 10 | C3H6 | 4-F | | |
| 03- | 11 | C3H6 | 4-Cl | | |
| 03- | 12 | C3H6 | 4-Br | | |
| 03- | 13 | C3H6 | 4-I | | |
| 03- | 14 | C3H6 | 4-Me | | |
| 03- | 15 | C3H6 | 4-Et | | |
| 03- | 16 | C3H6 | 4-nPr | | |
| 03- | 17 | C3H6 | 4-iPr | | |
| 03- | 18 | C3H6 | 4-nBu | | |
| 03- | 19 | C3H6 | 4-sec-Bu | | |
| 03- | 20 | C3H6 | 4-iBu | | |
| 03- | 21 | C3H6 | 4-tBu | | |
| 03- | 22 | C3H6 | 4-CF3 | | |
| 03- | 23 | C3H6 | 4-MeO | | |
| 03- | 24 | C3H6 | 4-EtO | | |
| 03- | 25 | C3H6 | 4-nPrO | | |
| 03- | 26 | C3H6 | 4-nBuO | | |
| 03- | 27 | C3H6 | 4-OCHF2 | | |
| 03- | 28 | C3H6 | 4-OCF3 | | |
| 03- | 29 | C3H6 | 4-OCH2CF3 | | |
| 03- | 30 | C3H6 | 4-OC2F4H | | |
| 03- | 31 | C3H6 | 4-OC3F6H | | |
| 03- | 32 | C3H6 | 4-SMe | | |
| 03- | 33 | C3H6 | 4-SOMe | | |
| 03- | 34 | C3H6 | 4-SO2Me | | |
| 03- | 35 | C3H6 | 4-SEt | | |
| 03- | 36 | C3H6 | 4-SOEt | | |
| 03- | 37 | C3H6 | 4-SO2Et | | |
| 03- | 38 | C3H6 | 4-SCF3 | | |
| 03- | 39 | C3H6 | 4-SOCF3 | | |
| 03- | 40 | C3H6 | 4-SO2CF3 | | |
| 03- | 41 | C3H6 | 4-CN | | |
| 03- | 42 | C3H6 | 4-NO2 | | |
| 03- | 43 | C3H6 | 4-NMe2 | | |
| 03- | 44 | C3H6 | 2,3-F2 | | |
| 03- | 45 | C3H6 | 2,4-F2 | | |
| 03- | 46 | C3H6 | 2,5-F2 | | |
| 03- | 47 | C3H6 | 2,6-F2 | | |
| 03- | 48 | C3H6 | 3,4-F2 | | |
| 03- | 49 | C3H6 | 3,5-F2 | | |
| 03- | 50 | C3H6 | 2,3-Cl2 | | |
| 03- | 51 | C3H6 | 2,4-Cl2 | | |
| 03- | 52 | C3H6 | 2,5-Cl2 | | |
| 03- | 53 | C3H6 | 2,6-Cl2 | | |
| 03- | 54 | C3H6 | 3,4-Cl2 | | |
| 03- | 55 | C3H6 | 3,5-Cl2 | | |
| 03- | 56 | C3H6 | 2-Cl-4-CF3 | | |
| 03- | 57 | C3H6 | 2-Cl-4-F | | |
| 03- | 58 | CH(CH3)CH2 | H | | |
| 03- | 59 | CH(CH3)CH2 | 2-F | | |
| 03- | 60 | CH(CH3)CH2 | 2-Cl | | |
| 03- | 61 | CH(CH3)CH2 | 2-Br | | |
| 03- | 62 | CH(CH3)CH2 | 2-I | | |
| 03- | 63 | CH(CH3)CH2 | 3-F | | |
| 03- | 64 | CH(CH3)CH2 | 3-Cl | | |
| 03- | 65 | CH(CH3)CH2 | 3-Br | | |
| 03- | 66 | CH(CH3)CH2 | 3-I | | |
| 03- | 67 | CH(CH3)CH2 | 4-F | | |
| 03- | 68 | CH(CH3)CH2 | 4-Cl | | |
| 03- | 69 | CH(CH3)CH2 | 4-Br | | |
| 03- | 70 | CH(CH3)CH2 | 4-I | | |
| 03- | 71 | CH(CH3)CH2 | 4-Me | | |
| 03- | 72 | CH(CH3)CH2 | 4-Et | | |
| 03- | 73 | CH(CH3)CH2 | 4-nPr | | |
| 03- | 74 | CH(CH3)CH2 | 4-iPr | | |
| 03- | 75 | CH(CH3)CH2 | 4-nBu | | |
| 03- | 76 | CH(CH3)CH2 | 4-sec-Bu | | |
| 03- | 77 | CH(CH3)CH2 | 4-iBu | | |
| 03- | 78 | CH(CH3)CH2 | 4-tBu | | |
| 03- | 79 | CH(CH3)CH2 | 4-CF3 | | |
| 03- | 80 | CH(CH3)CH2 | 4-MeO | | |
| 03- | 81 | CH(CH3)CH2 | 4-EtO | | |
| 03- | 82 | CH(CH3)CH2 | 4-nPrO | | |
| 03- | 83 | CH(CH3)CH2 | 4-nBuO | | |
| 03- | 84 | CH(CH3)CH2 | 4-OCHF2 | | |
| 03- | 85 | CH(CH3)CH2 | 4-OCF3 | | |
| 03- | 86 | CH(CH3)CH2 | 4-OCH2CF3 | | |
| 03- | 87 | CH(CH3)CH2 | 4-OC2F4H | | |
| 03- | 88 | CH(CH3)CH2 | 4-OC3F6H | | |
| 03- | 89 | CH(CH3)CH2 | 4-SMe | | |
| 03- | 90 | CH(CH3)CH2 | 4-SOMe | | |
| 03- | 91 | CH(CH3)CH2 | 4-SO2Me | | |
| 03- | 92 | CH(CH3)CH2 | 4-SEt | | |
| 03- | 93 | CH(CH3)CH2 | 4-SOEt | | |
| 03- | 94 | CH(CH3)CH2 | 4-SO2Et | | |
| 03- | 95 | CH(CH3)CH2 | 4-SCF3 | | |
| 03- | 96 | CH(CH3)CH2 | 4-SOCF3 | | |
| 03- | 97 | CH(CH3)CH2 | 4-SO2CF3 | | |
| 03- | 98 | CH(CH3)CH2 | 4-CN | | |
| 03- | 99 | CH(CH3)CH2 | 4-NO2 | | |
| 03- | 100 | CH(CH3)CH2 | 4-NMe2 | | |
| 03- | 101 | CH(CH3)CH2 | 2,3-F2 | | |
| 03- | 102 | CH(CH3)CH2 | 2,4-F2 | | |
| 03- | 103 | CH(CH3)CH2 | 2,5-F2 | | |
| 03- | 104 | CH(CH3)CH2 | 2,6-F2 | | |
| 03- | 105 | CH(CH3)CH2 | 3,4-F2 | | |
| 03- | 106 | CH(CH3)CH2 | 3,5-F2 | | |
| 03- | 107 | CH(CH3)CH2 | 2,3-Cl2 | | |
| 03- | 108 | CH(CH3)CH2 | 2,4-Cl2 | | |
| 03- | 109 | CH(CH3)CH2 | 2,5-Cl2 | | |
| 03- | 110 | CH(CH3)CH2 | 2,6-Cl2 | | |
| 03- | 111 | CH(CH3)CH2 | 3,4-Cl2 | | |
| 03- | 112 | CH(CH3)CH2 | 3,5-Cl2 | | |
| 03- | 113 | CH(CH3)CH2 | 2-Cl-4-CF3 | | |
| 03- | 114 | CH(CH3)CH2 | 2-Cl-4-F | | |
| 03- | 115 | CH2CH(CH3) | H | | |
| 03- | 116 | CH2CH(CH3) | 2-F | | |
| 03- | 117 | CH2CH(CH3) | 2-Cl | | |
| 03- | 118 | CH2CH(CH3) | 2-Br | | |
| 03- | 119 | CH2CH(CH3) | 2-I | | |
| 03- | 120 | CH2CH(CH3) | 3-F | | |
| 03- | 121 | CH2CH(CH3) | 3-Cl | | |
| 03- | 122 | CH2CH(CH3) | 3-Br | | |
| 03- | 123 | CH2CH(CH3) | 3-I | | |
| 03- | 124 | CH2CH(CH3) | 4-F | | |
| 03- | 125 | CH2CH(CH3) | 4-Cl | | |
| 03- | 126 | CH2CH(CH3) | 4-Br | | |
| 03- | 127 | CH2CH(CH3) | 4-I | | |

**Table 4: Compounds of Formula (Ia) in which the substituents have the following meanings: R¹ is substituted pyridine; (A)n is CH2; R² and R³ are each H; X is O;**

| Compound number | | R1 | CA REG No | NMR |
|---|---|---|---|---|
| 04- | 01 | 2-pyridyl | | |
| 04- | 02 | 3-F-2-pyridyl | | |
| 04- | 03 | 4-F-2-pyridyl | | |
| 04- | 04 | 5-F-2-pyridyl | | |
| 04- | 05 | 6-F-2-pyridyl | | |
| 04- | 06 | 3-Cl-2-pyridyl | | |
| 04- | 07 | 4-Cl-2-pyridyl | | |
| 04- | 08 | 5-Cl-2-pyridyl | | |
| 04- | 09 | 6-Cl-2-pyridyl | | |
| 04- | 10 | 3-Br-2-pyridyl | | |
| 04- | 11 | 4-Br-2-pyridyl | | |
| 04- | 12 | 5-Br-2-pyridyl | | |
| 04- | 13 | 6-Br-2-pyridyl | | |
| 04- | 14 | 3-I-2-pyridyl | | |
| 04- | 15 | 4-I-2-pyridyl | | |
| 04- | 16 | 5-I-2-pyridyl | | |
| 04- | 17 | 6-I-2-pyridyl | | |
| 04- | 18 | 3-CF3-2-pyridyl | | |
| 04- | 19 | 4-CF3-2-pyridyl | | |
| 04- | 20 | 5-CF3-2-pyridyl | | |
| 04- | 21 | 6-CF3-2-pyridyl | | |
| 04- | 22 | 3-Me-2-pyridyl | | |
| 04- | 23 | 4-Me-2-pyridyl | | |
| 04- | 24 | 5-Me-2-pyridyl | | |
| 04- | 25 | 6-Me-2-pyridyl | | |
| 04- | 26 | 3,5-Cl2-2-pyridyl | | |
| 04- | 27 | 3,5-Br2-2-pyridyl | | |
| 04- | 28 | 3-Cl-5-CF3-2-pyridyl | | |
| 04- | 29 | 3,5-Me-2-pyridyl | | |
| 04- | 30 | 3-pyridyl | | |
| 04- | 31 | 2-Cl-3-pyridyl | | |
| 04- | 32 | 4-Cl-3-pyridyl | | |
| 04- | 33 | 5-Cl-3-pyridyl | | |
| 04- | 34 | 6-Cl-3-pyridyl | | 1 H: 2,46; 2,74;3,60; 7,32; 7,70; 8,34; |
| 04- | 35 | 2,5-Cl2-3-pyridyl | | |
| 04- | 36 | 4-pyridyl | | |
| 04- | 37 | 2-Cl-4-pyridyl | | |
| 04- | 38 | 3-Cl-4-pyridyl | | |
| 04- | 39 | 3,5-Cl2-4-pyridyl | | |
| 04- | 40 | 6-MeO-2-pyridyl | | |
| 04- | 41 | 6-EtO-2-pyridyl | | |
| 04- | 42 | 6-CF3CH2O-2-pyridyl | | |
| 04- | 43 | 6-CHF2O-2-pyridyl | | |
| 04- | 44 | 6-MeO-3-pyridyl | | |
| 04- | 45 | 3-EtO-3-pyridyl | | |
| 04- | 46 | 6-CF3CH2O-3-pyridyl | | |
| 04- | 47 | 6-CHF2O-3-pyridyl | | |
| 04- | 48 | 2-pyrimidinyl | | |
| 04- | 49 | 4-pyrimidinyl | | |
| 04- | 50 | 4,6-Me2-2-pyrimidinyl | | |
| 04- | 51 | 2,6-Me2-4-pyrimidinyl | | |
| 04- | 52 | 2-pyrazinyl | | |
| 04- | 53 | 3-pyridazinyl | | |

**Table 5: Compounds of Formula (Ia) in which the substituents have the following meanings: R¹ is Phenyl substituted by R; n=0 in (A)n; R² and R³ are each H; X is O;**

| Compound number | | R | CA REG No | NMR |
|---|---|---|---|---|
| 05- | 01 | H | 19125-34-9 | |
| 05- | 02 | 2-F | 115012-46-9 | |
| 05- | 03 | 2-Cl | | |
| 05- | 04 | 2-Br | | |
| 05- | 05 | 2-I | | |
| 05- | 06 | 3-F | | |
| 05- | 07 | 3-Cl | | |
| 05- | 08 | 3-Br | | |
| 05- | 09 | 3-I | | |
| 05- | 10 | 4-F | | |
| 05- | 11 | 4-Cl | 113759-96-9 | |
| 05- | 12 | 4-Br | | |
| 05- | 13 | 4-I | | |
| 05- | 14 | 2-Me | | |
| 05- | 15 | 2-Et | | |
| 05- | 16 | 2-nPr | | |
| 05- | 17 | 2-iPr | | |
| 05- | 18 | 2-nBu | | |
| 05- | 19 | 2-sec-Bu | | |
| 05- | 20 | 2-iBu | | |
| 05- | 21 | 2-tBu | | |
| 05- | 22 | 2-CF3 | | |
| 05- | 23 | 2-MeO | | |
| 05- | 24 | 2-EtO | | |
| 05- | 25 | 2-nPrO | | |
| 05- | 26 | 2-nBuO | | |
| 05- | 27 | 2-OCHF2 | | |
| 05- | 28 | 2-OCF3 | | |
| 05- | 29 | 2-OCH2CF3 | | |
| 05- | 30 | 2-OC2F4H | | |
| 05- | 31 | 2-OC3F6H | | |
| 05- | 32 | 2-SMe | | |
| 05- | 33 | 2-SOMe | | |
| 05- | 34 | 2-SO2Me | | |
| 05- | 35 | 2-SEt | | |
| 05- | 36 | 2-SOEt | | |
| 05- | 37 | 2-SO2Et | | |
| 05- | 38 | 2-SCF3 | | |
| 05- | 39 | 2-SOCF3 | | |
| 05- | 40 | 2-SO2CF3 | | |
| 05- | 41 | 2-CN | | |
| 05- | 42 | 2-NO2 | | |
| 05- | 43 | 2-NMe2 | | |
| 05- | 44 | 3-Me | | |
| 05- | 45 | 3-Et | | |
| 05- | 46 | 3-nPr | | |
| 05- | 47 | 3-iPr | | |
| 05- | 48 | 3-nBu | | |
| 05- | 49 | 3-sec-Bu | | |
| 05- | 50 | 3-iBu | | |
| 05- | 51 | 3-tBu | | |
| 05- | 52 | 3-CF3 | | |
| 05- | 53 | 3-MeO | | |
| 05- | 54 | 3-EtO | | |
| 05- | 55 | 3-nPrO | | |
| 05- | 56 | 3-nBuO | | |
| 05- | 57 | 3-OCHF2 | | |
| 05- | 58 | 3-OCF3 | | |
| 05- | 59 | 3-OCH2CF3 | | |
| 05- | 60 | 3-OC2F4H | | |
| 05- | 61 | 3-OC3F6H | | |
| 05- | 62 | 3-SMe | | |
| 05- | 63 | 3-SOMe | | |
| 05- | 64 | 3-SO2Me | | |
| 05- | 65 | 3-SEt | | |
| 05- | 66 | 3-SOEt | | |
| 05- | 67 | 3-SO2Et | | |
| 05- | 68 | 3-SCF3 | | |
| 05- | 69 | 3-SOCF3 | | |
| 05- | 70 | 3-SO2CF3 | | |
| 05- | 71 | 3-CN | | |
| 05- | 72 | 3-NO2 | | |
| 05- | 73 | 3-NMe2 | | |
| 05- | 74 | 4-Me | 105123-89-5 | |
| 05- | 75 | 4-Et | | |
| 05- | 76 | 4-nPr | | |
| 05- | 77 | 4-iPr | | |
| 05- | 78 | 4-nBu | | |
| 05- | 79 | 4-sec-Bu | | |
| 05- | 80 | 4-iBu | | |
| 05- | 81 | 4-tBu | | |
| 05- | 82 | 4-CF3 | | |
| 05- | 83 | 4-MeO | 94635-24-2 | |
| 05- | 84 | 4-EtO | | |
| 05- | 85 | 4-nPrO | | |
| 05- | 86 | 4-nBuO | | |
| 05- | 87 | 4-OCHF2 | | |
| 05- | 88 | 4-OCF3 | | |
| 05- | 89 | 4-OCH2CF3 | | |
| 05- | 90 | 4-OC2F4H | | |
| 05- | 91 | 4-OC3F6H | | |
| 05- | 92 | 4-SMe | | |
| 05- | 93 | 4-SOMe | | |
| 05- | 94 | 4-SO2Me | | |
| 05- | 95 | 4-SEt | | |
| 05- | 96 | 4-SOEt | | |
| 05- | 97 | 4-SO2Et | | |
| 05- | 98 | 4-SCF3 | | |
| 05- | 99 | 4-SOCF3 | | |
| 05- | 100 | 4-SO2CF3 | | |
| 05- | 101 | 4-CN | | |
| 05- | 102 | 4-NO2 | | |
| 05- | 103 | 4-NMe2 | | |
| 05- | 104 | 2,3-F2 | | |
| 05- | 105 | 2,4-F2 | | |
| 05- | 106 | 2,5-F2 | | |
| 05- | 107 | 2,6-F2 | | |
| 05- | 108 | 3,4-F2 | | |
| 05- | 109 | 3,5-F2 | | |
| 05- | 110 | 2,3-Cl2 | | |
| 05- | 111 | 2,4-Cl2 | | |
| 05- | 112 | 2,5-Cl2 | | |
| 05- | 113 | 2,6-Cl2 | | |
| 05- | 114 | 3,4-Cl2 | | |
| 05- | 115 | 3,5-Cl2 | | |
| 05- | 116 | 2-Cl-4-CF3 | | |
| 05- | 117 | 2-Cl-4-F | | |
| 05- | 118 | 2,3-Me2 | | |
| 05- | 119 | 2,4-Me2 | | |
| 05- | 120 | 2,5-Me2 | | |
| 05- | 121 | 2,6-Me2 | | |
| 05- | 122 | 3,4-Me2 | | |
| 05- | 123 | 3,5-Me2 | | |
| 05- | 124 | 2,4,6-F3 | | |
| 05- | 125 | 3,4,5-F3 | | |
| 05- | 126 | 2,4,6-Cl3 | | |
| 05- | 127 | 2,3,4-Cl3 | | |
| 05- | 128 | 2,4,5-Cl3 | | |
| 05- | 129 | 3,4,5-Cl3 | | |
| 05- | 130 | 2,6-Cl2-4-CF3 | | |
| 05- | 131 | 2,4,6-Me3 | | |
| 05- | 132 | 2,4-Br2 | | |
| 05- | 133 | 3,4-Br2 | | |
| 05- | 134 | 2-Cl-4-Br | | |
| 05- | 135 | 2-Br-4-F | | |

**Table 6: Compounds of Formula (Ia) in which the substituents have the following meanings: R¹ is optionally substituted pyridine, pyrimidine, pyrazine and pyridazine; n=0 in (A)n; R² and R³ are each H; X is O;**

| Compound number | | R1 | CA REG No | NMR |
|---|---|---|---|---|
| 06- | 01 | 2-pyridyl | | |
| 06- | 02 | 3-F-2-pyridyl | | |
| 06- | 03 | 4-F-2-pyridyl | | |
| 06- | 04 | 5-F-2-pyridyl | | |
| 06- | 05 | 6-F-2-pyridyl | | |
| 06- | 06 | 3-Cl-2-pyridyl | | |
| 06- | 07 | 4-Cl-2-pyridyl | | |
| 06- | 08 | 5-Cl-2-pyridyl | | |
| 06- | 09 | 6-Cl-2-pyridyl | | |
| 06- | 10 | 3-Br-2-pyridyl | | |
| 06- | 11 | 4-Br-2-pyridyl | | |
| 06- | 12 | 5-Br-2-pyridyl | | |
| 06- | 13 | 6-Br-2-pyridyl | | |
| 06- | 14 | 3-1-2-pyridyl | | |
| 06- | 15 | 4-l-2-pyridyl | | |
| 06- | 16 | 5-l-2-pyridyl | | |
| 06- | 17 | 6-I-2-pyridyl | | |
| 06- | 18 | 3-CF3-2-pyridyl | | |
| 06- | 19 | 4-CF3-2-pyridyl | | |
| 06- | 20 | 5-CF3-2-pyridyl | | 1 H: 2,48; 3,92; 6,68; 7,62; 8,37; |
| 06- | 21 | 6-CF3-2-pyridyl | | |
| 06- | 22 | 3-Me-2-pyridyl | | |
| 06- | 23 | 4-Me-2-pyridyl | | |
| 06- | 24 | 5-Me-2-pyridyl | | |
| 06- | 25 | 6-Me-2-pyridyl | | |
| 06- | 26 | 3,5-Cl2-2-pyridyl | | |
| 06- | 27 | 3,5-Br2-2-pyridyl | | |
| 06- | 28 | 3-Cl-5-CF3-2-pyridyl | | |
| 06- | 29 | 3,5-Me-2-pyridyl | | |
| 06- | 30 | 3-pyridyl | | |
| 06- | 31 | 2-Cl-3-pyridyl | | |
| 06- | 32 | 4-Cl-3-pyridyl | | |
| 06- | 33 | 5-Cl-3-pyridyl | | |
| 06- | 34 | 6-Cl-3-pyridyl | | |
| 06- | 35 | 2,5-Cl2-3-pyridyl | | |
| 06- | 36 | 4-pyridyl | | |
| 06- | 37 | 2-Cl-4-pyridyl | | |
| 06- | 38 | 3-Cl-4-pyridyl | | |
| 06- | 39 | 3,5-Cl2-4-pyridyl | | |
| 06- | 40 | 6-MeO-2-pyridyl | | |
| 06- | 41 | 6-EtO-2-pyridyl | | |
| 06- | 42 | 6-CF3CH2O-2-pyridyl | | |
| 06- | 43 | 6-CHF2O-2-pyridyl | | |
| 06- | 44 | 6-MeO-3-pyridyl | | |
| 06- | 45 | 3-EtO-3-pyridyl | | |
| 06- | 46 | 6-CF3CH2O-3-pyridyl | | |
| 06- | 47 | 6-CHF2O-3-pyridyl | | |
| 06- | 48 | 6-CF3CH2O-2-pyridyl | | |
| 06- | 49 | 6-CHF2O-2-pyridyl | | |
| 06- | 50 | 2-pyrimidinyl | | |
| 06- | 51 | 4-pyrimidinyl | | |
| 06- | 52 | 4,6-Me2-2-pyrimidinyl | | |
| 06- | 53 | 2,6-Me2-4-pyrimidinyl | | |
| 06- | 54 | 2-pyrazinyl | | |
| 06- | 55 | 3-pyridazinyl | | |

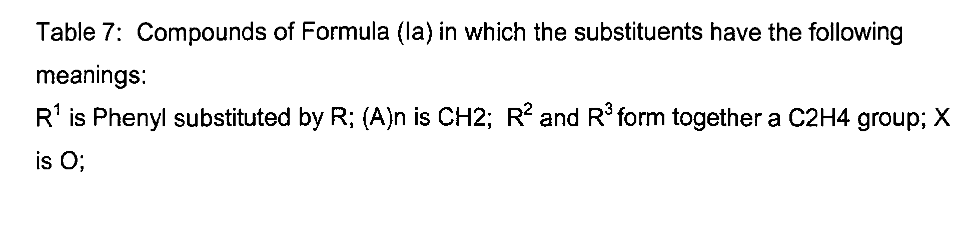

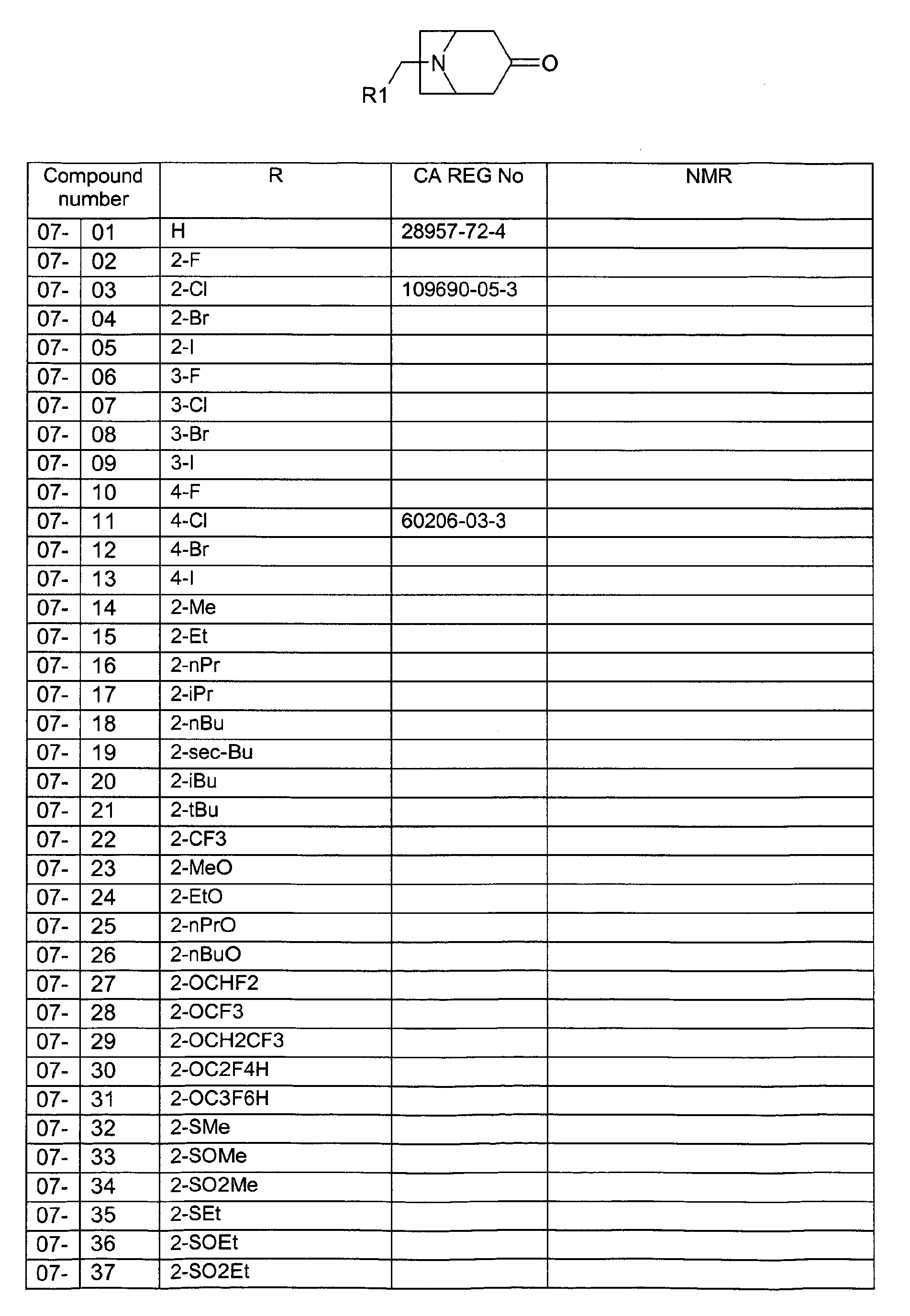

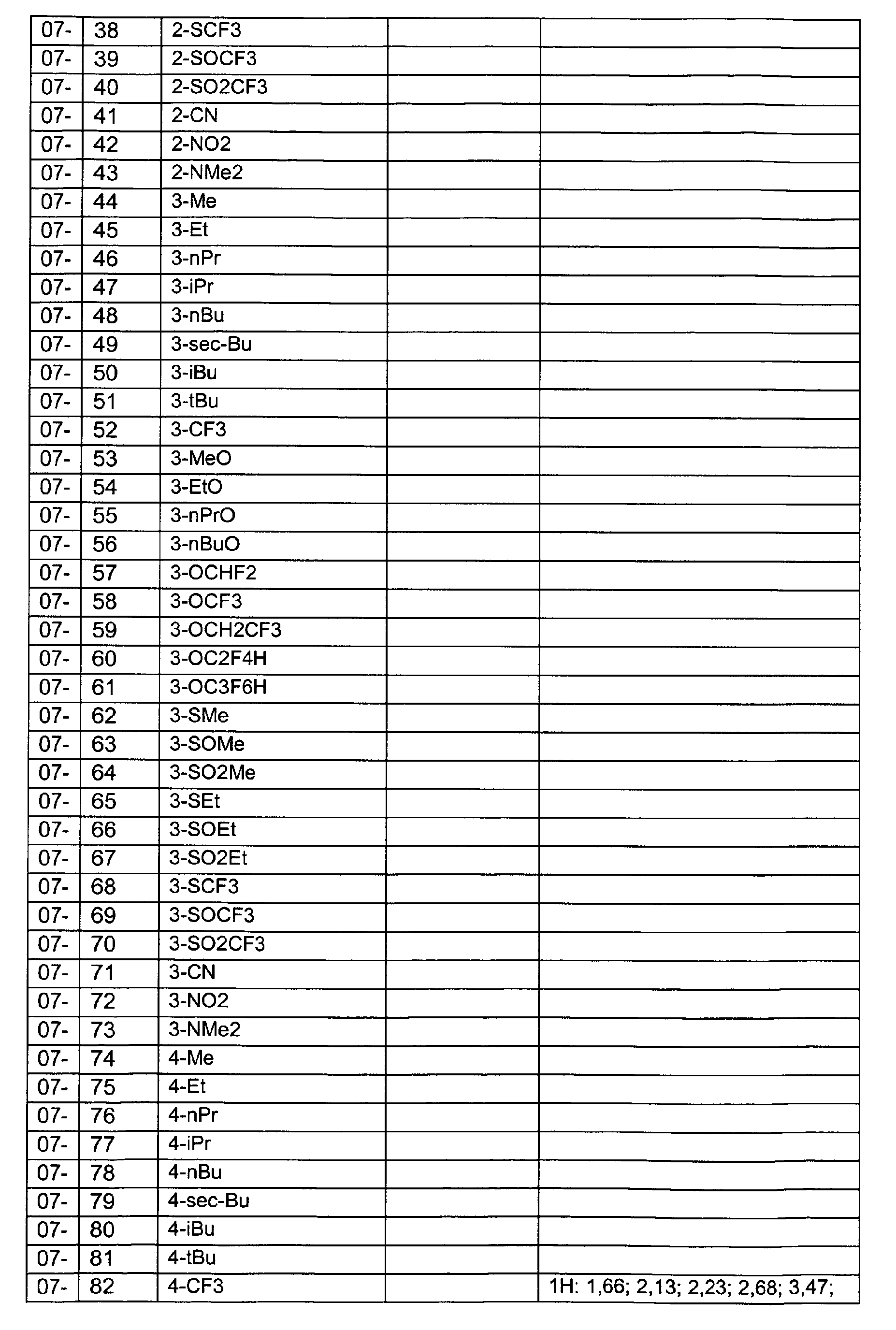

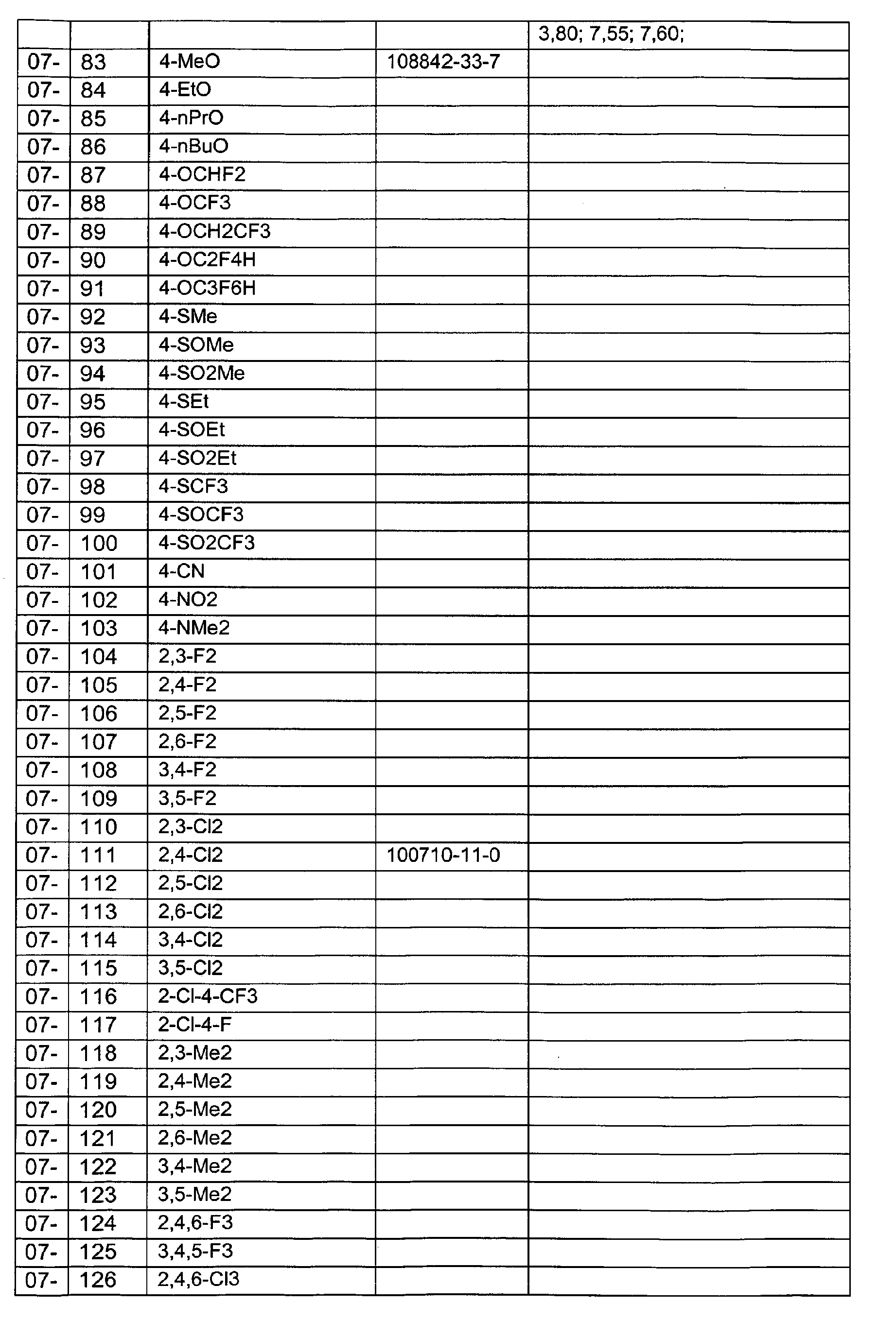

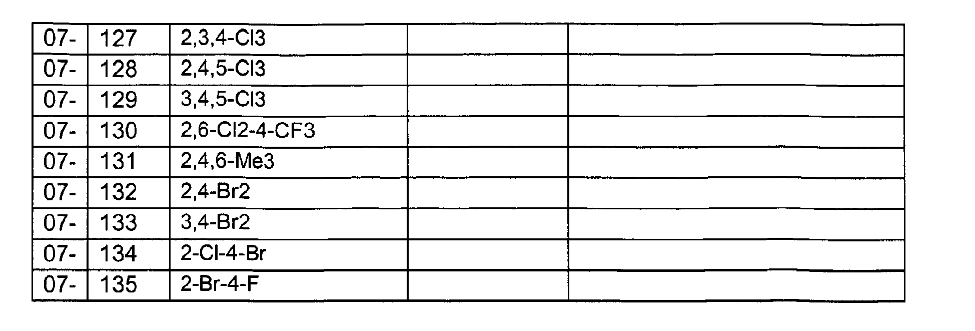

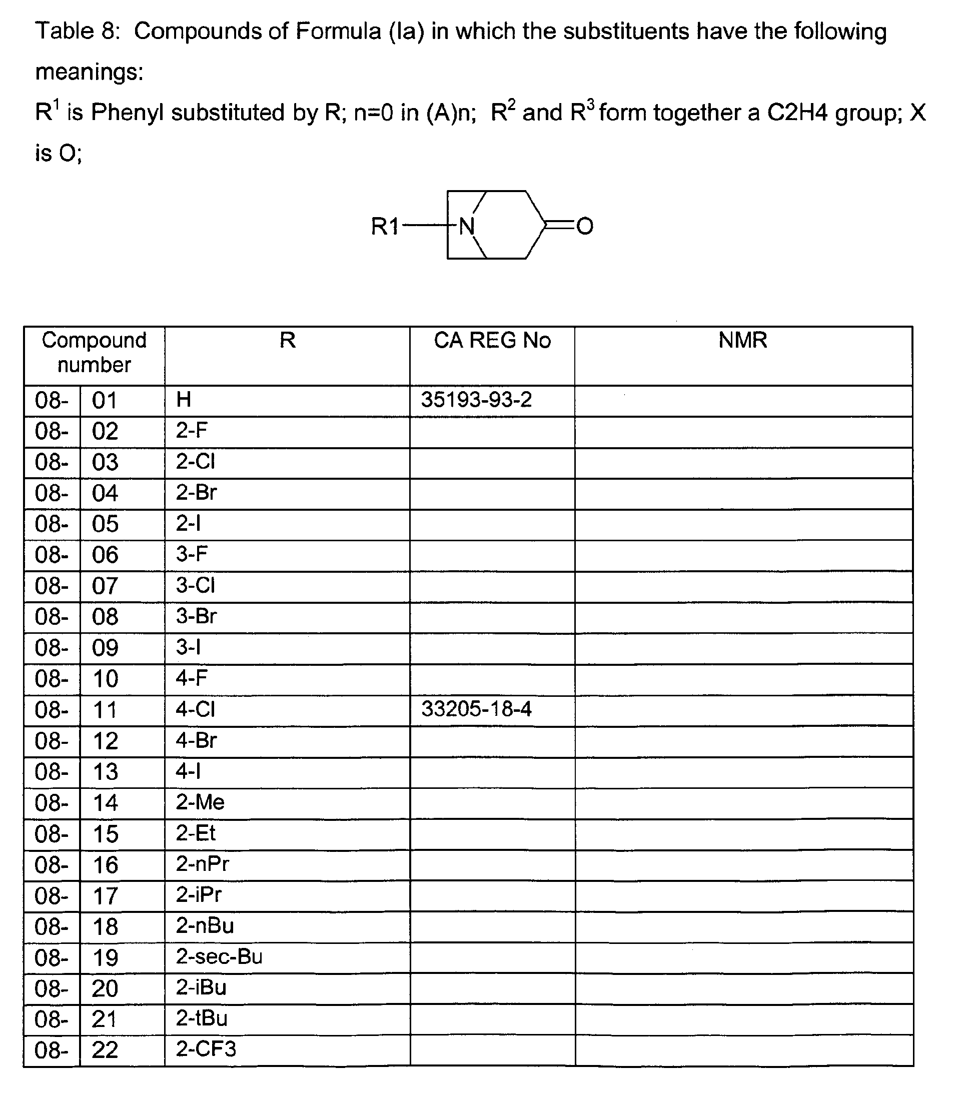

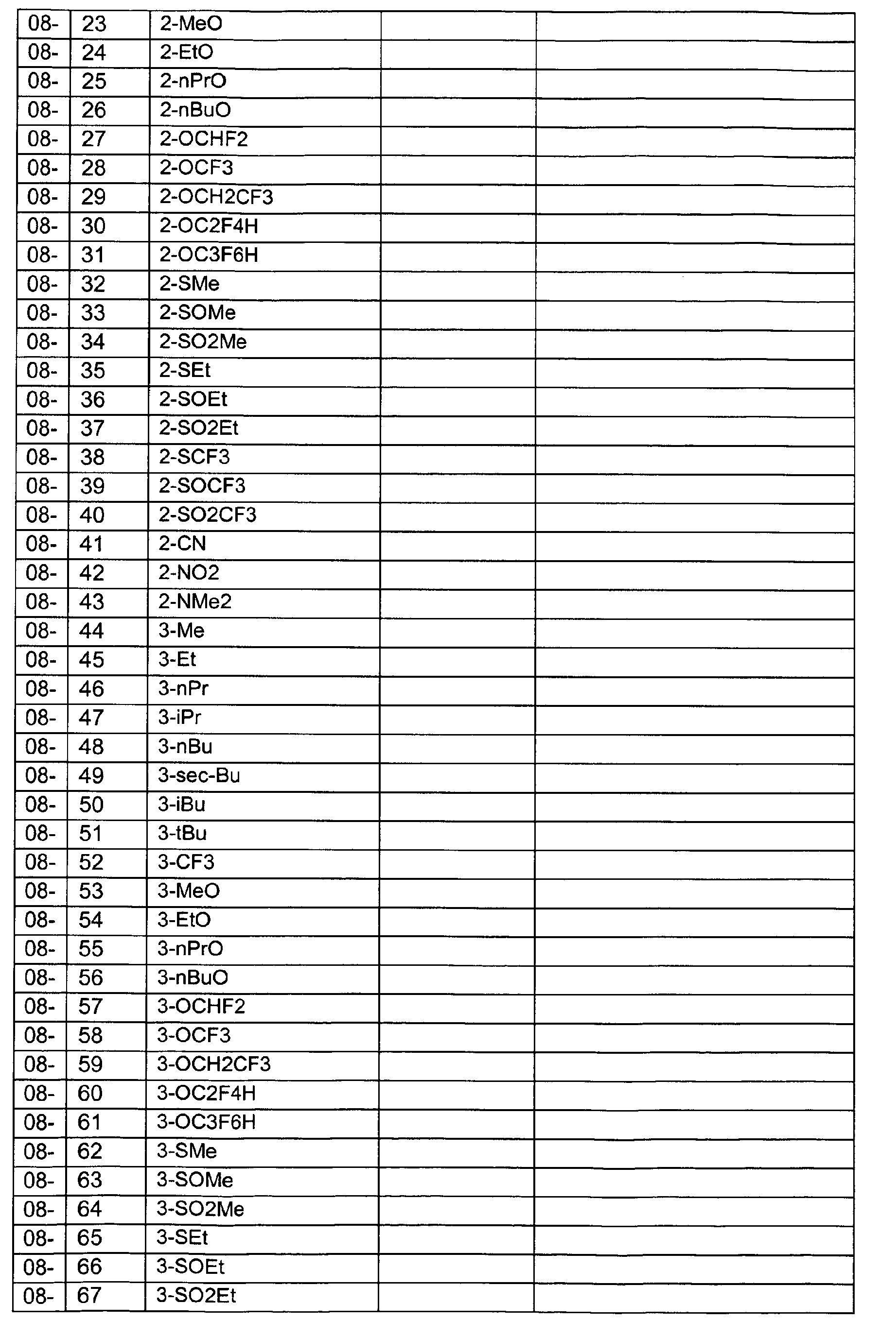

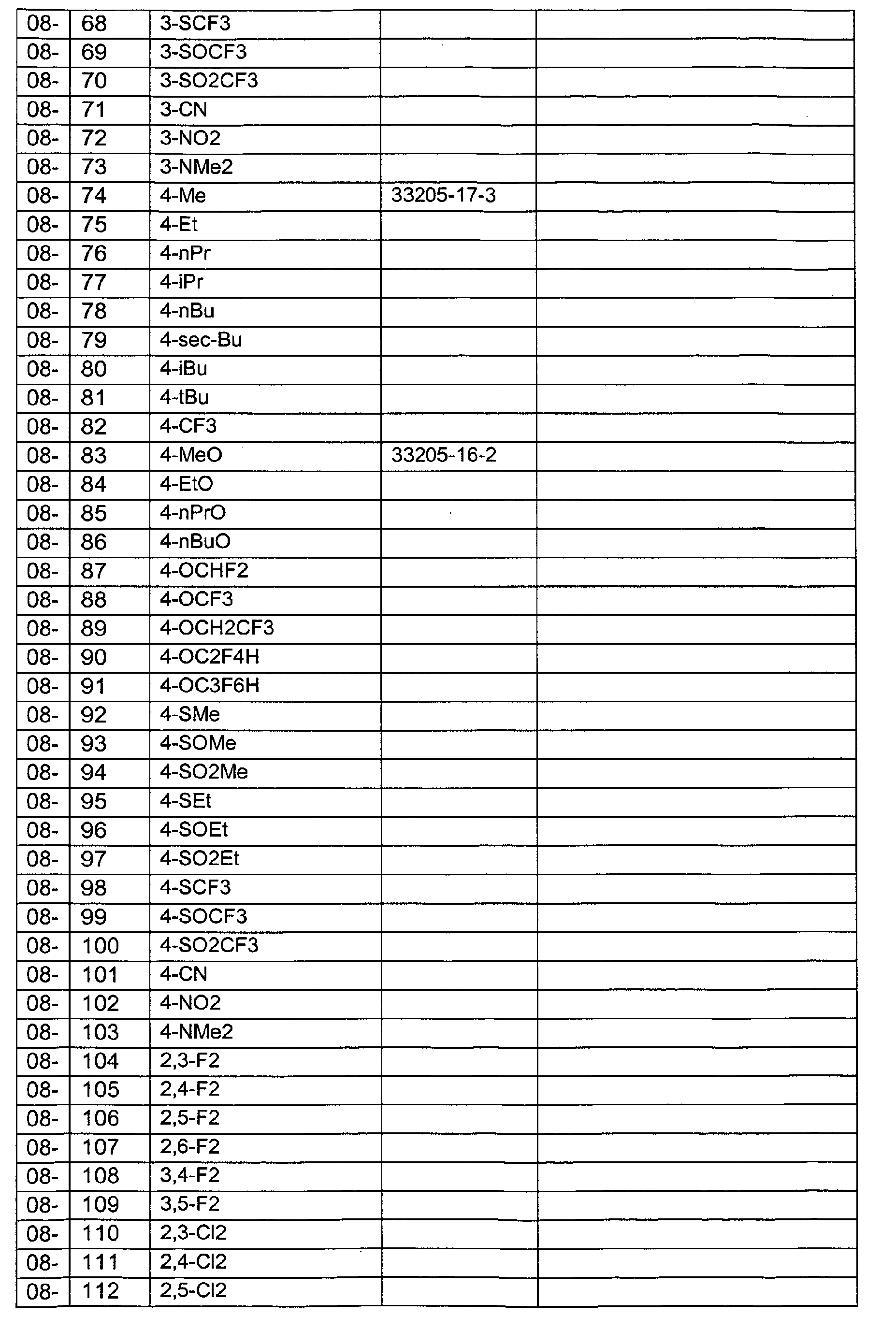

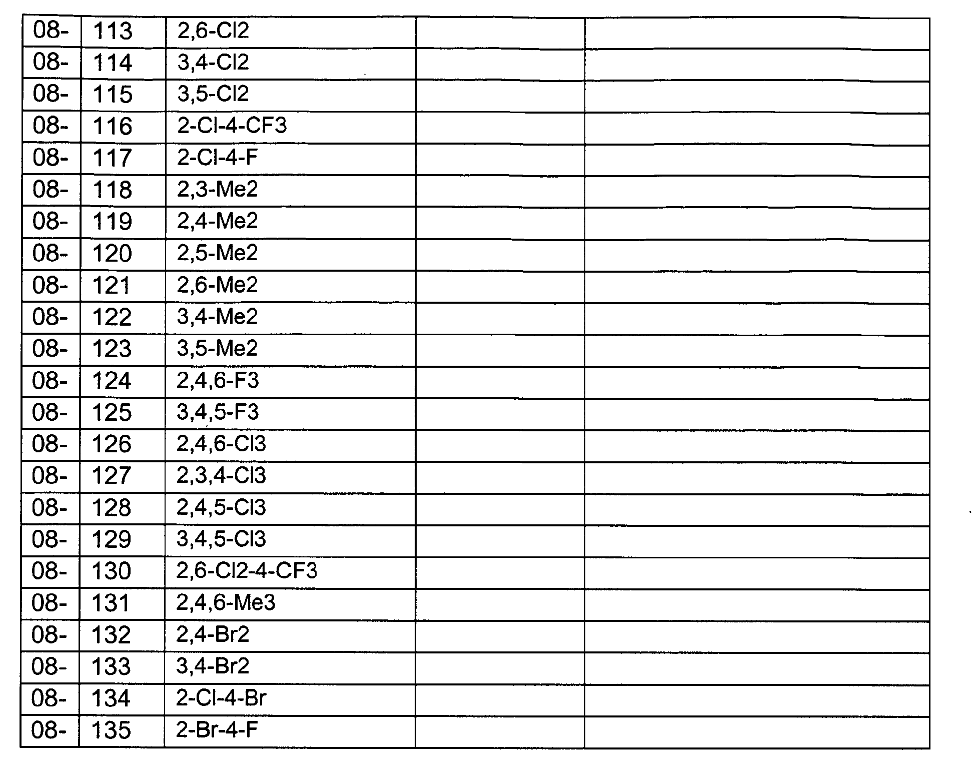

**Table 9: Compounds of Formula (la) in which the substituents have the following meanings: R¹-(A)n- is (C₄-C₉)-alkyl, (C₃-C₇)-cycloalkyl, (C₃-C₇)-cycloalkylenyl; R² and R³ are each H; X is O;**

| Compound number | | R1-A | CA REG No | NMR |
|---|---|---|---|---|
| 09- | 01 | nC4H9 | 23081-86-9 | |
| 09- | 02 | nC5H11 | 72544-07-1 | |
| 09- | 03 | nC6H13 | 71072-22-5 | |
| 09- | 04 | nC7H15 | | 1H: 0,81; 1,22; 1,45; 2,39; 2,66; |
| 09- | 05 | nC8H17 | | |
| 09- | 06 | nC9H19 | | |
| 09- | 07 | c-C3H5 | | |
| 09- | 08 | c-C4H7 | | |
| 09- | 09 | c-C5H9 | | |
| 09- | 10 | c-C6H11 | | |
| 09- | 11 | c-C7H13 | | |
| 09- | 12 | cyclopent-2-enyl | | |
| 09- | 13 | cyclopent-3-enyl | | |
| 09- | 14 | cyclohex-2-enyl | | |
| 09- | 15 | cyclohex-3-enyl | | |
| 09- | 16 | c-C3H5-CH2 | 49682-96-4 | |
| 09- | 17 | c-C4H7-CH2 | | |
| 09- | 18 | c-C5H9-CH2 | | |
| 09- | 19 | c-C6H11-CH2 | 64306-76-9 | |
| 09- | 20 | c-C7H13-CH2 | | |

**Table 10: Compounds of Formula (Ia) in which the substituents have the following meanings: R¹ is Phenyl substituted by R; (A)n is CH2; R² and R³ are each H; X is N-OR;**

| Compound number | | X | R | CA REG No | NMR |
|---|---|---|---|---|---|
| 10- | 01 | N-OH | H | | |
| 10- | 02 | N-OH | 2-F | | |
| 10- | 03 | N-OH | 2-Cl | | |
| 10- | 04 | N-OH | 2-Br | | |
| 10- | 05 | N-OH | 2-I | | |
| 10- | 06 | N-OH | 3-F | | |
| 10- | 07 | N-OH | 3-Cl | | |
| 10- | 08 | N-OH | 3-Br | | |
| 10- | 09 | N-OH | 3-I | | |
| 10- | 10 | N-OH | 4-F | | |
| 10- | 11 | N-OH | 4-Cl | | |
| 10- | 12 | N-OH | 4-Br | | |
| 10- | 13 | N-OH | 4-I | | |
| 10- | 14 | N-OH | 4-Me | | |
| 10- | 15 | N-OH | 4-Et | | |
| 10- | 16 | N-OH | 4-nPr | | |
| 10- | 17 | N-OH | 4-iPr | | |
| 10- | 18 | N-OH | 4-nBu | | |
| 10- | 19 | N-OH | 4-sec-Bu | | |
| 10- | 20 | N-OH | 4-iBu | | |
| 10- | 21 | N-OH | 4-tBu | | |
| 10- | 22 | N-OH | 4-CF3 | | |
| 10- | 23 | N-OH | 4-MeO | | |
| 10- | 24 | N-OH | 4-EtO | | |
| 10- | 25 | N-OH | 4-nPrO | | |
| 10- | 26 | N-OH | 4-nBuO | | |
| 10- | 27 | N-OH | 4-OCHF2 | | |
| 10- | 28 | N-OH | 4-OCF3 | | |
| 10- | 29 | N-OH | 4-OCH2CF3 | | |
| 10- | 30 | N-OH | 4-OC2F4H | | |
| 10- | 31 | N-OH | 4-OC3F6H | | |
| 10- | 32 | N-OH | 4-SMe | | |
| 10- | 33 | N-OH | 4-SOMe | | |
| 10- | 34 | N-OH | 4-SO2Me | | |
| 10- | 35 | N-OH | 4-SEt | | |
| 10- | 36 | N-OH | 4-SOEt | | |
| 10- | 37 | N-OH | 4-SO2Et | | |
| 10- | 38 | N-OH | 4-SCF3 | | |
| 10- | 39 | N-OH | 4-SOCF3 | | |
| 10- | 40 | N-OH | 4-SO2CF3 | | |
| 10- | 41 | N-OH | 4-CN | | |
| 10- | 42 | N-OH | 4-NO2 | | |
| 10- | 43 | N-OH | 4-NMe2 | | |
| 10- | 44 | N-OMe | H | | |
| 10- | 45 | N-OMe | 2-F | | |
| 10- | 46 | N-OMe | 2-Cl | | |
| 10- | 47 | N-OMe | 2-Br | | |
| 10- | 48 | N-OMe | 2-I | | |
| 10- | 49 | N-OMe | 3-F | | |
| 10- | 50 | N-OMe | 3-Cl | | |
| 10- | 51 | N-OMe | 3-Br | | |
| 10- | 52 | N-OMe | 3-I | | |
| 10- | 53 | N-OMe | 4-F | | |
| 10- | 54 | N-OMe | 4-Cl | | |
| 10- | 55 | N-OMe | 4-Br | | |
| 10- | 56 | N-OMe | 4-I | | |
| 10- | 57 | N-OMe | 4-Me | | |
| 10- | 58 | N-OMe | 4-Et | | |
| 10- | 59 | N-OMe | 4-nPr | | |
| 10- | 60 | N-OMe | 4-iPr | | |
| 10- | 61 | N-OMe | 4-nBu | | |
| 10- | 62 | N-OMe | 4-sec-Bu | | |
| 10- | 63 | N-OMe | 4-iBu | | |
| 10- | 64 | N-OMe | 4-tBu | | |
| 10- | 65 | N-OMe | 4-CF3 | | |
| 10- | 66 | N-OMe | 4-MeO | | |
| 10- | 67 | N-OMe | 4-EtO | | |
| 10- | 68 | N-OMe | 4-nPrO | | |
| 10- | 69 | N-OMe | 4-nBuO | | |
| 10- | 70 | N-OMe | 4-OCHF2 | | |
| 10- | 71 | N-OMe | 4-OCF3 | | |
| 10- | 72 | N-OMe | 4-OCH2CF3 | | |
| 10- | 73 | N-OMe | 4-OC2F4H | | |
| 10- | 74 | N-OMe | 4-OC3F6H | | |
| 10- | 75 | N-OMe | 4-SMe | | |
| 10- | 76 | N-OMe | 4-SOMe | | |
| 10- | 77 | N-OMe | 4-SO2Me | | |
| 10- | 78 | N-OMe | 4-SEt | | |
| 10- | 79 | N-OMe | 4-SOEt | | |
| 10- | 80 | N-OMe | 4-SO2Et | | |
| 10- | 81 | N-OMe | 4-SCF3 | | |
| 10- | 82 | N-OMe | 4-SOCF3 | | |
| 10- | 83 | N-OMe | 4-SO2CF3 | | |
| 10- | 84 | N-OMe | 4-CN | | |
| 10- | 85 | N-OMe | 4-NO2 | | |
| 10- | 86 | N-OMe | 4-NMe2 | | |
| 10- | 87 | N-OEt | H | | |
| 10- | 88 | N-OEt | 2-F | | |
| 10- | 89 | N-OEt | 2-Cl | | |
| 10- | 90 | N-OEt | 2-Br | | |
| 10- | 91 | N-OEt | 2-I | | |
| 10- | 92 | N-OEt | 3-F | | |
| 10- | 93 | N-OEt | 3-Cl | | |
| 10- | 94 | N-OEt | 3-Br | | |
| 10- | 95 | N-OEt | 3-I | | |
| 10- | 96 | N-OEt | 4-F | | |
| 10- | 97 | N-OEt | 4-Cl | | |
| 10- | 98 | N-OEt | 4-Br | | |
| 10- | 99 | N-OEt | 4-I | | |
| 10- | 100 | N-OEt | 4-Me | | |
| 10- | 101 | N-OEt | 4-Et | | |
| 10- | 102 | N-OEt | 4-nPr | | |
| 10- | 103 | N-OEt | 4-iPr | | |
| 10- | 104 | N-OEt | 4-nBu | | |
| 10- | 105 | N-OEt | 4-sec-Bu | | |
| 10- | 106 | N-OEt | 4-iBu | | |
| 10- | 107 | N-OEt | 4-tBu | | |
| 10- | 108 | N-OEt | 4-CF3 | | |
| 10- | 109 | N-OEt | 4-MeO | | |
| 10- | 110 | N-OEt | 4-EtO | | |
| 10- | 111 | N-OEt | 4-nPrO | | |
| 10- | 112 | N-OEt | 4-nBuO | | |
| 10- | 113 | N-OEt | 4-OCHF2 | | |
| 10- | 114 | N-OEt | 4-OCF3 | | |
| 10- | 115 | N-OEt | 4-OCH2CF3 | | |
| 10- | 116 | N-OEt | 4-OC2F4H | | |
| 10- | 117 | N-OEt | 4-OC3F6H | | |
| 10- | 118 | N-OEt | 4-SMe | | |
| 10- | 119 | N-OEt | 4-SOMe | | |
| 10- | 120 | N-OEt | 4-SO2Me | | |
| 10- | 121 | N-OEt | 4-SEt | | |
| 10- | 122 | N-OEt | 4-SOEt | | |
| 10- | 123 | N-OEt | 4-SO2Et | | |
| 10- | 124 | N-OEt | 4-SCF3 | | |
| 10- | 125 | N-OEt | 4-SOCF3 | | |
| 10- | 126 | N-OEt | 4-SO2CF3 | | |
| 10- | 127 | N-OEt | 4-CN | | |
| 10- | 128 | N-OEt | 4-NO2 | | |
| 10- | 129 | N-OEt | 4-NMe2 | | |

**Table 11: Compounds of Formula (Ib) in which the substituents have the following meanings: R¹ is Phenyl substituted by R; (A)n is CH2; R² and R³ are each H; R⁴ and R⁵ together with C4 of the piperidine ring form a 5-6 membered ring;**

| Compound number | | R | R4-R5 | CA REG No | NMR |
|---|---|---|---|---|---|
| 11- | 01 | H | O-C2H4-O | 37943-54-7 | |
| 11- | 02 | 2-F | O-C2H4-O | | |
| 11- | 03 | 2-Cl | O-C2H4-O | | |
| 11- | 04 | 2-Br | O-C2H4-O | | |
| 11- | 05 | 2-I | O-C2H4-O | | |
| 11- | 06 | 3-F | O-C2H4-O | | |
| 11- | 07 | 3-Cl | O-C2H4-O | | |
| 11- | 08 | 3-Br | O-C2H4-O | | |
| 11- | 09 | 3-I | O-C2H4-O | | |
| 11- | 10 | 4-F | O-C2H4-O | | |
| 11- | 11 | 4-Cl | O-C2H4-O | | |
| 11- | 12 | 4-Br | O-C2H4-O | | |
| 11- | 13 | 4-I | O-C2H4-O | | |
| 11- | 14 | 4-Me | O-C2H4-O | | |
| 11- | 15 | 4-Et | O-C2H4-O | | |
| 11- | 16 | 4-nPr | O-C2H4-O | | |
| 11- | 17 | 4-iPr | O-C2H4-O | | |
| 11- | 18 | 4-nBu | O-C2H4-O | | |
| 11- | 19 | 4-sec-Bu | O-C2H4-O | | |
| 11- | 20 | 4-iBu | O-C2H4-O | | |
| 11- | 21 | 4-tBu | O-C2H4-O | | |
| 11- | 22 | 4-CF3 | O-C2H4-O | | 1H: 1,74, 2,51, 3,56, 3,94, 7,44, 7,55 |
| 11- | 23 | 4-MeO | O-C2H4-O | | |
| 11- | 24 | 4-EtO | O-C2H4-O | | |
| 11- | 25 | 4-nPrO | O-C2H4-O | | |
| 11- | 26 | 4-nBuO | O-C2H4-O | | |
| 11- | 27 | 4-OCHF2 | O-C2H4-O | | |
| 11- | 28 | 4-OCF3 | O-C2H4-O | | |
| 11- | 29 | 4-OCH2CF3 | O-C2H4-O | | |
| 11- | 30 | 4-OC2F4H | O-C2H4-O | | |
| 11- | 31 | 4-OC3F6H | O-C2H4-O | | |
| 11- | 32 | 4-SMe | O-C2H4-O | | |
| 11- | 33 | 4-SOMe | O-C2H4-O | | |
| 11- | 34 | 4-SO2Me | O-C2H4-O | | |
| 11- | 35 | 4-SEt | O-C2H4-O | | |
| 11- | 36 | 4-SOEt | O-C2H4-O | | |
| 11- | 37 | 4-SO2Et | O-C2H4-O | | |
| 11- | 38 | 4-SCF3 | O-C2H4-O | | |
| 11- | 39 | 4-SOCF3 | O-C2H4-O | | |
| 11- | 40 | 4-SO2CF3 | O-C2H4-O | | |
| 11- | 41 | 4-CN | O-C2H4-O | | |
| 11- | 42 | 4-NO2 | O-C2H4-O | | |
| 11- | 43 | 4-NMe2 | O-C2H4-O | | |
| 11- | 44 | 2,3-F2 | O-C2H4-O | | |
| 11- | 45 | 2,4-F2 | O-C2H4-O | | |
| 11- | 46 | 2,5-F2 | O-C2H4-O | | |
| 11- | 47 | 2,6-F2 | O-C2H4-O | | |
| 11- | 48 | 3,4-F2 | O-C2H4-O | | |
| 11- | 49 | 3,5-F2 | O-C2H4-O | | |
| 11- | 50 | 2,3-Cl2 | O-C2H4-O | | |
| 11- | 51 | 2,4-Cl2 | O-C2H4-O | | |
| 11- | 52 | 2,5-Cl2 | O-C2H4-O | | |
| 11- | 53 | 2,6-Cl2 | O-C2H4-O | | |
| 11- | 54 | 3,4-Cl2 | O-C2H4-O | | |
| 11- | 55 | 3,5-Cl2 | O-C2H4-O | | |
| 11- | 56 | 2-Cl-4-CF3 | O-C2H4-O | | |
| 11- | 57 | 2-Cl-4-F | O-C2H4-O | | |
| 11- | 58 | H | O-C3H6-O | | |
| 11- | 59 | 2-F | O-C3H6-O | | |
| 11- | 60 | 2-Cl | O-C3H6-O | | |
| 11- | 61 | 2-Br | O-C3H6-O | | |
| 11- | 62 | 2-I | O-C3H6-O | | |
| 11- | 63 | 3-F | O-C3H6-O | | |
| 11- | 64 | 3-Cl | O-C3H6-O | | |
| 11- | 65 | 3-Br | O-C3H6-O | | |
| 11- | 66 | 3-I | O-C3H6-O | | |
| 11- | 67 | 4-F | O-C3H6-O | | |
| 11- | 68 | 4-Cl | O-C3H6-O | | |
| 11- | 69 | 4-Br | O-C3H6-O | | |
| 11- | 70 | 4-I | O-C3H6-O | | |
| 11- | 71 | 4-Me | O-C3H6-O | | |
| 11- | 72 | 4-Et | O-C3H6-O | | |
| 11- | 73 | 4-nPr | O-C3H6-O | | |
| 11- | 74 | 4-iPr | O-C3H6-O | | |
| 11- | 75 | 4-nBu | O-C3H6-O | | |
| 11- | 76 | 4-sec-Bu | O-C3H6-O | | |
| 11- | 77 | 4-iBu | O-C3H6-O | | |
| 11- | 78 | 4-tBu | O-C3H6-O | | |
| 11- | 79 | 4-CF3 | O-C3H6-O | | |
| 11- | 80 | 4-MeO | O-C3H6-O | | |
| 11- | 81 | 4-EtO | O-C3H6-O | | |
| 11- | 82 | 4-nPrO | O-C3H6-O | | |
| 11- | 83 | 4-nBuO | O-C3H6-O | | |
| 11- | 84 | 4-OCHF2 | O-C3H6-O | | |
| 11- | 85 | 4-OCF3 | O-C3H6-O | | |
| 11- | 86 | 4-OCH2CF3 | O-C3H6-O | | |
| 11- | 87 | 4-OC2F4H | O-C3H6-O | | |
| 11- | 88 | 4-OC3F6H | O-C3H6-O | | |
| 11- | 89 | 4-SMe | O-C3H6-O | | |
| 11- | 90 | 4-SOMe | O-C3H6-O | | |
| 11- | 91 | 4-SO2Me | O-C3H6-O | | |
| 11- | 92 | 4-SEt | O-C3H6-O | | |
| 11- | 93 | 4-SOEt | O-C3H6-O | | |
| 11- | 94 | 4-SO2Et | O-C3H6-O | | |
| 11- | 95 | 4-SCF3 | O-C3H6-O | | |
| 11- | 96 | 4-SOCF3 | O-C3H6-O | | |
| 11- | 97 | 4-SO2CF3 | O-C3H6-O | | |
| 11- | 98 | 4-CN | O-C3H6-O | | |
| 11- | 99 | 4-NO2 | O-C3H6-O | | |
| 11- | 100 | 4-NMe2 | O-C3H6-O | | |
| 11- | 101 | 2,3-F2 | O-C3H6-O | | |
| 11- | 102 | 2,4-F2 | O-C3H6-O | | |
| 11- | 103 | 2,5-F2 | O-C3H6-O | | |
| 11- | 104 | 2,6-F2 | O-C3H6-O | | |
| 11- | 105 | 3,4-F2 | O-C3H6-O | | |
| 11- | 106 | 3,5-F2 | O-C3H6-O | | |
| 11- | 107 | 2,3-Cl2 | O-C3H6-O | | |
| 11- | 108 | 2,4-Cl2 | O-C3H6-O | | |
| 11- | 109 | 2,5-Cl2 | O-C3H6-O | | |
| 11- | 110 | 2,6-Cl2 | O-C3H6-O | | |
| 11- | 111 | 3,4-Cl2 | O-C3H6-O | | |
| 11- | 112 | 2,5-Cl2 | O-C3H6-O | | |
| 11- | 113 | 2-Cl-4-CF3 | O-C3H6-O | | |
| 11- | 114 | 2-Cl-4-F | O-C3H6-O | | |

**Table 12: Compounds of Formula (lb) in which the substituents have the following meanings: R¹ is Phenyl substituted by R; (A)n is CH2; R² and R³ are each H; R⁴ is (C₁-C₃)-alkoxy and R⁵ is H;**

| Compound number | | R | R4 | CA REG No | NMR |
|---|---|---|---|---|---|
| 12- | 01 | H | OMe | | |
| 12- | 02 | 2-F | OMe | | |
| 12- | 03 | 2-Cl | OMe | | |
| 12- | 04 | 2-Br | OMe | | |
| 12- | 05 | 2-I | OMe | | |
| 12- | 06 | 3-F | OMe | | |
| 12- | 07 | 3-Cl | OMe | | |
| 12- | 08 | 3-Br | OMe | | |
| 12- | 09 | 3-I | OMe | | |
| 12- | 10 | 4-F | OMe | | |
| 12- | 11 | 4-Cl | OMe | | 1 H: 1,57; 1,87; 2,12; 2,68; 3,20; 3,32; 3,43; 7,24; |
| 12- | 12 | 4-Br | OMe | | |
| 12- | 13 | 4-I | OMe | | |
| 12- | 14 | 4-Me | OMe | | |
| 12- | 15 | 4-Et | OMe | | |
| 12- | 16 | 4-nPr | OMe | | |
| 12- | 17 | 4-iPr | OMe | | |
| 12- | 18 | 4-nBu | OMe | | |
| 12- | 19 | 4-sec-Bu | OMe | | |
| 12- | 20 | 4-iBu | OMe | | |
| 12- | 21 | 4-tBu | OMe | | |
| 12- | 22 | 4-CF3 | OMe | | |
| 12- | 23 | 4-MeO | OMe | | |
| 12- | 24 | 4-EtO | OMe | | |
| 12- | 25 | 4-nPrO | OMe | | |
| 12- | 26 | 4-nBuO | OMe | | |
| 12- | 27 | 4-OCHF2 | OMe | | |
| 12- | 28 | 4-OCF3 | OMe | | |
| 12- | 29 | 4-OCH2CF3 | OMe | | |
| 12- | 30 | 4-OC2F4H | OMe | | |
| 12- | 31 | 4-OC3F6H | OMe | | |
| 12- | 32 | 4-SMe | OMe | | |
| 12- | 33 | 4-SOMe | OMe | | |
| 12- | 34 | 4-SO2Me | OMe | | |
| 12- | 35 | 4-SEt | OMe | | |
| 12- | 36 | 4-SOEt | OMe | | |
| 12- | 37 | 4-SO2Et | OMe | | |
| 12- | 38 | 4-SCF3 | OMe | | |
| 12- | 39 | 4-SOCF3 | OMe | | |
| 12- | 40 | 4-SO2CF3 | OMe | | |
| 12- | 41 | 4-CN | OMe | | |
| 12- | 42 | 4-NO2 | OMe | | |
| 12- | 43 | 4-NMe2 | OMe | | |
| 12- | 44 | 2,3-F2 | OMe | | |
| 12- | 45 | 2,4-F2 | OMe | | |
| 12- | 46 | 2,5-F2 | OMe | | |
| 12- | 47 | 2,6-F2 | OMe | | |
| 12- | 48 | 3,4-F2 | OMe | | |
| 12- | 49 | 3,5-F2 | OMe | | |
| 12- | 50 | 2,3-Cl2 | OMe | | |
| 12- | 51 | 2,4-Cl2 | OMe | | |
| 12- | 52 | 2,5-Cl2 | OMe | | |
| 12- | 53 | 2,6-Cl2 | OMe | | |
| 12- | 54 | 3,4-Cl2 | OMe | | |
| 12- | 55 | 3,5-Cl2 | OMe | | |
| 12- | 56 | 2-Cl-4-CF3 | OMe | | |
| 12- | 57 | 2-Cl-4-F | OMe | | |
| 12- | 58 | H | OEt | 1142-04-7 | |
| 12- | 59 | 2-F | OEt | | |
| 12- | 60 | 2-Cl | OEt | | |
| 12- | 61 | 2-Br | OEt | | |
| 12- | 62 | 2-I | OEt | | |
| 12- | 63 | 3-F | OEt | | |
| 12- | 64 | 3-Cl | OEt | | |
| 12- | 65 | 3-Br | OEt | | |
| 12- | 66 | 3-I | OEt | | |
| 12- | 67 | 4-F | OEt | | |
| 12- | 68 | 4-Cl | OEt | | |
| 12- | 69 | 4-Br | OEt | | |
| 12- | 70 | 4-I | OEt | | |
| 12- | 71 | 4-Me | OEt | | |
| 12- | 72 | 4-Et | OEt | | |
| 12- | 73 | 4-nPr | OEt | | |
| 12- | 74 | 4-iPr | OEt | | |
| 12- | 75 | 4-nBu | OEt | | |
| 12- | 76 | 4-sec-Bu | OEt | | |
| 12- | 77 | 4-iBu | OEt | | |
| 12- | 78 | 4-tBu | OEt | | |
| 12- | 79 | 4-CF3 | OEt | | |
| 12- | 80 | 4-MeO | OEt | | |
| 12- | 81 | 4-EtO | OEt | | |
| 12- | 82 | 4-nPrO | OEt | | |
| 12- | 83 | 4-nBuO | OEt | | |
| 12- | 84 | 4-OCHF2 | OEt | | |
| 12- | 85 | 4-OCF3 | OEt | | |
| 12- | 86 | 4-OCH2CF3 | OEt | | |
| 12- | 87 | 4-OC2F4H | OEt | | |
| 12- | 88 | 4-OC3F6H | OEt | | |
| 12- | 89 | 4-SMe | OEt | | |
| 12- | 90 | 4-SOMe | OEt | | |
| 12- | 91 | 4-SO2Me | OEt | | |
| 12- | 92 | 4-SEt | OEt | | |
| 12- | 93 | 4-SOEt | OEt | | |
| 12- | 94 | 4-SO2Et | OEt | | |
| 12- | 95 | 4-SCF3 | OEt | | |
| 12- | 96 | 4-SOCF3 | OEt | | |
| 12- | 97 | 4-SO2CF3 | OEt | | |
| 12- | 98 | 4-CN | OEt | | |
| 12- | 99 | 4-NO2 | OEt | | |
| 12- | 100 | 4-NMe2 | OEt | | |
| 12- | 101 | 2,3-F2 | OEt | | |
| 12- | 102 | 2,4-F2 | OEt | | |
| 12- | 103 | 2,5-F2 | OEt | | |
| 12- | 104 | 2,6-F2 | OEt | | |
| 12- | 105 | 3,4-F2 | OEt | | |
| 12- | 106 | 3,5-F2 | OEt | | |
| 12- | 107 | 2,3-Cl2 | OEt | | |
| 12- | 108 | 2,4-Cl2 | OEt | | |
| 12- | 109 | 2,5-Cl2 | OEt | | |
| 12- | 110 | 2,6-Cl2 | OEt | | |
| 12- | 111 | 3,4-Cl2 | OEt | | |
| 12- | 112 | 2,5-Cl2 | OEt | | |
| 12- | 113 | 2-Cl-4-CF3 | OEt | | |
| 12- | 114 | 2-Cl-4-F | OEt | | |
| 12- | 115 | H | OnPr | | |
| 12- | 116 | 2-F | OnPr | | |
| 12- | 117 | 2-Cl | OnPr | | |
| 12- | 118 | 2-Br | OnPr | | |
| 12- | 119 | 2-I | OnPr | | |
| 12- | 120 | 3-F | OnPr | | |
| 12- | 121 | 3-Cl | OnPr | | |
| 12- | 122 | 3-Br | OnPr | | |
| 12- | 123 | 3-I | OnPr | | |
| 12- | 124 | 4-F | OnPr | | |
| 12- | 125 | 4-Cl | OnPr | | |
| 12- | 126 | 4-Br | OnPr | | |
| 12- | 127 | 4-I | OnPr | | |

**Table 13: Compounds of Formula (Ib) in which the substituents have the following meanings: R¹ is Phenyl substituted by R; (A)n is CH2; R² and R³ are each H; R⁴ is H or (C₁-C₂)-alkyl and R⁵ is H;**

| Compound number | | R | R4 | CA REG No | NMR |
|---|---|---|---|---|---|
| 13- | 01 | H | H | | |
| 13- | 02 | 2-F | H | | |
| 13- | 03 | 2-Cl | H | | |
| 13- | 04 | 2-Br | H | | |
| 13- | 05 | 2-I | H | | |
| 13- | 06 | 3-F | H | | |
| 13- | 07 | 3-Cl | H | | |
| 13- | 08 | 3-Br | H | | |
| 13- | 09 | 3-I | H | | |
| 13- | 10 | 4-F | H | | |
| 13- | 11 | 4-Cl | H | | |
| 13- | 12 | 4-Br | H | | |
| 13- | 13 | 4-I | H | | |
| 13- | 14 | H | Me | | |
| 13- | 15 | 2-F | Me | | |
| 13- | 16 | 2-Cl | Me | | |
| 13- | 17 | 2-Br | Me | | |
| 13- | 18 | 2-I | Me | | |
| 13- | 19 | 3-F | Me | | |
| 13- | 20 | 3-Cl | Me | | |
| 13- | 21 | 3-Br | Me | | |
| 13- | 22 | 3-I | Me | | |
| 13- | 23 | 4-F | Me | | |
| 13- | 24 | 4-Cl | Me | | |
| 13- | 25 | 4-Br | Me | | |
| 13- | 26 | 4-I | Me | | |
| 13- | 27 | 4-Me | Me | | |
| 13- | 28 | 4-Et | Me | | |
| 13- | 29 | 4-nPr | Me | | |
| 13- | 30 | 4-iPr | Me | | |
| 13- | 31 | 4-nBu | Me | | |
| 13- | 32 | 4-sec-Bu | Me | | |
| 13- | 33 | 4-iBu | Me | | |
| 13- | 34 | 4-tBu | Me | | |
| 13- | 35 | 4-CF3 | Me | | |
| 13- | 36 | 4-MeO | Me | | |
| 13- | 37 | 4-EtO | Me | | |
| 13- | 38 | 4-nPrO | Me | | |
| 13- | 39 | 4-nBuO | Me | | |
| 13- | 40 | 4-OCHF2 | Me | | |
| 13- | 41 | 4-OCF3 | Me | | |
| 13- | 42 | 4-OCH2CF3 | Me | | |
| 13- | 43 | 4-OC2F4H | Me | | |
| 13- | 44 | 4-OC3F6H | Me | | |
| 13- | 45 | 4-SMe | Me | | |
| 13- | 46 | 4-SOMe | Me | | |
| 13- | 47 | 4-SO2Me | Me | | |
| 13- | 48 | 4-SEt | Me | | |
| 13- | 49 | 4-SOEt | Me | | |
| 13- | 50 | 4-SO2Et | Me | | |
| 13- | 51 | 4-SCF3 | Me | | |
| 13- | 52 | 4-SOCF3 | Me | | |
| 13- | 53 | 4-SO2CF3 | Me | | |
| 13- | 54 | 4-CN | Me | | |
| 13- | 55 | 4-NO2 | Me | | |
| 13- | 56 | 4-NMe2 | Me | | |
| 13- | 57 | 2,3-F2 | Me | | |
| 13- | 58 | 2,4-F2 | Me | | |
| 13- | 59 | 2,5-F2 | Me | | |
| 13- | 60 | 2,6-F2 | Me | | |
| 13- | 61 | 3,4-F2 | Me | | |
| 13- | 62 | 3,5-F2 | Me | | |
| 13- | 63 | 2,3-Cl2 | Me | | |
| 13- | 64 | 2,4-Cl2 | Me | | |
| 13- | 65 | 2,5-Cl2 | Me | | |
| 13- | 66 | 2,6-Cl2 | Me | | |
| 13- | 67 | 3,4-Cl2 | Me | | |
| 13- | 68 | 3,5-Cl2 | Me | | |
| 13- | 69 | 2-Cl-4-CF3 | Me | | |
| 13- | 70 | 2-Cl-4-F | Me | | |
| 13- | 71 | H | Et | | |
| 13- | 72 | 2-F | Et | | |
| 13- | 73 | 2-Cl | Et | | |
| 13- | 74 | 2-Br | Et | | |
| 13- | 75 | 2-I | Et | | |
| 13- | 76 | 3-F | Et | | |
| 13- | 77 | 3-Cl | Et | | |
| 13- | 78 | 3-Br | Et | | |
| 13- | 79 | 3-I | Et | | |
| 13- | 80 | 4-F | Et | | |
| 13- | 81 | 4-Cl | Et | | |
| 13- | 82 | 4-Br | Et | | |
| 13- | 83 | 4-I | Et | | |
| 13- | 84 | 4-Me | Et | | |
| 13- | 85 | 4-Et | Et | | |
| 13- | 86 | 4-nPr | Et | | |
| 13- | 87 | 4-iPr | Et | | |
| 13- | 88 | 4-nBu | Et | | |
| 13- | 89 | 4-sec-Bu | Et | | |
| 13- | 90 | 4-iBu | Et | | |
| 13- | 91 | 4-tBu | Et | | |
| 13- | 92 | 4-CF3 | Et | | |
| 13- | 93 | 4-MeO | Et | | |
| 13- | 94 | 4-EtO | Et | | |
| 13- | 95 | 4-nPrO | Et | | |
| 13- | 96 | 4-nBuO | Et | | |
| 13- | 97 | 4-OCHF2 | Et | | |
| 13- | 98 | 4-OCF3 | Et | | |
| 13- | 99 | 4-OCH2CF3 | Et | | |
| 13- | 100 | 4-OC2F4H | Et | | |
| 13- | 101 | 4-OC3F6H | Et | | |
| 13- | 102 | 4-SMe | Et | | |
| 13- | 103 | 4-SOMe | Et | | |
| 13- | 104 | 4-SO2Me | Et | | |
| 13- | 105 | 4-SEt | Et | | |
| 13- | 106 | 4-SOEt | Et | | |
| 13- | 107 | 4-SO2Et | Et | | |
| 13- | 108 | 4-SCF3 | Et | | |
| 13- | 109 | 4-SOCF3 | Et | | |
| 13- | 110 | 4-SO2CF3 | Et | | |
| 13- | 111 | 4-CN | Et | | |
| 13- | 112 | 4-NO2 | Et | | |
| 13- | 113 | 4-NMe2 | Et | | |
| 13- | 114 | 2,3-F2 | Et | | |
| 13- | 115 | 2,4-F2 | Et | | |
| 13- | 116 | 2,5-F2 | Et | | |
| 13- | 117 | 2,6-F2 | Et | | |
| 13- | 118 | 3,4-F2 | Et | | |
| 13- | 119 | 3,5-F2 | Et | | |
| 13- | 120 | 2,3-Cl2 | Et | | |
| 13- | 121 | 2,4-Cl2 | Et | | |
| 13- | 122 | 2,5-Cl2 | Et | | |
| 13- | 123 | 2,6-Cl2 | Et | | |
| 13- | 124 | 3,4-Cl2 | Et | | |
| 13- | 125 | 2,5-Cl2 | Et | | |
| 13- | 126 | 2-Cl-4-CF3 | Et | | |
| 13- | 127 | 2-Cl-4-F | Et | | |

**Table 14: Compounds of Formula (Ic) in which the substituents have the following meanings: R¹ is Phenyl substituted by R; (A)n is CH2; R² and R³ are each H; R⁶ is OH and R⁷ is H;**

| Compound number | | R | CA REG No | NMR |
|---|---|---|---|---|
| 14- | 01 | H | 4727-72-4 | |
| 14- | 02 | 2-F | | |
| 14- | 03 | 2-Cl | | |
| 14- | 04 | 2-Br | | |
| 14- | 05 | 2-I | | |
| 14- | 06 | 3-F | | |
| 14- | 07 | 3-Cl | | |
| 14- | 08 | 3-Br | | |
| 14- | 09 | 3-I | | |
| 14- | 10 | 4-F | | |
| 14- | 11 | 4-Cl | | |
| 14- | 12 | 4-Br | | |
| 14- | 13 | 4-I | | |
| 14- | 14 | 2-Me | | |
| 14- | 15 | 2-Et | | |
| 14- | 16 | 2-nPr | | |
| 14- | 17 | 2-iPr | | |
| 14- | 18 | 2-nBu | | |
| 14- | 19 | 2-sec-Bu | | |
| 14- | 20 | 2-iBu | | |
| 14- | 21 | 2-tBu | | |
| 14- | 22 | 2-CF3 | | |
| 14- | 23 | 2-MeO | | |
| 14- | 24 | 2-EtO | | |
| 14- | 25 | 2-nPrO | | |
| 14- | 26 | 2-nBuO | | |
| 14- | 27 | 2-OCHF2 | | |
| 14- | 28 | 2-OCF3 | | |
| 14- | 29 | 2-OCH2CF3 | | |
| 14- | 30 | 2-OC2F4H | | |
| 14- | 31 | 2-OC3F6H | | |
| 14- | 32 | 2-SMe | | |
| 14- | 33 | 2-SOMe | | |
| 14- | 34 | 2-SO2Me | | |
| 14- | 35 | 2-SEt | | |
| 14- | 36 | 2-SOEt | | |
| 14- | 37 | 2-SO2Et | | |
| 14- | 38 | 2-SCF3 | | |
| 14- | 39 | 2-SOCF3 | | |
| 14- | 40 | 2-SO2CF3 | | |
| 14- | 41 | 2-CN | | |
| 14- | 42 | 2-NO2 | | |
| 14- | 43 | 2-NMe2 | | |
| 14- | 44 | 3-Me | | |
| 14- | 45 | 3-Et | | |
| 14- | 46 | 3-nPr | | |
| 14- | 47 | 3-iPr | | |
| 14- | 48 | 3-nBu | | |
| 14- | 49 | 3-sec-Bu | | |
| 14- | 50 | 3-iBu | | |
| 14- | 51 | 3-tBu | | |
| 14- | 52 | 3-CF3 | | |
| 14- | 53 | 3-MeO | | |
| 14- | 54 | 3-EtO | | |
| 14- | 55 | 3-nPrO | | |
| 14- | 56 | 3-nBuO | | |
| 14- | 57 | 3-OCHF2 | | |
| 14- | 58 | 3-OCF3 | | |
| 14- | 59 | 3-OCH2CF3 | | |
| 14- | 60 | 3-OC2F4H | | |
| 14- | 61 | 3-OC3F6H | | |
| 14- | 62 | 3-SMe | | |
| 14- | 63 | 3-SOMe | | |
| 14- | 64 | 3-SO2Me | | |
| 14- | 65 | 3-SEt | | |
| 14- | 66 | 3-SOEt | | |
| 14- | 67 | 3-SO2Et | | |
| 14- | 68 | 3-SCF3 | | |
| 14- | 69 | 3-SOCF3 | | |
| 14- | 70 | 3-SO2CF3 | | |
| 14- | 71 | 3-CN | | |
| 14- | 72 | 3-NO2 | | |
| 14- | 73 | 3-NMe2 | | |
| 14- | 74 | 4-Me | | |
| 14- | 75 | 4-Et | | |
| 14- | 76 | 4-nPr | | |
| 14- | 77 | 4-iPr | | |
| 14- | 78 | 4-nBu | | |
| 14- | 79 | 4-sec-Bu | | |
| 14- | 80 | 4-iBu | | |
| 14- | 81 | 4-tBu | | |
| 14- | 82 | 4-CF3 | | 1H: 1,60; 1,76; 1,89; 2,16; 2,74; 3,53; 3,71; 7,44; 7,56; |
| 14- | 83 | 4-MeO | | |
| 14- | 84 | 4-EtO | | |
| 14- | 85 | 4-nPrO | | |
| 14- | 86 | 4-nBuO | | |
| 14- | 87 | 4-OCHF2 | | |
| 14- | 88 | 4-OCF3 | | |
| 14- | 89 | 4-OCH2CF3 | | |
| 14- | 90 | 4-OC2F4H | | |
| 14- | 91 | 4-OC3F6H | | |
| 14- | 92 | 4-SMe | | |
| 14- | 93 | 4-SOMe | | |
| 14- | 94 | 4-SO2Me | | |
| 14- | 95 | 4-SEt | | |
| 14- | 96 | 4-SOEt | | |
| 14- | 97 | 4-SO2Et | | |
| 14- | 98 | 4-SCF3 | | |
| 14- | 99 | 4-SOCF3 | | |
| 14- | 100 | 4-SO2CF3 | | |
| 14- | 101 | 4-CN | | |
| 14- | 102 | 4-NO2 | | |
| 14- | 103 | 4-NMe2 | | |
| 14- | 104 | 2,3-F2 | | |
| 14- | 105 | 2,4-F2 | | |
| 14- | 106 | 2,5-F2 | | |
| 14- | 107 | 2,6-F2 | | |
| 14- | 108 | 3,4-F2 | | |
| 14- | 109 | 3,5-F2 | | |
| 14- | 110 | 2,3-Cl2 | | |
| 14- | 111 | 2,4-Cl2 | | |
| 14- | 112 | 2,5-Cl2 | | |
| 14- | 113 | 2,6-Cl2 | | |
| 14- | 114 | 3,4-Cl2 | | |
| 14- | 115 | 3,5-Cl2 | | |
| 14- | 116 | 2-Cl-4-CF3 | | |
| 14- | 117 | 2-Cl-4-F | | |
| 14- | 118 | 2,3-Me2 | | |
| 14- | 119 | 2,4-Me2 | | |
| 14- | 120 | 2,5-Me2 | | |
| 14- | 121 | 2,6-Me2 | | |
| 14- | 122 | 3,4-Me2 | | |
| 14- | 123 | 3,5-Me2 | | |
| 14- | 124 | 2,4,6-F3 | | |
| 14- | 125 | 3,4,5-F3 | | |
| 14- | 126 | 2,4,6-Cl3 | | |
| 14- | 127 | 2,3,4-Cl3 | | |
| 14- | 128 | 2,4,5-Cl3 | | |
| 14- | 129 | 3,4,5-Cl3 | | |
| 14- | 130 | 2,6-Cl2-4-CF3 | | |
| 14- | 131 | 2,4,6-Me3 | | |
| 14- | 132 | 2,4-Br2 | | |
| 14- | 133 | 3,4-Br2 | | |
| 14- | 134 | 2-Cl-4-Br | | |
| 14- | 135 | 2-Br-4-F | | |

**Table 15: Compounds of Formula (Ic) in which the substituents have the following meanings: R¹ is Phenyl substituted by R; (A)n is CH2; R² and R³ are each H; R⁶ is O-COCH3 and R⁷ is H;**

| Compound number | | R | CA REG No | NMR |
|---|---|---|---|---|
| 15- | 01 | H | | |
| 15- | 02 | 2-F | | |
| 15- | 03 | 2-Cl | | |
| 15- | 04 | 2-Br | | |
| 15- | 05 | 2-I | | |
| 15- | 06 | 3-F | | |
| 15- | 07 | 3-Cl | | |
| 15- | 08 | 3-Br | | |
| 15- | 09 | 3-I | | |
| 15- | 10 | 4-F | | |
| 15- | 11 | 4-Cl | | |
| 15- | 12 | 4-Br | | |
| 15- | 13 | 4-I | | |
| 15- | 14 | 2-Me | | |
| 15- | 15 | 2-Et | | |
| 15- | 16 | 2-nPr | | |
| 15- | 17 | 2-iPr | | |
| 15- | 18 | 2-nBu | | |
| 15- | 19 | 2-sec-Bu | | |
| 15- | 20 | 2-iBu | | |
| 15- | 21 | 2-tBu | | |
| 15- | 22 | 2-CF3 | | |
| 15- | 23 | 2-MeO | | |
| 15- | 24 | 2-EtO | | |
| 15- | 25 | 2-nPrO | | |
| 15- | 26 | 2-nBuO | | |
| 15- | 27 | 2-OCHF2 | | |
| 15- | 28 | 2-OCF3 | | |
| 15- | 29 | 2-OCH2CF3 | | |
| 15- | 30 | 2-OC2F4H | | |
| 15- | 31 | 2-OC3F6H | | |
| 15- | 32 | 2-SMe | | |
| 15- | 33 | 2-SOMe | | |
| 15- | 34 | 2-SO2Me | | |
| 15- | 35 | 2-SEt | | |
| 15- | 36 | 2-SOEt | | |
| 15- | 37 | 2-SO2Et | | |
| 15- | 38 | 2-SCF3 | | |
| 15- | 39 | 2-SOCF3 | | |
| 15- | 40 | 2-SO2CF3 | | |
| 15- | 41 | 2-CN | | |
| 15- | 42 | 2-NO2 | | |
| 15- | 43 | 2-NMe2 | | |
| 15- | 44 | 3-Me | | |
| 15- | 45 | 3-Et | | |
| 15- | 46 | 3-nPr | | |
| 15- | 47 | 3-iPr | | |
| 15- | 48 | 3-nBu | | |
| 15- | 49 | 3-sec-Bu | | |
| 15- | 50 | 3-iBu | | |
| 15- | 51 | 3-tBu | | |
| 15- | 52 | 3-CF3 | | |
| 15- | 53 | 3-MeO | | |
| 15- | 54 | 3-EtO | | |
| 15- | 55 | 3-nPrO | | |
| 15- | 56 | 3-nBuO | | |
| 15- | 57 | 3-OCHF2 | | |
| 15- | 58 | 3-OCF3 | | |
| 15- | 59 | 3-OCH2CF3 | | |
| 15- | 60 | 3-OC2F4H | | |
| 15- | 61 | 3-OC3F6H | | |
| 15- | 62 | 3-SMe | | |
| 15- | 63 | 3-SOMe | | |
| 15- | 64 | 3-SO2Me | | |
| 15- | 65 | 3-SEt | | |
| 15- | 66 | 3-SOEt | | |
| 15- | 67 | 3-SO2Et | | |
| 15- | 68 | 3-SCF3 | | |
| 15- | 69 | 3-SOCF3 | | |
| 15- | 70 | 3-SO2CF3 | | |
| 15- | 71 | 3-CN | | |
| 15- | 72 | 3-NO2 | | |
| 15- | 73 | 3-NMe2 | | |
| 15- | 74 | 4-Me | | |
| 15- | 75 | 4-Et | | |
| 15- | 76 | 4-nPr | | |
| 15- | 77 | 4-iPr | | |
| 15- | 78 | 4-nBu | | |
| 15- | 79 | 4-sec-Bu | | |
| 15- | 80 | 4-iBu | | |
| 15- | 81 | 4-tBu | | |
| 15- | 82 | 4-CF3 | | |
| 15- | 83 | 4-MeO | | |
| 15- | 84 | 4-EtO | | |
| 15- | 85 | 4-nPrO | | |
| 15- | 86 | 4-nBuO | | |
| 15- | 87 | 4-OCHF2 | | |
| 15- | 88 | 4-OCF3 | | |
| 15- | 89 | 4-OCH2CF3 | | |
| 15- | 90 | 4-OC2F4H | | |
| 15- | 91 | 4-OC3F6H | | |
| 15- | 92 | 4-SMe | | |
| 15- | 93 | 4-SOMe | | |
| 15- | 94 | 4-SO2Me | | |
| 15- | 95 | 4-SEt | | |
| 15- | 96 | 4-SOEt | | |
| 15- | 97 | 4-SO2Et | | |
| 15- | 98 | 4-SCF3 | | |
| 15- | 99 | 4-SOCF3 | | |
| 15- | 100 | 4-SO2CF3 | | |
| 15- | 101 | 4-CN | | |
| 15- | 102 | 4-NO2 | | |
| 15- | 103 | 4-NMe2 | | |
| 15- | 104 | 2,3-F2 | | |
| 15- | 105 | 2,4-F2 | | |
| 15- | 106 | 2,5-F2 | | |
| 15- | 107 | 2,6-F2 | | |
| 15- | 108 | 3,4-F2 | | |
| 15- | 109 | 3,5-F2 | | |
| 15- | 110 | 2,3-Cl2 | | |
| 15- | 111 | 2,4-Cl2 | | |
| 15- | 112 | 2,5-Cl2 | | |
| 15- | 113 | 2,6-Cl2 | | |
| 15- | 114 | 3,4-Cl2 | | |
| 15- | 115 | 3,5-Cl2 | | |
| 15- | 116 | 2-Cl-4-CF3 | | |
| 15- | 117 | 2-Cl-4-F | | |
| 15- | 118 | 2,3-Me2 | | |
| 15- | 119 | 2,4-Me2 | | |
| 15- | 120 | 2,5-Me2 | | |
| 15- | 121 | 2,6-Me2 | | |
| 15- | 122 | 3,4-Me2 | | |
| 15- | 123 | 3,5-Me2 | | |
| 15- | 124 | 2,4,6-F3 | | |
| 15- | 125 | 3,4,5-F3 | | |
| 15- | 126 | 2,4,6-Cl3 | | |
| 15- | 127 | 2,3,4-Cl3 | | |
| 15- | 128 | 2,4,5-Cl3 | | |
| 15- | 129 | 3,4,5-Cl3 | | |
| 15- | 130 | 2,6-Cl2-4-CF3 | | |
| 15- | 131 | 2,4,6-Me3 | | |
| 15- | 132 | 2,4-Br2 | | |
| 15- | 133 | 3,4-Br2 | | |
| 15- | 134 | 2-Cl-4-Br | | |
| 15- | 135 | 2-Br-4-F | | |

**Table 16: Compounds of Formula (Ic) in which the substituents have the following meanings: R¹ is Phenyl substituted by R; (A)n is CH2; R² and R³ are each H; R⁶ is O-COC2H5 and R⁷ is H;**

| Compound number | | R | CA REG No | NMR |
|---|---|---|---|---|
| 16- | 01 | H | | |
| 16- | 02 | 2-F | | |
| 16- | 03 | 2-Cl | | |
| 16- | 04 | 2-Br | | |
| 16- | 05 | 2-I | | |
| 16- | 06 | 3-F | | |
| 16- | 07 | 3-Cl | | |
| 16- | 08 | 3-Br | | |
| 16- | 09 | 3-I | | |
| 16- | 10 | 4-F | | |
| 16- | 11 | 4-Cl | | |
| 16- | 12 | 4-Br | | |
| 16- | 13 | 4-I | | |
| 16- | 14 | 2-Me | | |
| 16- | 15 | 2-Et | | |
| 16- | 16 | 2-nPr | | |
| 16- | 17 | 2-iPr | | |
| 16- | 18 | 2-nBu | | |
| 16- | 19 | 2-sec-Bu | | |
| 16- | 20 | 2-iBu | | |
| 16- | 21 | 2-tBu | | |
| 16- | 22 | 2-CF3 | | |
| 16- | 23 | 2-MeO | | |
| 16- | 24 | 2-EtO | | |
| 16- | 25 | 2-nPrO | | |
| 16- | 26 | 2-nBuO | | |
| 16- | 27 | 2-OCHF2 | | |
| 16- | 28 | 2-OCF3 | | |
| 16- | 29 | 2-OCH2CF3 | | |
| 16- | 30 | 2-OC2F4H | | |
| 16- | 31 | 2-OC3F6H | | |
| 16- | 32 | 2-SMe | | |
| 16- | 33 | 2-SOMe | | |
| 16- | 34 | 2-SO2Me | | |
| 16- | 35 | 2-SEt | | |
| 16- | 36 | 2-SOEt | | |
| 16- | 37 | 2-SO2Et | | |
| 16- | 38 | 2-SCF3 | | |
| 16- | 39 | 2-SOCF3 | | |
| 16- | 40 | 2-SO2CF3 | | |
| 16- | 41 | 2-CN | | |
| 16- | 42 | 2-NO2 | | |
| 16- | 43 | 2-NMe2 | | |
| 16- | 44 | 3-Me | | |
| 16- | 45 | 3-Et | | |
| 16- | 46 | 3-nPr | | |
| 16- | 47 | 3-iPr | | |
| 16- | 48 | 3-nBu | | |
| 16- | 49 | 3-sec-Bu | | |
| 16- | 50 | 3-iBu | | |
| 16- | 51 | 3-tBu | | |
| 16- | 52 | 3-CF3 | | |
| 16- | 53 | 3-MeO | | |
| 16- | 54 | 3-EtO | | |
| 16- | 55 | 3-nPrO | | |
| 16- | 56 | 3-nBuO | | |
| 16- | 57 | 3-OCHF2 | | |
| 16- | 58 | 3-OCF3 | | |
| 16- | 59 | 3-OCH2CF3 | | |
| 16- | 60 | 3-OC2F4H | | |
| 16- | 61 | 3-OC3F6H | | |
| 16- | 62 | 3-SMe | | |
| 16- | 63 | 3-SOMe | | |
| 16- | 64 | 3-SO2Me | | |
| 16- | 65 | 3-SEt | | |
| 16- | 66 | 3-SOEt | | |
| 16- | 67 | 3-SO2Et | | |
| 16- | 68 | 3-SCF3 | | |
| 16- | 69 | 3-SOCF3 | | |
| 16- | 70 | 3-SO2CF3 | | |
| 16- | 71 | 3-CN | | |
| 16- | 72 | 3-NO2 | | |
| 16- | 73 | 3-NMe2 | | |
| 16- | 74 | 4-Me | | |
| 16- | 75 | 4-Et | | |
| 16- | 76 | 4-nPr | | |
| 16- | 77 | 4-iPr | | |
| 16- | 78 | 4-nBu | | |
| 16- | 79 | 4-sec-Bu | | |
| 16- | 80 | 4-iBu | | |
| 16- | 81 | 4-tBu | | |
| 16- | 82 | 4-CF3 | | 1 H: 4,81, CHO-CO; |
| 16- | 83 | 4-MeO | | |
| 16- | 84 | 4-EtO | | |
| 16- | 85 | 4-nPrO | | |
| 16- | 86 | 4-nBuO | | |
| 16- | 87 | 4-OCHF2 | | |
| 16- | 88 | 4-OCF3 | | |
| 16- | 89 | 4-OCH2CF3 | | |
| 16- | 90 | 4-OC2F4H | | |
| 16- | 91 | 4-OC3F6H | | |
| 16- | 92 | 4-SMe | | |
| 16- | 93 | 4-SOMe | | |
| 16- | 94 | 4-SO2Me | | |
| 16- | 95 | 4-SEt | | |
| 16- | 96 | 4-SOEt | | |
| 16- | 97 | 4-SO2Et | | |
| 16- | 98 | 4-SCF3 | | |
| 16- | 99 | 4-SOCF3 | | |
| 16- | 100 | 4-SO2CF3 | | |
| 16- | 101 | 4-CN | | |
| 16- | 102 | 4-NO2 | | |
| 16- | 103 | 4-NMe2 | | |
| 16- | 104 | 2,3-F2 | | |
| 16- | 105 | 2,4-F2 | | |
| 16- | 106 | 2,5-F2 | | |
| 16- | 107 | 2,6-F2 | | |
| 16- | 108 | 3,4-F2 | | |
| 16- | 109 | 3,5-F2 | | |
| 16- | 110 | 2,3-Cl2 | | |
| 16- | 111 | 2,4-Cl2 | | |
| 16- | 112 | 2,5-Cl2 | | |
| 16- | 113 | 2,6-Cl2 | | |
| 16- | 114 | 3,4-Cl2 | | |
| 16- | 115 | 3,5-Cl2 | | |
| 16- | 116 | 2-Cl-4-CF3 | | |
| 16- | 117 | 2-Cl-4-F | | |
| 16- | 118 | 2,3-Me2 | | |
| 16- | 119 | 2,4-Me2 | | |
| 16- | 120 | 2,5-Me2 | | |
| 16- | 121 | 2,6-Me2 | | |
| 16- | 122 | 3,4-Me2 | | |
| 16- | 123 | 3,5-Me2 | | |
| 16- | 124 | 2,4,6-F3 | | |
| 16- | 125 | 3,4,5-F3 | | |
| 16- | 126 | 2,4,6-Cl3 | | |
| 16- | 127 | 2,3,4-Cl3 | | |
| 16- | 128 | 2,4,5-Cl3 | | |
| 16- | 129 | 3,4,5-Cl3 | | |
| 16- | 130 | 2,6-Cl2-4-CF3 | | |
| 16- | 131 | 2,4,6-Me3 | | |
| 16- | 132 | 2,4-Br2 | | |
| 16- | 133 | 3,4-Br2 | | |
| 16- | 134 | 2-Cl-4-Br | | |
| 16- | 135 | 2-Br-4-F | | |

**Table 17: Compounds of Formula (Ic) in which the substituents have the following meanings: R¹ is Phenyl substituted by R; (A)n is CH2; R² and R³ are each H; R⁶ is COO(C₁-C₄)-alkyl and R⁷ is H;**

| Compound number | | R | R6 | CA REG No | NMR |
|---|---|---|---|---|---|
| 17- | 01 | H | COOMe | 10315-06-7 | |
| 17- | 02 | 2-F | COOMe | | |
| 17- | 03 | 2-Cl | COOMe | | |
| 17- | 04 | 2-Br | COOMe | | |
| 17- | 05 | 2-I | COOMe | | |
| 17- | 06 | 3-F | COOMe | | |
| 17- | 07 | 3-Cl | COOMe | | |
| 17- | 08 | 3-Br | COOMe | | |
| 17- | 09 | 3-I | COOMe | | |
| 17- | 10 | 4-F | COOMe | | |
| 17- | 11 | 4-Cl | COOMe | | |
| 17- | 12 | 4-Br | COOMe | | |
| 17- | 13 | 4-I | COOMe | | |
| 17- | 14 | 4-Me | COOMe | | |
| 17- | 15 | 4-Et | COOMe | | |
| 17- | 16 | 4-nPr | COOMe | | |
| 17- | 17 | 4-iPr | COOMe | | |
| 17- | 18 | 4-nBu | COOMe | | |
| 17- | 19 | 4-sec-Bu | COOMe | | |
| 17- | 20 | 4-iBu | COOMe | | |
| 17- | 21 | 4-tBu | COOMe | | |
| 17- | 22 | 4-CF3 | COOMe | | |
| 17- | 23 | 4-MeO | COOMe | | |
| 17- | 24 | 4-EtO | COOMe | | |
| 17- | 25 | 4-nPrO | COOMe | | |
| 17- | 26 | 4-nBuO | COOMe | | |
| 17- | 27 | 4-OCHF2 | COOMe | | |
| 17- | 28 | 4-OCF3 | COOMe | | |
| 17- | 29 | 4-OCH2CF3 | COOMe | | |
| 17- | 30 | 4-OC2F4H | COOMe | | |
| 17- | 31 | 4-OC3F6H | COOMe | | |
| 17- | 32 | 4-SMe | COOMe | | |
| 17- | 33 | 4-SOMe | COOMe | | |
| 17- | 34 | 4-SO2Me | COOMe | | |
| 17- | 35 | 4-SEt | COOMe | | |
| 17- | 36 | 4-SOEt | COOMe | | |
| 17- | 37 | 4-SO2Et | COOMe | | |
| 17- | 38 | 4-SCF3 | COOMe | | |
| 17- | 39 | 4-SOCF3 | COOMe | | |
| 17- | 40 | 4-SO2CF3 | COOMe | | |
| 17- | 41 | 4-CN | COOMe | | |
| 17- | 42 | 4-NO2 | COOMe | | |
| 17- | 43 | 4-NMe2 | COOMe | | |
| 17- | 44 | 2,3-F2 | COOMe | | |
| 17- | 45 | 2,4-F2 | COOMe | | |
| 17- | 46 | 2,5-F2 | COOMe | | |
| 17- | 47 | 2,6-F2 | COOMe | | |
| 17- | 48 | 3,4-F2 | COOMe | | |
| 17- | 49 | 3,5-F2 | COOMe | | |
| 17- | 50 | 2,3-Cl2 | COOMe | | |
| 17- | 51 | 2,4-Cl2 | COOMe | | |
| 17- | 52 | 2,5-Cl2 | COOMe | | |
| 17- | 53 | 2,6-Cl2 | COOMe | | |
| 17- | 54 | 3,4-Cl2 | COOMe | | |
| 17- | 55 | 2,5-Cl2 | COOMe | | |
| 17- | 56 | 2-Cl-4-CF3 | COOMe | | |
| 17- | 57 | 2-Cl-4-F | COOMe | | |
| 17- | 58 | H | COOEt | 24228-40-8 | |
| 17- | 59 | 2-F | COOEt | | |
| 17- | 60 | 2-Cl | COOEt | | |
| 17- | 61 | 2-Br | COOEt | | |
| 17- | 62 | 2-I | COOEt | | |
| 17- | 63 | 3-F | COOEt | | |
| 17- | 64 | 3-Cl | COOEt | | |
| 17- | 65 | 3-Br | COOEt | | |
| 17- | 66 | 3-I | COOEt | | |
| 17- | 67 | 4-F | COOEt | | |
| 17- | 68 | 4-Cl | COOEt | | |
| 17- | 69 | 4-Br | COOEt | | |
| 17- | 70 | 4-I | COOEt | | |
| 17- | 71 | 4-Me | COOEt | | |
| 17- | 72 | 4-Et | COOEt | | |
| 17- | 73 | 4-nPr | COOEt | | |
| 17- | 74 | 4-iPr | COOEt | | |
| 17- | 75 | 4-nBu | COOEt | | |
| 17- | 76 | 4-sec-Bu | COOEt | | |
| 17- | 77 | 4-iBu | COOEt | | |
| 17- | 78 | 4-tBu | COOEt | | |
| 17- | 79 | 4-CF3 | COOEt | | |
| 17- | 80 | 4-MeO | COOEt | | |
| 17- | 81 | 4-EtO | COOEt | | |
| 17- | 82 | 4-nPrO | COOEt | | |
| 17- | 83 | 4-nBuO | COOEt | | |
| 17- | 84 | 4-OCHF2 | COOEt | | |
| 17- | 85 | 4-OCF3 | COOEt | | |
| 17- | 86 | 4-OCH2CF3 | COOEt | | |
| 17- | 87 | 4-OC2F4H | COOEt | | |
| 17- | 88 | 4-OC3F6H | COOEt | | |
| 17- | 89 | 4-SMe | COOEt | | |
| 17- | 90 | 4-SOMe | COOEt | | |
| 17- | 91 | 4-SO2Me | COOEt | | |
| 17- | 92 | 4-SEt | COOEt | | |
| 17- | 93 | 4-SOEt | COOEt | | |
| 17- | 94 | 4-SO2Et | COOEt | | |
| 17- | 95 | 4-SCF3 | COOEt | | |
| 17- | 96 | 4-SOCF3 | COOEt | | |
| 17- | 97 | 4-SO2CF3 | COOEt | | |
| 17- | 98 | 4-CN | COOEt | | |
| 17- | 99 | 4-NO2 | COOEt | | |
| 17- | 100 | 4-NMe2 | COOEt | | |
| 17- | 101 | 2,3-F2 | COOEt | | |
| 17- | 102 | 2,4-F2 | COOEt | | |
| 17- | 103 | 2,5-F2 | COOEt | | |
| 17- | 104 | 2,6-F2 | COOEt | | |
| 17- | 105 | 3,4-F2 | COOEt | | |
| 17- | 106 | 3,5-F2 | COOEt | | |
| 17- | 107 | 2,3-CI2 | COOEt | | |
| 17- | 108 | 2,4-CI2 | COOEt | | 1H: 1,24; 1,81; 2,13; 2,29; 2,83; 3,53; 4,13; 7,21; 7,34; 7,42; |
| 17- | 109 | 2,5-Cl2 | COOEt | | |
| 17- | 110 | 2,6-Cl2 | COOEt | | |
| 17- | 111 | 3,4-Cl2 | COOEt | | |
| 17- | 112 | 2,5-Cl2 | COOEt | | |
| 17- | 113 | 2-Cl-4-CF3 | COOEt | | |
| 17- | 114 | 2-Cl-4-F | COOEt | | |
| 17- | 115 | H | COOnPr | | |
| 17- | 116 | 2-F | COOnPr | | |
| 17- | 117 | 2-Cl | COOnPr | | |
| 17- | 118 | 2-Br | COOnPr | | |
| 17- | 119 | 2-I | COOnPr | | |
| 17- | 120 | 3-F | COOnPr | | |
| 17- | 121 | 3-Cl | COOnPr | | |
| 17- | 122 | 3-Br | COOnPr | | |
| 17- | 123 | 3-I | COOnPr | | |
| 17- | 124 | 4-F | COOnPr | | |
| 17- | 125 | 4-Cl | COOnPr | | |
| 17- | 126 | 4-Br | COOnPr | | |
| 17- | 127 | 4-I | COOnPr | | |

**Table 18: Compounds of Formula (Ic) in which the substituents have the following meanings:R¹ is Phenyl substituted by R; (A)n is CH2; R² and R³ are each H; R⁶ is optionally substituted CH2Phenyl and R⁷ is H;**

| Compound number | | R | R6 | CA REG No | NMR |
|---|---|---|---|---|---|
| 18- | 01 | H | CH2C6H5 | | |
| 18- | 02 | 2-F | CH2C6H5 | | |
| 18- | 03 | 2-Cl | CH2C6H5 | | |
| 18- | 04 | 2-Br | CH2C6H5 | | |
| 18- | 05 | 2-I | CH2C6H5 | | |
| 18- | 06 | 3-F | CH2C6H5 | | |
| 18- | 07 | 3-Cl | CH2C6H5 | | |
| 18- | 08 | 3-Br | CH2C6H5 | | |
| 18- | 09 | 3-I | CH2C6H5 | | |
| 18- | 10 | 4-F | CH2C6H5 | | |
| 18- | 11 | 4-Cl | CH2C6H5 | | |
| 18- | 12 | 4-Br | CH2C6H5 | | |
| 18- | 13 | 4-I | CH2C6H5 | | |
| 18- | 14 | 4-Me | CH2C6H5 | | |
| 18- | 15 | 4-Et | CH2C6H5 | | |
| 18- | 16 | 4-nPr | CH2C6H5 | | |
| 18- | 17 | 4-iPr | CH2C6H5 | | |
| 18- | 18 | 4-nBu | CH2C6H5 | | |
| 18- | 19 | 4-sec-Bu | CH2C6H5 | | |
| 18- | 20 | 4-iBu | CH2C6H5 | | |
| 18- | 21 | 4-tBu | CH2C6H5 | | |
| 18- | 22 | 4-CF3 | CH2C6H5 | | |
| 18- | 23 | 4-MeO | CH2C6H5 | | |
| 18- | 24 | 4-EtO | CH2C6H5 | | |
| 18- | 25 | 4-nPrO | CH2C6H5 | | |
| 18- | 26 | 4-nBuO | CH2C6H5 | | |
| 18- | 27 | 4-OCHF2 | CH2C6H5 | | |
| 18- | 28 | 4-OCF3 | CH2C6H5 | | |
| 18- | 29 | 4-OCH2CF3 | CH2C6H5 | | |
| 18- | 30 | 4-OC2F4H | CH2C6H5 | | |
| 18- | 31 | 4-OC3F6H | CH2C6H5 | | |
| 18- | 32 | 4-SMe | CH2C6H5 | | |
| 18- | 33 | 4-SOMe | CH2C6H5 | | |
| 18- | 34 | 4-SO2Me | CH2C6H5 | | |
| 18- | 35 | 4-SEt | CH2C6H5 | | |
| 18- | 36 | 4-SOEt | CH2C6H5 | | |
| 18- | 37 | 4-SO2Et | CH2C6H5 | | |
| 18- | 38 | 4-SCF3 | CH2C6H5 | | |
| 18- | 39 | 4-SOCF3 | CH2C6H5 | | |
| 18- | 40 | 4-SO2CF3 | CH2C6H5 | | |
| 18- | 41 | 4-CN | CH2C6H5 | | |
| 18- | 42 | 4-NO2 | CH2C6H5 | | |
| 18- | 43 | 4-NMe2 | CH2C6H5 | | |
| 18- | 44 | 2,3-F2 | CH2C6H5 | | |
| 18- | 45 | 2,4-F2 | CH2C6H5 | | |
| 18- | 46 | 2,5-F2 | CH2C6H5 | | |
| 18- | 47 | 2,6-F2 | CH2C6H5 | | |
| 18- | 48 | 3,4-F2 | CH2C6H5 | | |
| 18- | 49 | 3,5-F2 | CH2C6H5 | | |
| 18- | 50 | 2,3-Cl2 | CH2C6H5 | | |
| 18- | 51 | 2,4-Cl2 | CH2C6H5 | | |
| 18- | 52 | 2,5-Cl2 | CH2C6H5 | | |
| 18- | 53 | 2,6-Cl2 | CH2C6H5 | | |
| 18- | 54 | 3,4-Cl2 | CH2C6H5 | | |
| 18- | 55 | 3,5-Cl2 | CH2C6H5 | | |
| 18- | 56 | 2-Cl-4-CF3 | CH2C6H5 | | |
| 18- | 57 | 2-Cl-4-F | CH2C6H5 | | |
| 18- | 58 | H | CH2-4-ClC6H4 | | |
| 18- | 59 | 2-F | CH2-4-ClC6H4 | | |
| 18- | 60 | 2-Cl | CH2-4-ClC6H4 | | |
| 18- | 61 | 2-Br | CH2-4-ClC6H4 | | |
| 18- | 62 | 2-I | CH2-4-ClC6H4 | | |
| 18- | 63 | 3-F | CH2-4-ClC6H4 | | |
| 18- | 64 | 3-Cl | CH2-4-ClC6H4 | | |
| 18- | 65 | 3-Br | CH2-4-ClC6H4 | | |
| 18- | 66 | 3-I | CH2-4-ClC6H4 | | |
| 18- | 67 | 4-F | CH2-4-ClC6H4 | | |
| 18- | 68 | 4-Cl | CH2-4-ClC6H4 | | |
| 18- | 69 | 4-Br | CH2-4-ClC6H4 | | |
| 18- | 70 | 4-I | CH2-4-ClC6H4 | | |
| 18- | 71 | 4-Me | CH2-4-ClC6H4 | | |
| 18- | 72 | 4-Et | CH2-4-ClC6H4 | | |
| 18- | 73 | 4-nPr | CH2-4-ClC6H4 | | |
| 18- | 74 | 4-iPr | CH2-4-ClC6H4 | | |
| 18- | 75 | 4-nBu | CH2-4-ClC6H4 | | |
| 18- | 76 | 4-sec-Bu | CH2-4-ClC6H4 | | |
| 18- | 77 | 4-iBu | CH2-4-ClC6H4 | | |
| 18- | 78 | 4-tBu | CH2-4-ClC6H4 | | |
| 18- | 79 | 4-CF3 | CH2-4-ClC6H4 | | |
| 18- | 80 | 4-MeO | CH2-4-ClC6H4 | | |
| 18- | 81 | 4-EtO | CH2-4-ClC6H4 | | |
| 18- | 82 | 4-nPrO | CH2-4-ClC6H4 | | |
| 18- | 83 | 4-nBuO | CH2-4-ClC6H4 | | |
| 18- | 84 | 4-OCHF2 | CH2-4-ClC6H4 | | |
| 18- | 85 | 4-OCF3 | CH2-4-ClC6H4 | | |
| 18- | 86 | 4-OCH2CF3 | CH2-4-ClC6H4 | | |
| 18- | 87 | 4-OC2F4H | CH2-4-ClC6H4 | | |
| 18- | 88 | 4-OC3F6H | CH2-4-ClC6H4 | | |
| 18- | 89 | 4-SMe | CH2-4-ClC6H4 | | |
| 18- | 90 | 4-SOMe | CH2-4-ClC6H4 | | |
| 18- | 91 | 4-SO2Me | CH2-4-ClC6H4 | | |
| 18- | 92 | 4-SEt | CH2-4-ClC6H4 | | |
| 18- | 93 | 4-SOEt | CH2-4-ClC6H4 | | |
| 18- | 94 | 4-SO2Et | CH2-4-ClC6H4 | | |
| 18- | 95 | 4-SCF3 | CH2-4-ClC6H4 | | |
| 18- | 96 | 4-SOCF3 | CH2-4-ClC6H4 | | |
| 18- | 97 | 4-SO2CF3 | CH2-4-ClC6H4 | | |
| 18- | 98 | 4-CN | CH2-4-ClC6H4 | | |
| 18- | 99 | 4-NO2 | CH2-4-ClC6H4 | | |
| 18- | 100 | 4-NMe2 | CH2-4-ClC6H4 | | |
| 18- | 101 | 2,3-F2 | CH2-4-ClC6H4 | | |
| 18- | 102 | 2,4-F2 | CH2-4-ClC6H4 | | |
| 18- | 103 | 2,5-F2 | CH2-4-ClC6H4 | | |
| 18- | 104 | 2,6-F2 | CH2-4-ClC6H4 | | |
| 18- | 105 | 3,4-F2 | CH2-4-ClC6H4 | | |
| 18- | 106 | 3,5-F2 | CH2-4-ClC6H4 | | |
| 18- | 107 | 2,3-Cl2 | CH2-4-ClC6H4 | | |
| 18- | 108 | 2,4-Cl2 | CH2-4-ClC6H4 | | |
| 18- | 109 | 2,5-Cl2 | CH2-4-ClC6H4 | | |
| 18- | 110 | 2,6-Cl2 | CH2-4-ClC6H4 | | |
| 18- | 111 | 3,4-Cl2 | CH2-4-ClC6H4 | | |
| 18- | 112 | 2,5-Cl2 | CH2-4-ClC6H4 | | |
| 18- | 113 | 2-Cl-4-CF3 | CH2-4-ClC6H4 | | |
| 18- | 114 | 2-Cl-4-F | CH2-4-ClC6H4 | | |

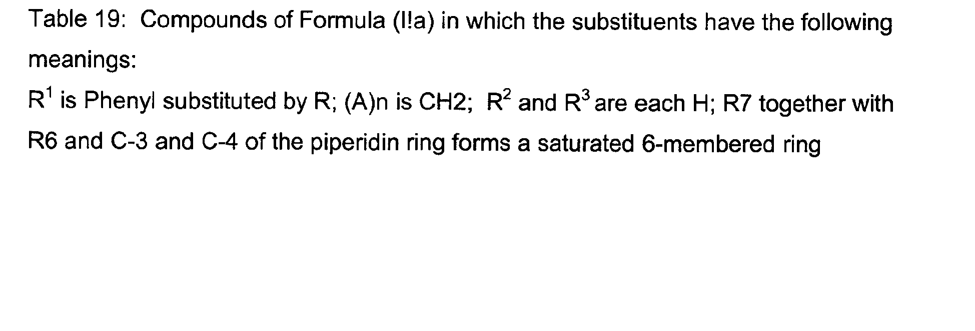

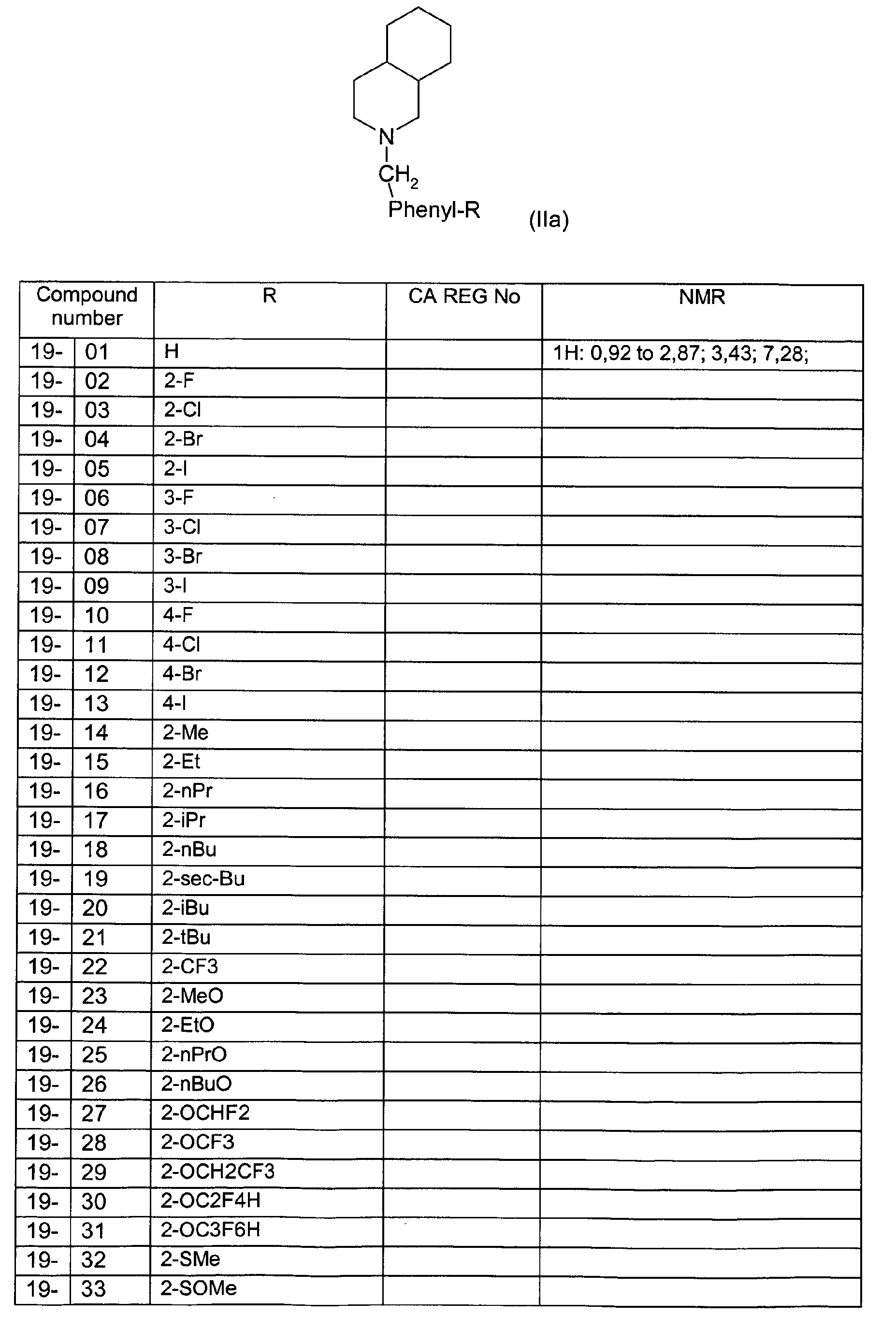

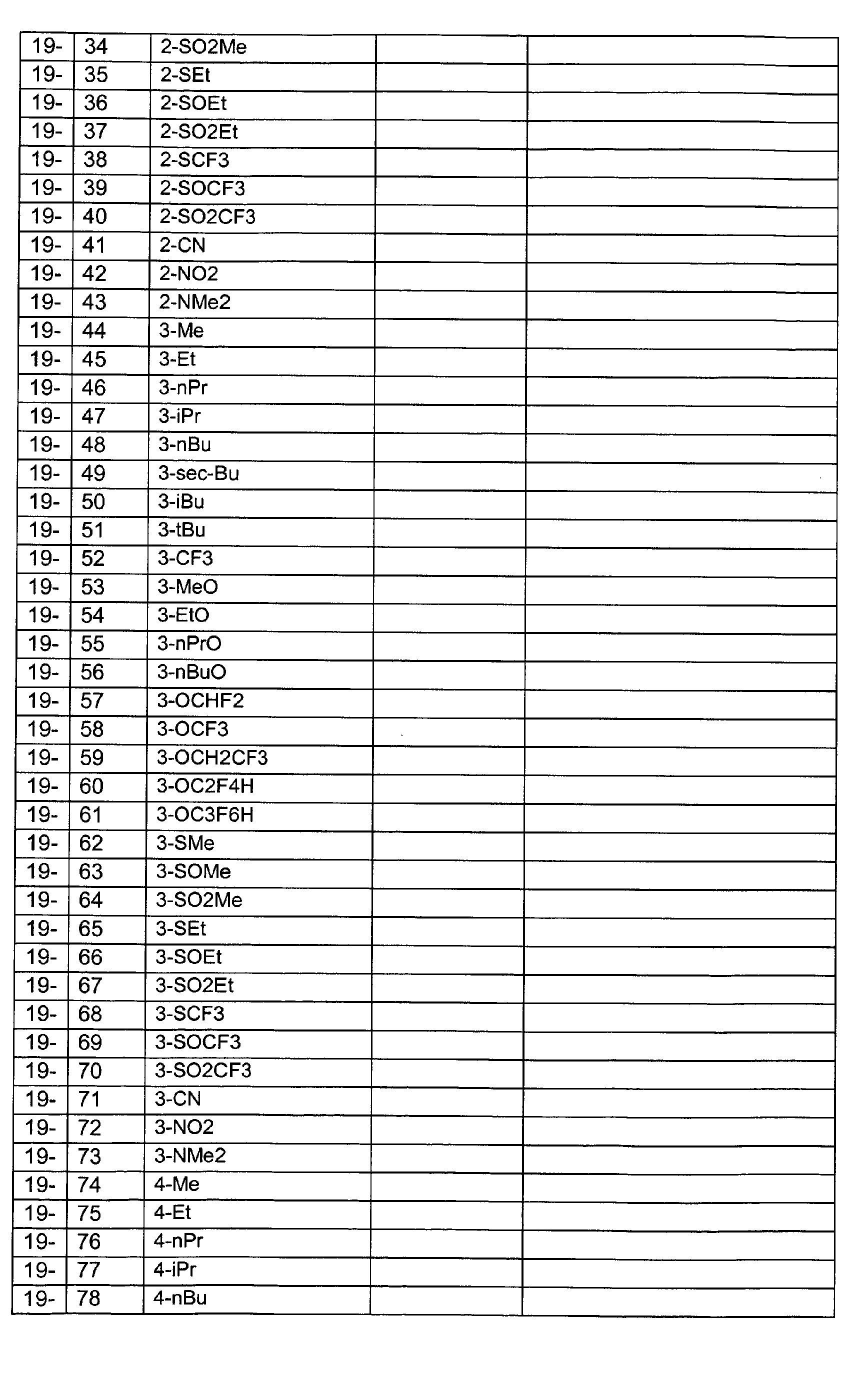

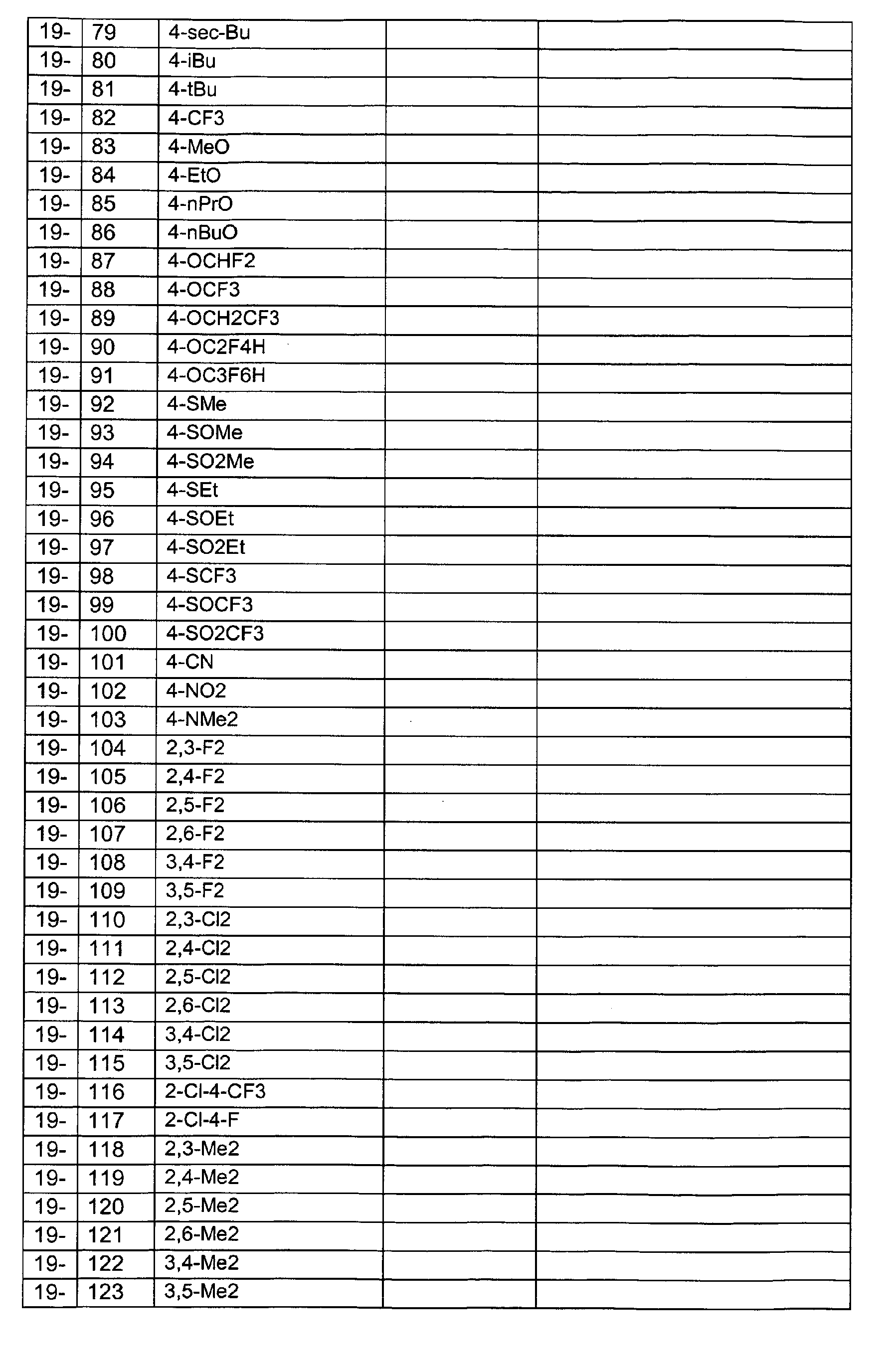

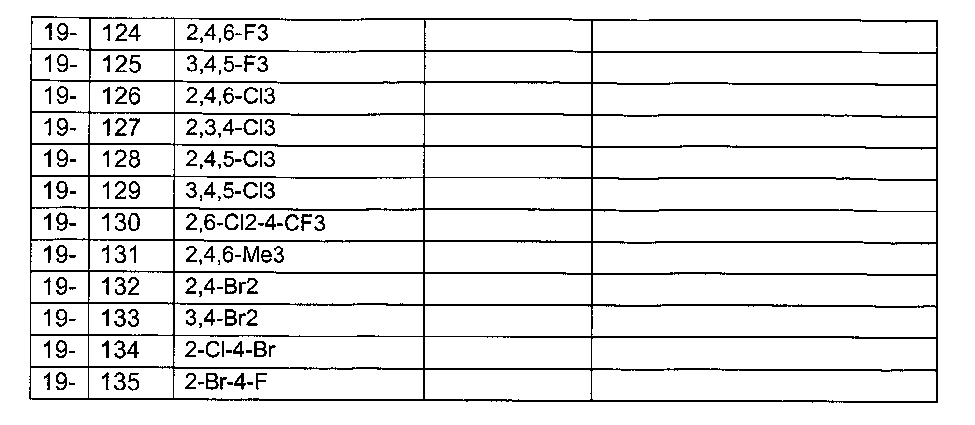

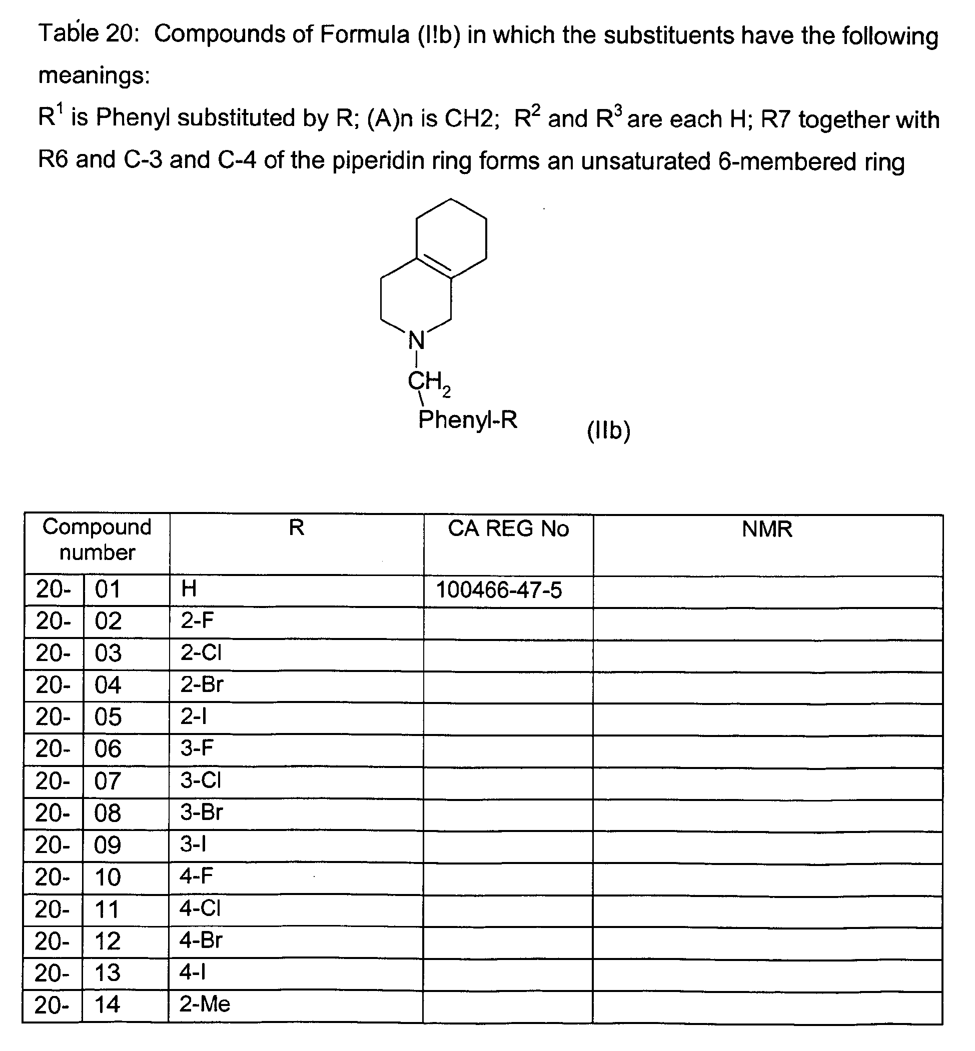

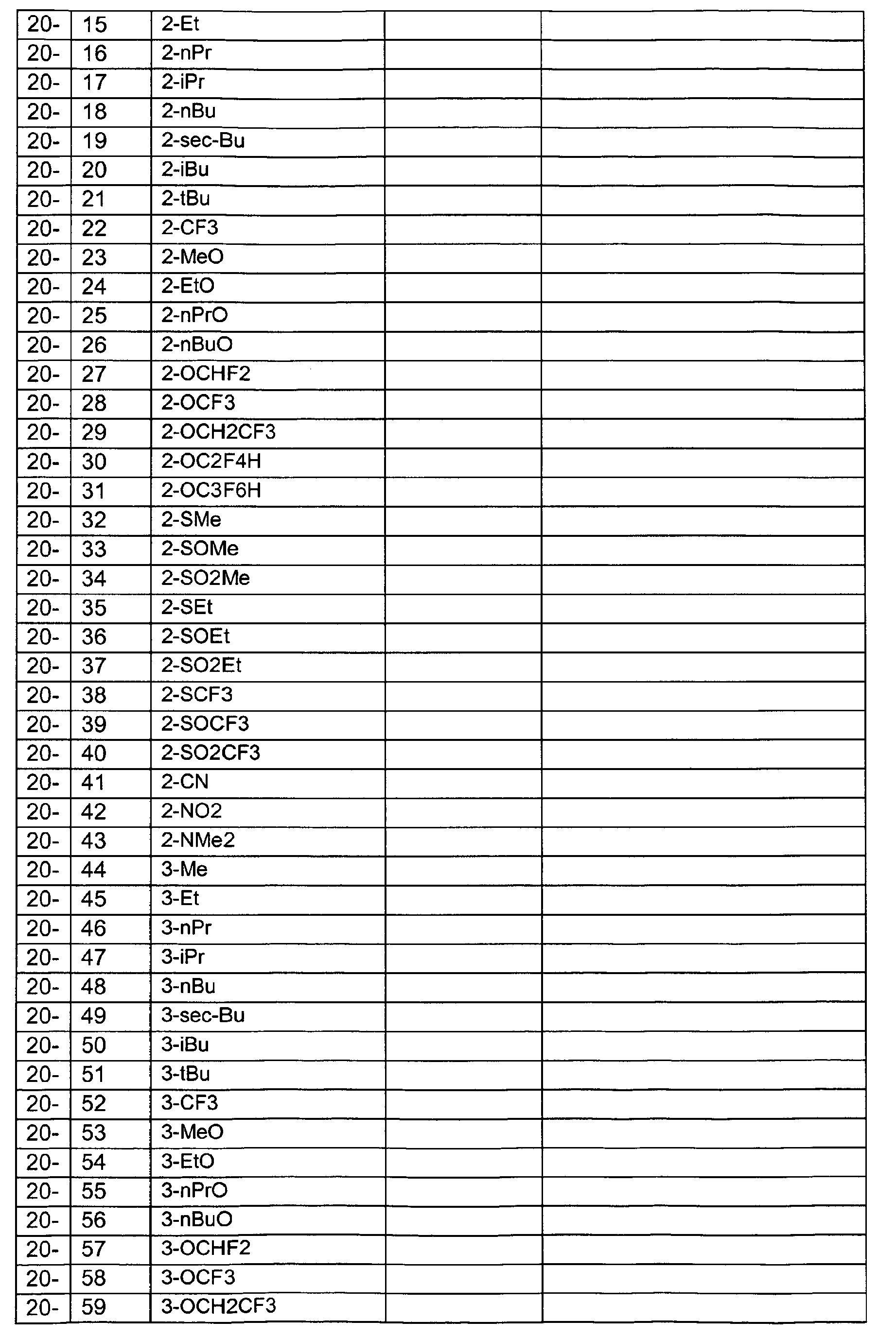

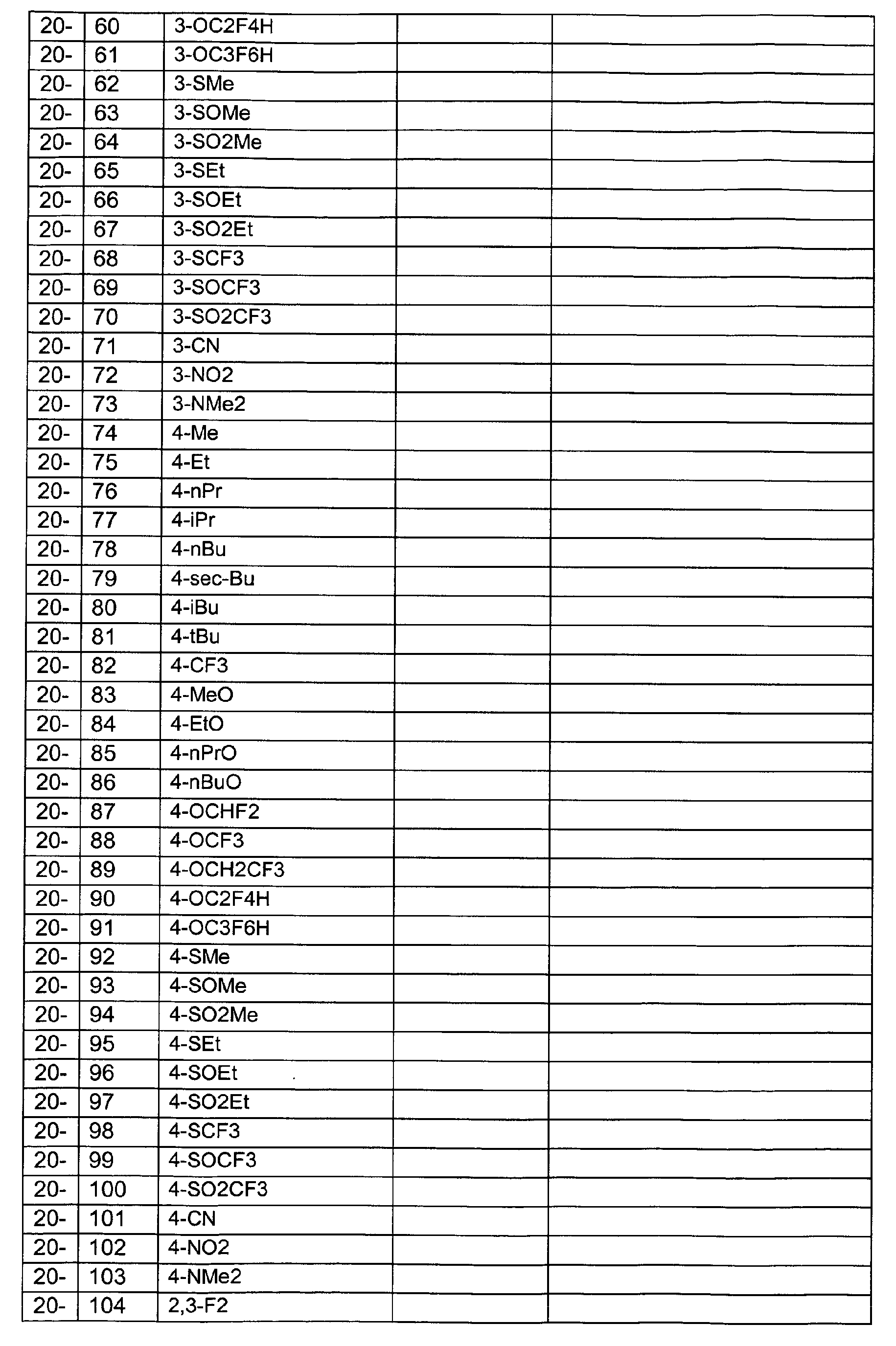

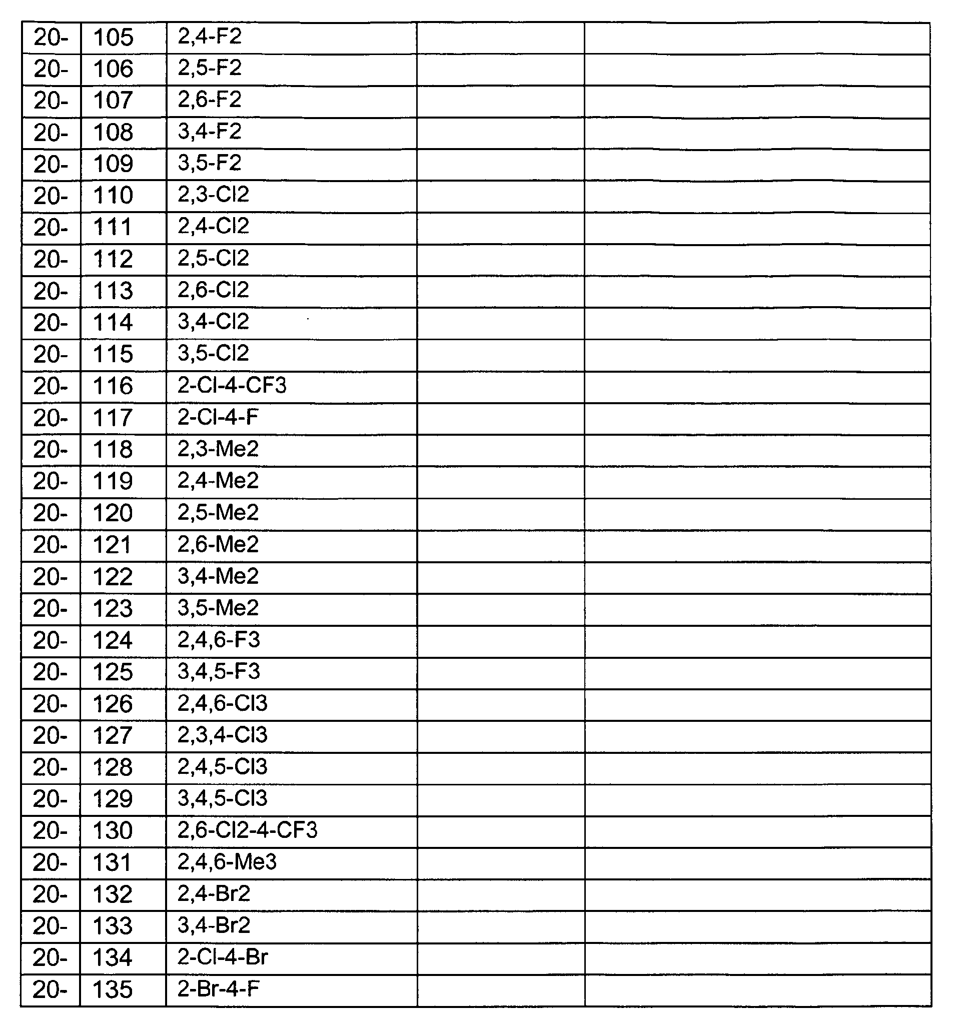

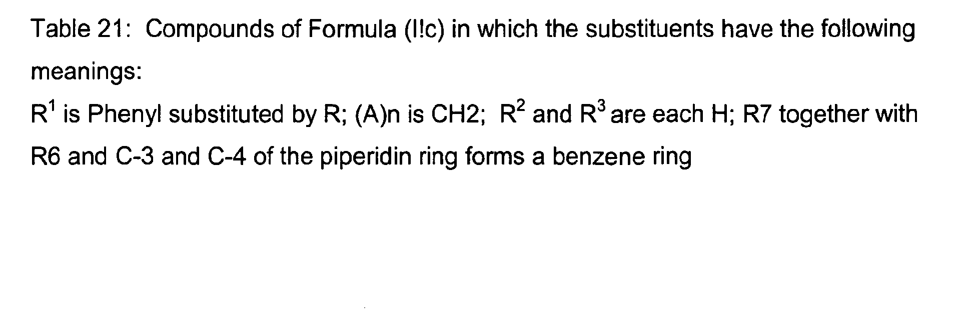

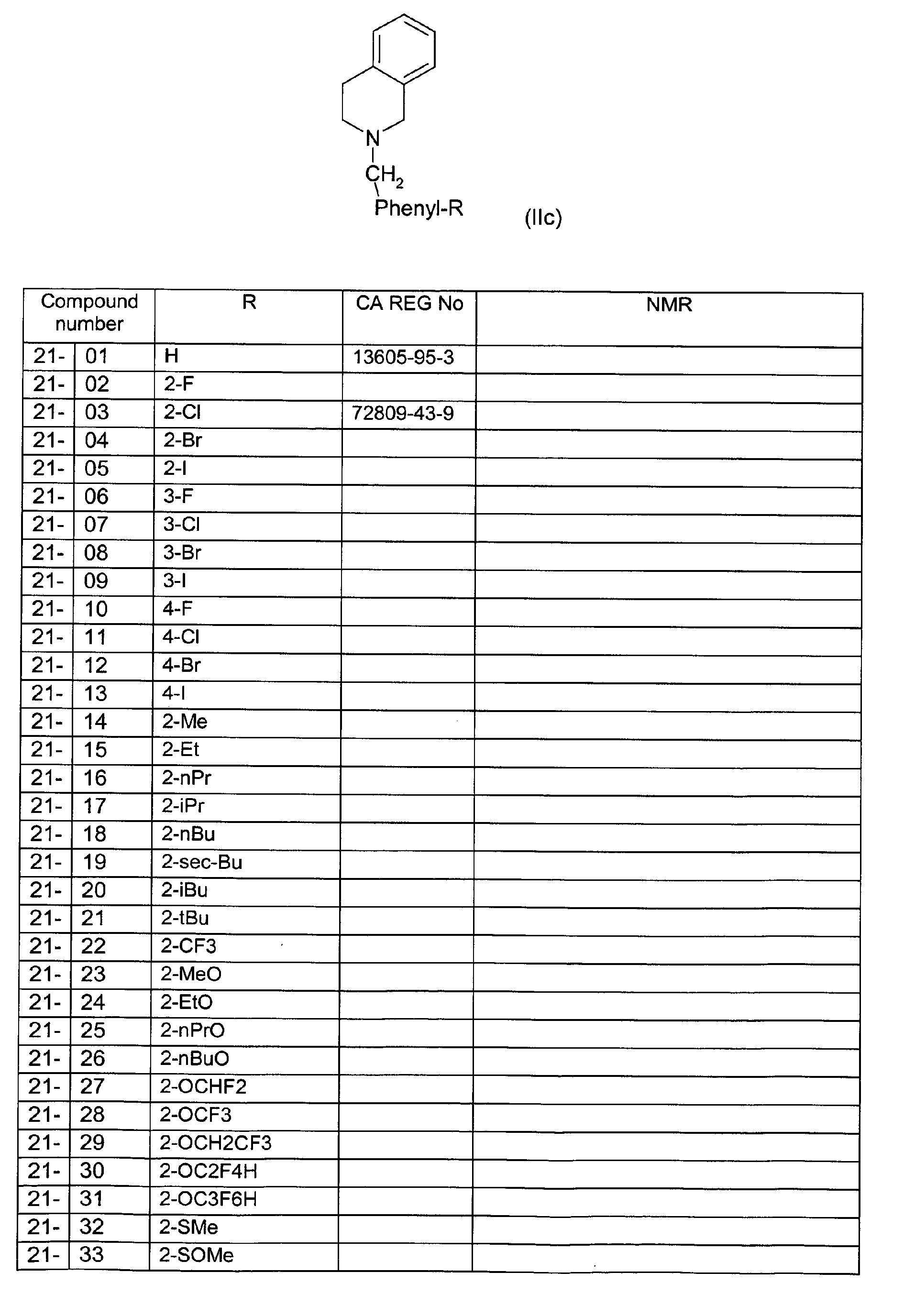

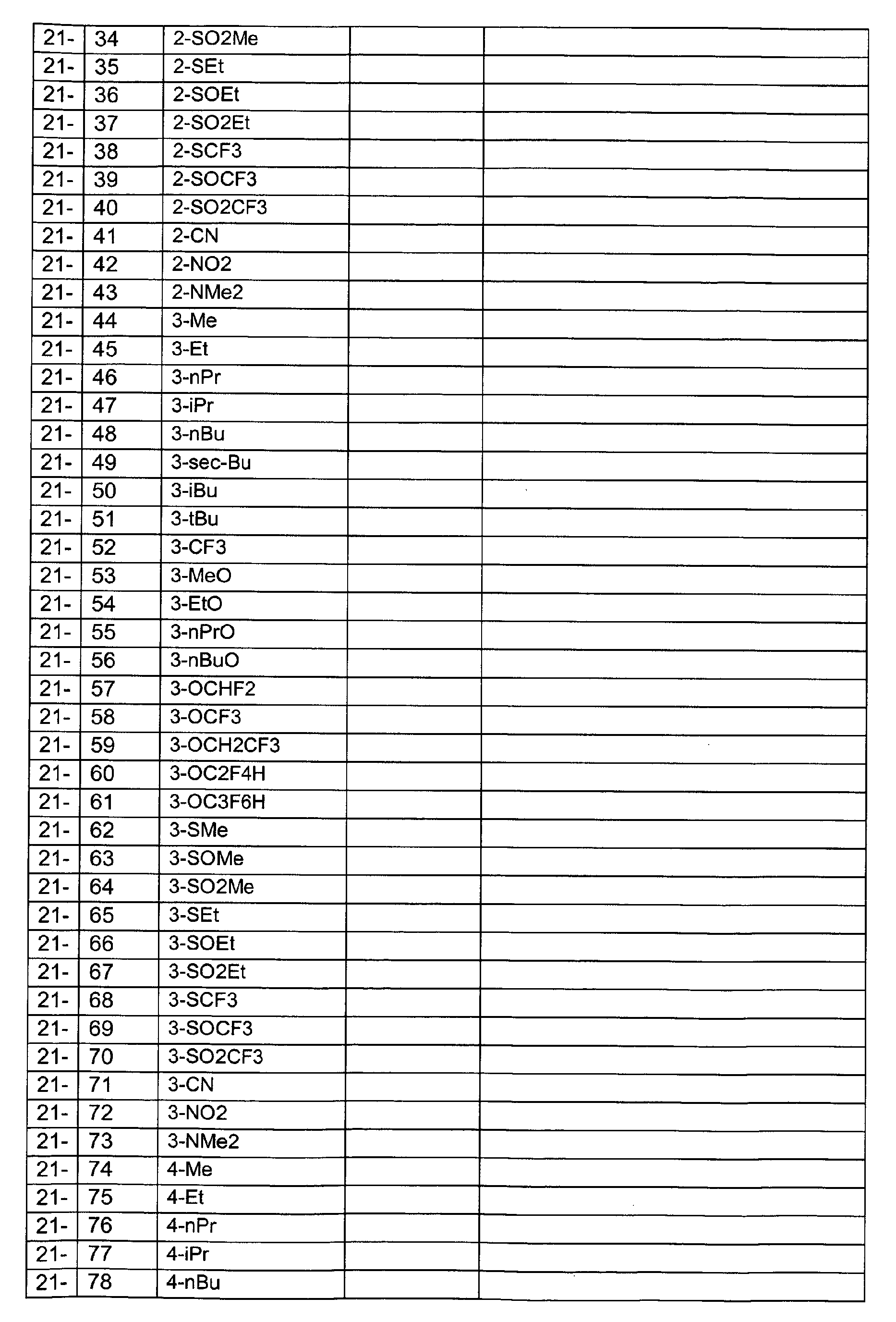

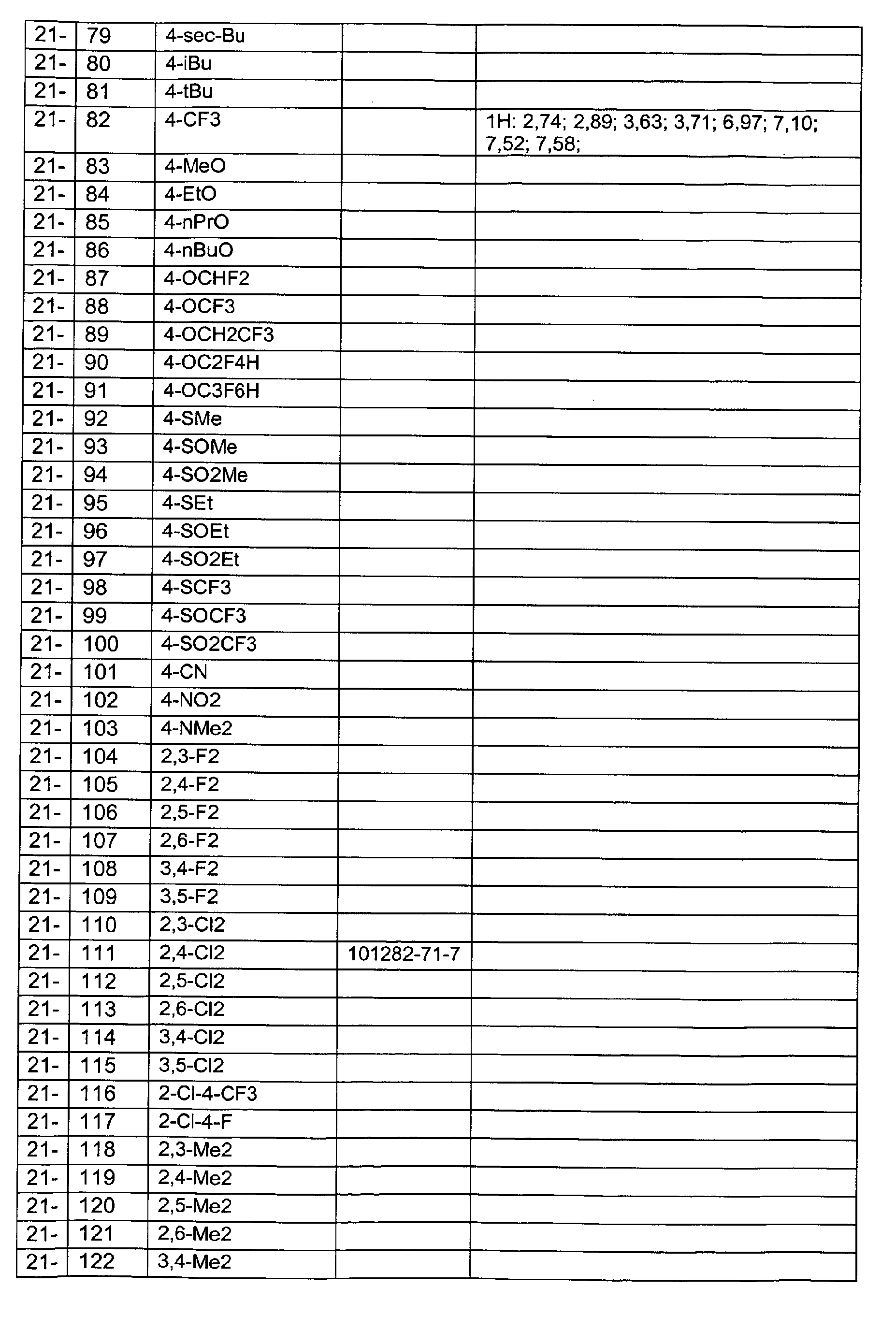

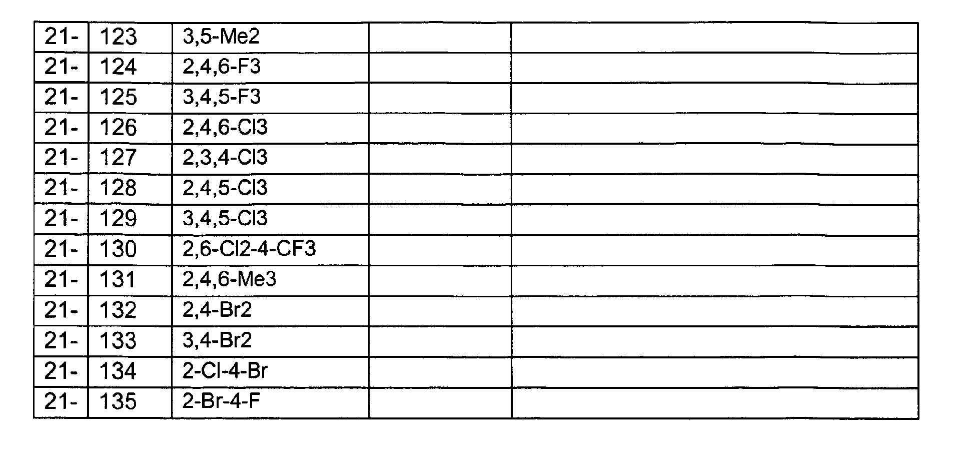

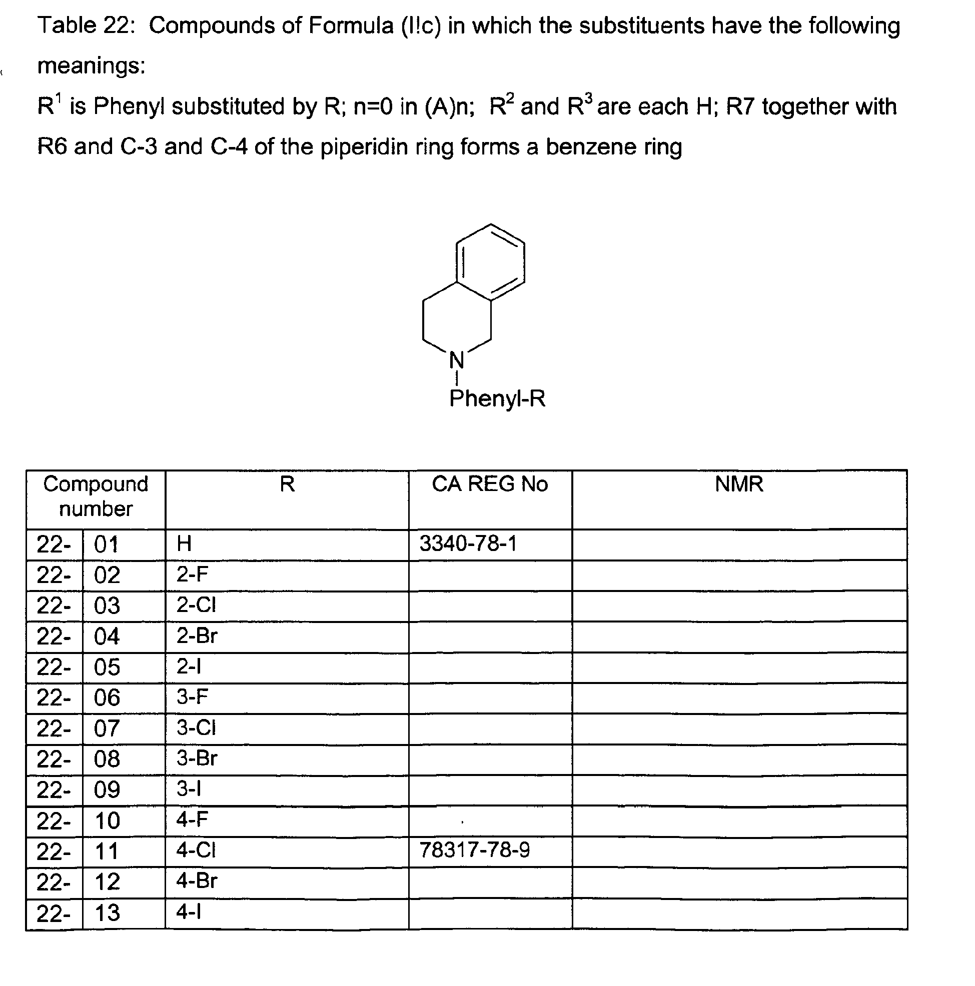

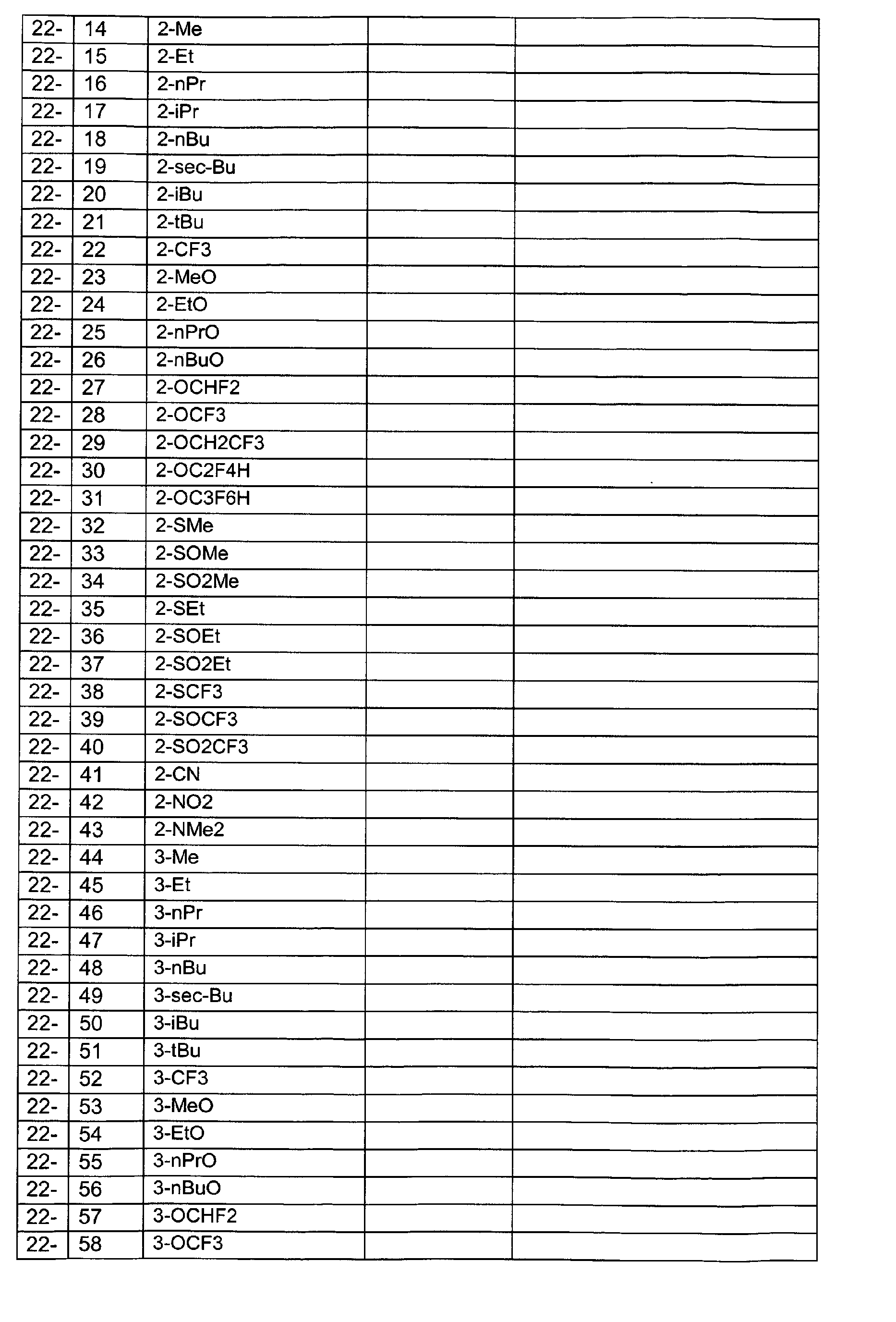

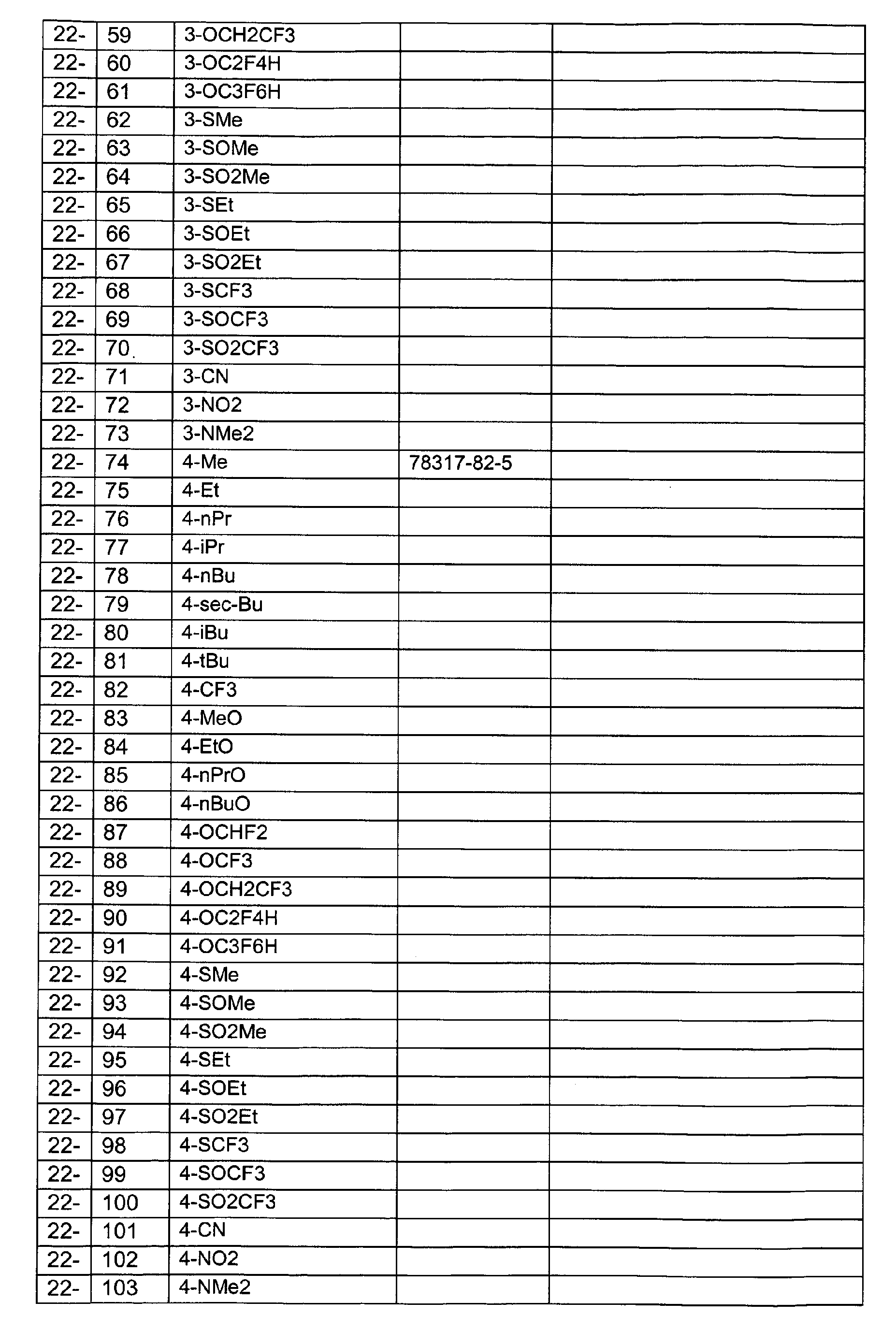

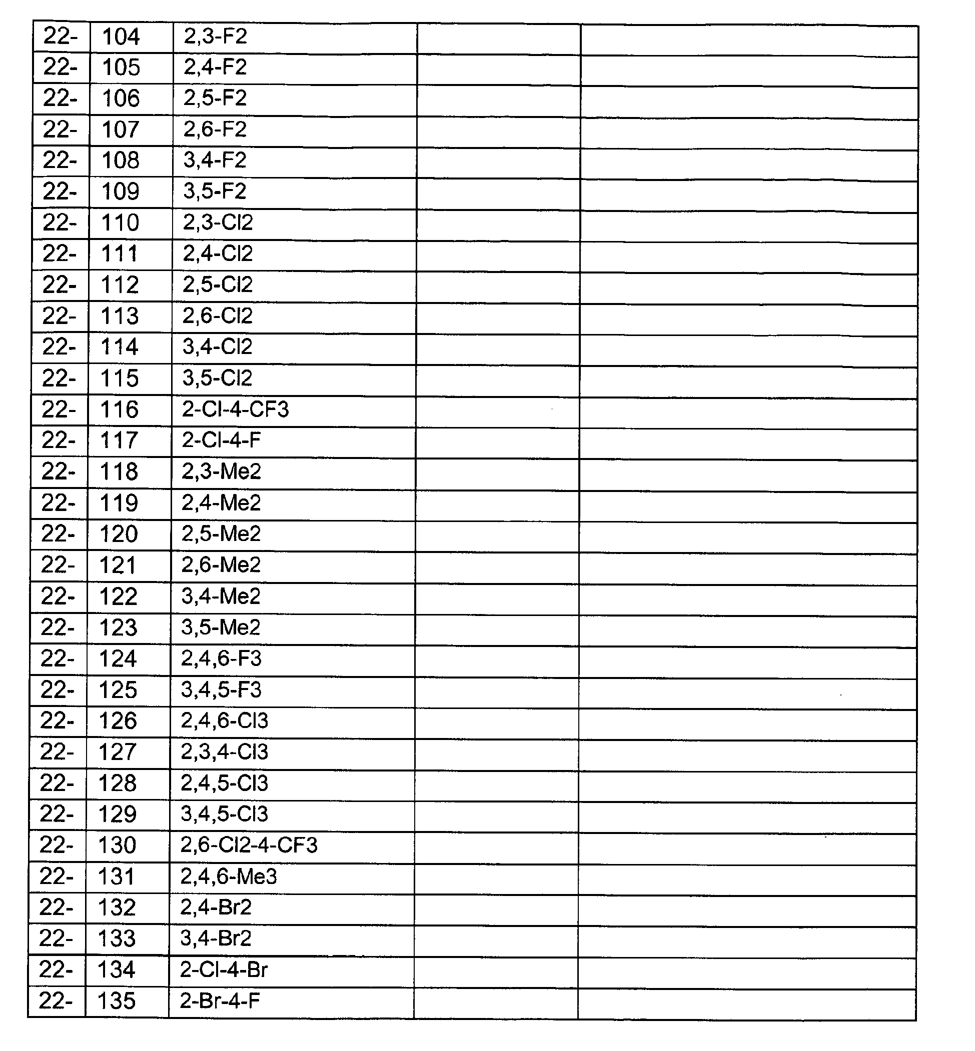

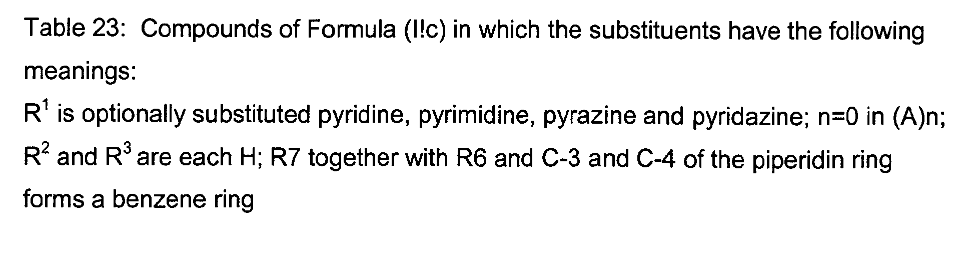

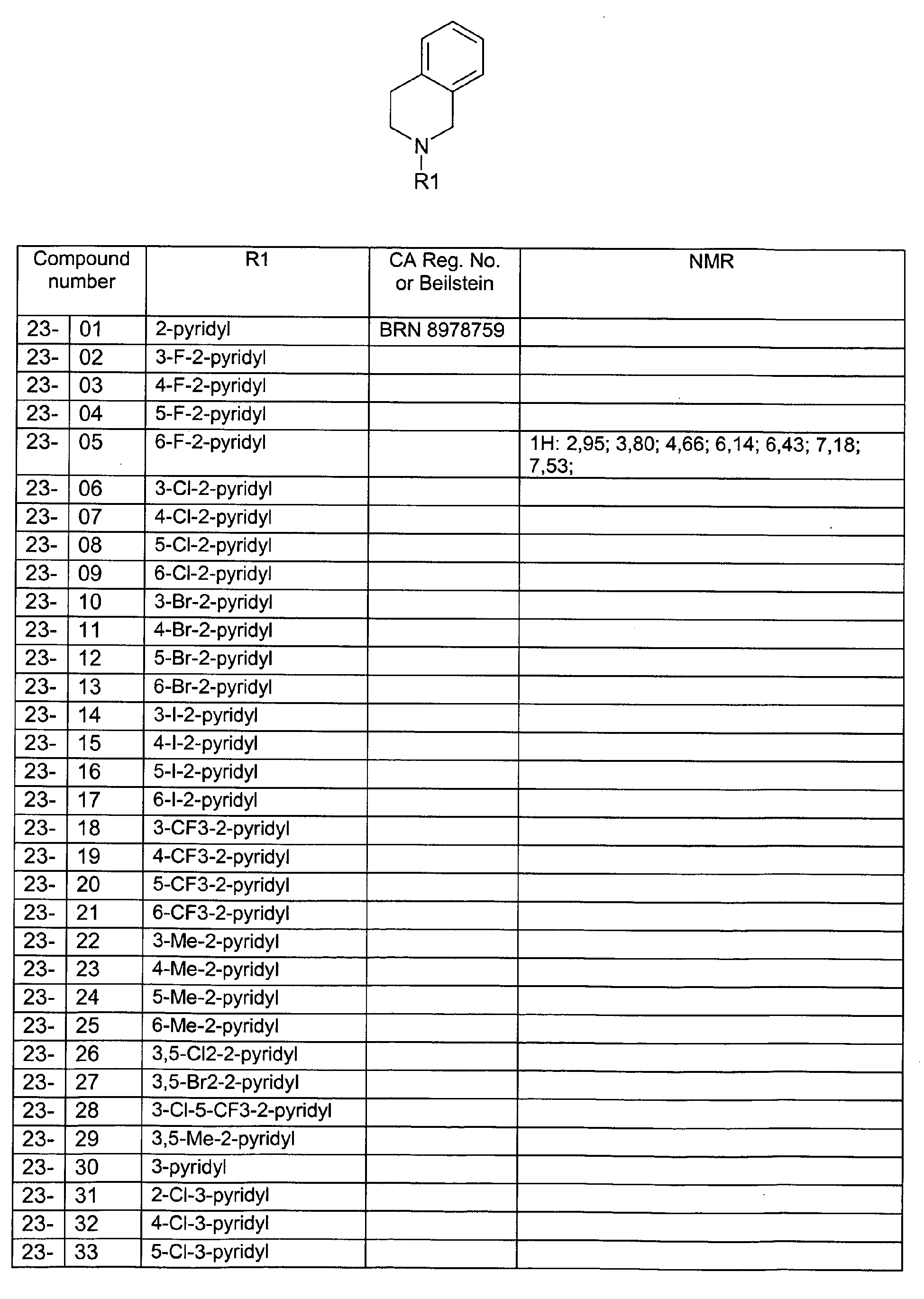

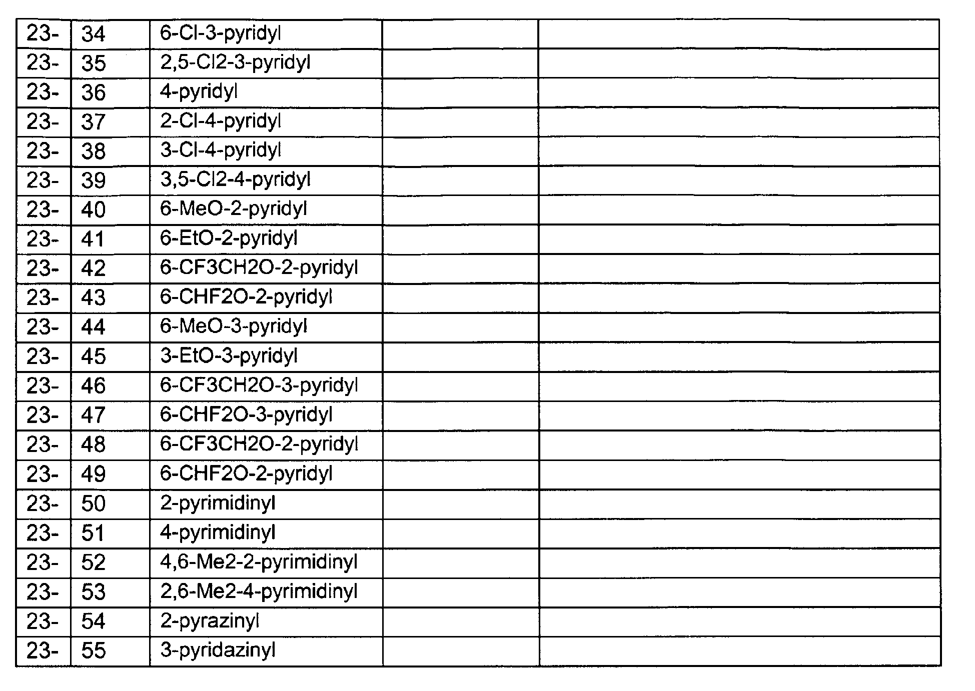

**Table 24: Compounds of Formula (Ib) in which the substituents have the following meanings: R¹ is Phenyl substituted by R; n=0 in (A)n ; R² and R³ are each H; R⁴ and R⁵ together with C-4 of the piperidine ring form a 5-6 membered ring;**

| Compound number | | R | R4-R5 | CA REG No | NMR |
|---|---|---|---|---|---|
| 24- | 01 | H | O-C2H4-O | | |
| 24- | 02 | 2-F | O-C2H4-O | | |
| 24- | 03 | 2-Cl | O-C2H4-O | | |
| 24- | 04 | 2-Br | O-C2H4-O | | |
| 24- | 05 | 2-I | O-C2H4-O | | |
| 24- | 06 | 3-F | O-C2H4-O | | |
| 24- | 07 | 3-Cl | O-C2H4-O | | |
| 24- | 08 | 3-Br | O-C2H4-O | | |
| 24- | 09 | 3-I | O-C2H4-O | | |
| 24- | 10 | 4-F | O-C2H4-O | | |
| 24- | 11 | 4-Cl | O-C2H4-O | | |
| 24- | 12 | 4-Br | O-C2H4-O | | |
| 24- | 13 | 4-I | O-C2H4-O | | |
| 24- | 14 | 4-Me | O-C2H4-O | | |
| 24- | 15 | 4-Et | O-C2H4-O | | |
| 24- | 16 | 4-nPr | O-C2H4-O | | |
| 24- | 17 | 4-iPr | O-C2H4-O | | |
| 24- | 18 | 4-nBu | O-C2H4-O | | |
| 24- | 19 | 4-sec-Bu | O-C2H4-O | | |
| 24- | 20 | 4-iBu | O-C2H4-O | | |
| 24- | 21 | 4-tBu | O-C2H4-O | | |
| 24- | 22 | 4-CF3 | O-C2H4-O | | |
| 24- | 23 | 4-MeO | O-C2H4-O | | |
| 24- | 24 | 4-EtO | O-C2H4-O | | |
| 24- | 25 | 4-nPrO | O-C2H4-0 | | |
| 24- | 26 | 4-nBuO | O-C2H4-O | | |
| 24- | 27 | 4-OCHF2 | O-C2H4-O | | |
| 24- | 28 | 4-OCF3 | O-C2H4-O | | |
| 24- | 29 | 4-OCH2CF3 | O-C2H4-O | | |
| 24- | 30 | 4-OC2F4H | O-C2H4-O | | |
| 24- | 31 | 4-OC3F6H | O-C2H4-O | | |
| 24- | 32 | 4-SMe | O-C2H4-O | | |
| 24- | 33 | 4-SOMe | O-C2H4-O | | |
| 24- | 34 | 4-SO2Me | O-C2H4-O | | |
| 24- | 35 | 4-SEt | O-C2H4-O | | |
| 24- | 36 | 4-SOEt | O-C2H4-O | | |
| 24- | 37 | 4-SO2Et | O-C2H4-O | | |
| 24- | 38 | 4-SCF3 | O-C2H4-O | | |
| 24- | 39 | 4-SOCF3 | O-C2H4-O | | |
| 24- | 40 | 4-SO2CF3 | O-C2H4-O | | |
| 24- | 41 | 4-CN | O-C2H4-O | | |
| 24- | 42 | 4-NO2 | O-C2H4-O | | |
| 24- | 43 | 4-NMe2 | O-C2H4-O | | |
| 24- | 44 | 2,3-F2 | O-C2H4-O | | |
| 24- | 45 | 2,4-F2 | O-C2H4-O | | |
| 24- | 46 | 2,5-F2 | O-C2H4-O | | |
| 24- | 47 | 2,6-F2 | O-C2H4-O | | |
| 24- | 48 | 3,4-F2 | O-C2H4-O | | |
| 24- | 49 | 3,5-F2 | O-C2H4-O | | |
| 24- | 50 | 2,3-Cl2 | O-C2H4-O | | |
| 24- | 51 | 2,4-Cl2 | O-C2H4-O | | |
| 24- | 52 | 2,5-Cl2 | O-C2H4-O | | |
| 24- | 53 | 2,6-CI2 | O-C2H4-O | | |
| 24- | 54 | 3,4-Cl2 | O-C2H4-O | | |
| 24- | 55 | 3,5-CI2 | O-C2H4-O | | |
| 24- | 56 | 2-CI-4-CF3 | O-C2H4-O | | 1 H: 1,91; 3,17; 4,01; 7,09; 7,45; 7,60; |
| 24- | 57 | 2-Cl-4-F | O-C2H4-O | | |
| 24- | 58 | H | O-C3H6-O | | |
| 24- | 59 | 2-F | O-C3H6-O | | |
| 24- | 60 | 2-Cl | O-C3H6-O | | |
| 24- | 61 | 2-Br | O-C3H6-O | | |
| 24- | 62 | 2-I | O-C3H6-O | | |
| 24- | 63 | 3-F | O-C3H6-O | | |
| 24- | 64 | 3-Cl | O-C3H6-O | | |
| 24- | 65 | 3-Br | O-C3H6-O | | |
| 24- | 66 | 3-I | O-C3H6-O | | |
| 24- | 67 | 4-F | O-C3H6-O | | |
| 24- | 68 | 4-Cl | O-C3H6-O | | |
| 24- | 69 | 4-Br | O-C3H6-O | | |
| 24- | 70 | 4-I | O-C3H6-O | | |
| 24- | 71 | 4-Me | O-C3H6-O | | |
| 24- | 72 | 4-Et | O-C3H6-O | | |
| 24- | 73 | 4-nPr | O-C3H6-O | | |
| 24- | 74 | 4-iPr | O-C3H6-O | | |
| 24- | 75 | 4-nBu | O-C3H6-O | | |
| 24- | 76 | 4-sec-Bu | O-C3H6-O | | |
| 24- | 77 | 4-iBu | O-C3H6-O | | |
| 24- | 78 | 4-tBu | O-C3H6-O | | |
| 24- | 79 | 4-CF3 | O-C3H6-O | | |
| 24- | 80 | 4-MeO | O-C3H6-O | | |
| 24- | 81 | 4-EtO | O-C3H6-O | | |
| 24- | 82 | 4-nPrO | O-C3H6-O | | |
| 24- | 83 | 4-nBuO | O-C3H6-O | | |
| 24- | 84 | 4-OCHF2 | O-C3H6-O | | |
| 24- | 85 | 4-OCF3 | O-C3H6-O | | |
| 24- | 86 | 4-OCH2CF3 | O-C3H6-O | | |
| 24- | 87 | 4-OC2F4H | O-C3H6-O | | |
| 24- | 88 | 4-OC3F6H | O-C3H6-O | | |
| 24- | 89 | 4-SMe | O-C3H6-O | | |
| 24- | 90 | 4-SOMe | O-C3H6-O | | |
| 24- | 91 | 4-SO2Me | O-C3H6-O | | |
| 24- | 92 | 4-SEt | O-C3H6-O | | |
| 24- | 93 | 4-SOEt | O-C3H6-O | | |
| 24- | 94 | 4-SO2Et | O-C3H6-O | | |
| 24- | 95 | 4-SCF3 | O-C3H6-O | | |
| 24- | 96 | 4-SOCF3 | O-C3H6-O | | |
| 24- | 97 | 4-SO2CF3 | O-C3H6-O | | |
| 24- | 98 | 4-CN | O-C3H6-O | | |
| 24- | 99 | 4-NO2 | O-C3H6-O | | |
| 24- | 100 | 4-NMe2 | O-C3H6-O | | |
| 24- | 101 | 2,3-F2 | O-C3H6-O | | |
| 24- | 102 | 2,4-F2 | O-C3H6-O | | |
| 24- | 103 | 2,5-F2 | O-C3H6-O | | |
| 24- | 104 | 2,6-F2 | O-C3H6-O | | |
| 24- | 105 | 3,4-F2 | O-C3H6-O | | |
| 24- | 106 | 3,5-F2 | O-C3H6-O | | |
| 24- | 107 | 2,3-Cl2 | O-C3H6-O | | |
| 24- | 108 | 2,4-Cl2 | O-C3H6-O | | |
| 24- | 109 | 2,5-Cl2 | O-C3H6-O | | |
| 24- | 110 | 2,6-Cl2 | O-C3H6-O | | |
| 24- | 111 | 3,4-Cl2 | O-C3H6-O | | |
| 24- | 112 | 2,5-Cl2 | O-C3H6-O | | |
| 24- | 113 | 2-Cl-4-CF3 | O-C3H6-O | | |
| 24- | 114 | 2-Cl-4-F | O-C3H6-O | | |

### Methods for pesticidal use:

The following representative test procedure, using compounds of the invention, was conducted to determine the parasiticidal activity of compounds of the invention.

### Biological Examples:

### Method A:

### Screening method to test contact activity against Rhipicephalus sanguineus (Brown dog tick)

Solutions of the test compounds were dropped onto filter paper, dried and the filter paper placed into test tubes and infested with 20-30 larvae (L1) of Rhipicephalus sanguineus and the tubes closed with a clip. The treated Rhipicephalus sanguineus were held in a climate chamber (25°C, 90% RH) and the percentage efficacy assessed 24 hours after application in comparison with the untreated control. Compound numbers 1-01, 1-03, 1-07, 1-08, 1-10, 1-11, 1-12, 1-13, 1-14, 1-22, 1-23, 1-44, 1-52, 1-53, 1-74, 1-75, 1-81, 1-82, 1-83, 1-92, 1-105, 1-107, 1-110, 1-111, 1-112, 1-113, 1-114, 1-115, 1-119, 1-122, 2-01, 2-11, 2-58, 2-68, 2-125, 3-01, 3-115, 4-34, 6-20, 7-01, 7-11, 9-04, 9-19, 10-44, 11-22, 13-11, 14-11, 14-82, 15-01, 16-01, 16-11, 16-82, 17-58, 17-79, 17-108, 18-01, 18-22, 19-01, 19-82, 19-111, 21-01, 21-82, 21-111, 21-123, 23-05, 23-20, 24-56 gave at least 80% contact control of Rhipicephalus sanguineus at a test concentration of 1000 ppm.

### Method B:

Screening method to test contact activity of compounds against Ctenocephalides felis (Cat flea)
Solutions of the test compounds were dropped onto filter paper, dried and the filter paper placed into test tubes and infested with 10 adults of Ctenocephalides felis. The treated Ctenocephalides felis were held in a climate chamber (26°C, 80% RH) and the percentage efficacy assessed 24 hours and 48 hours after application in comparison with the untreated control.
Compound numbers 1-01, 1-07, 1-08, 1-10, 1-11, 1-12, 1-14, 1-44, 1-52, 1,82, 1-105, 1-111, 1-119, 2-11, 9-04, 9-19 gave at least 70% contact control of Ctenocephalides felis at a test concentration of 1000 ppm.

## Claims

1. Use of substituted piperidine derivatives of formula (I) or a pesticidally acceptable salt thereof, for the control of pests 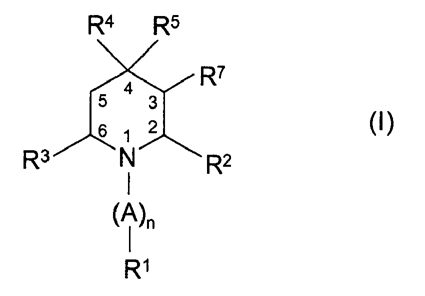 wherein:
A is a straight or branched (C₁-C₃)-alkylene or (C₁-C₃)-haloalkylene
R¹ is (C₆-C₁₄)-aryl, unsubstituted or substituted
by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or is (C₁-C₆)-alkyl, (C₃-C₇)-cycloalkyl, (C₃-C₇)-cycloalkenyl unsubstituted or substituted by one or more radicals selected from the group consisting of halogen, (C₁-C₆)-alkoxy, (C₁-C₆)-haloalkoxy, CN, NO₂, S(O)ₚR¹², NR¹³R¹⁴, OH and oxo;
or a heterocyclyl or heteroaryl, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
R² and R³ are each independently H or (C₁-C₃)-alkyl; or wherein R² and R³ may form together a (C₁-C₆)-alkylene bridge ;
R⁴ and R⁵ are each independently H, (C₁-C₆)-alkyl, (C₂-C₆)-alkenyl or (C₁-C₆)-alkoxy; wherein in case R⁴ and R⁵ are each independently (C₁-C₆)-alkyl, (C₂-C₆)-alkenyl or (C₁-C₆)-alkoxy both groups together may form a 4-7 membered ring with the carbon atom in position 4 (C-4) of the piperidine ring; and
wherein the residues may be unsubstituted or substituted by one or more radicals selected from the group consisting of halogen, (C₁₋C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴;
or in case R⁴ is H or (C₁-C₆)-alkyl, R⁵ may be also OH, OCOR⁸, OCOOR⁹, OCO-COO(C₁-C₄)-alkyl, COO(C₁-C₄)-alkyl , COOH, CH₂Phenyl,
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴;
or in case R⁵ is a binding electron pair, R⁴ may form together with R⁵ a residue X selected from the group consisting of O, S, N-OH, N-O-(C₁₋C₆)-alkyl, N-O-CO-R⁸, N-O-COOR⁹
R⁷ is H, (C₁-C₃)-alkyl, (C₂-C₄)-alkenyl ;
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or wherein the residues
R⁷ and R⁵ and the carbon atoms in position 3 (C-3) and in position 4 (C-4) of the piperidin ring form a (C₃-C₇)-cycloalkyl, (C₃-C₇)-cycloalkenyl or a (C₆-C₁₄)-aryl residue, either unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
R⁸ is H, (C₂-C₆)-alkenyl, (C₂-C₆)-alkynyl, (C₃-C₇)-cycloalkyl or (C₁-C₆)-alkyl, unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴ ;
R⁹ is H, (C₃-C₆)-alkenyl, (C₃-C₆)-alkynyl, (C₃-C₇)-cycloalkyl or (C₁-C₆)-alkyl, unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴
R¹⁰ is (C₆-C₁₄)-aryl, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴
R¹¹ is a saturated or unsaturated heteroaromatic or heterocyclyl radical, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
R¹² is (C₁-C₆)-alkyl or (C₁-C₆)-haloalkyl
R¹³ and R¹⁴ are each independently H, (C₁-C₆)-alkyl, (C₁-C₆)-haloalkyl, (C₂₋C₆)-alkenyl, (C₂-C₆)-alkynyl, (C₃-C₇)-cycloalkyl or (C₃-C₇)-cycloalkyl-(C₁₋C₆)-alkyl; or a group wherein R¹³ and R¹⁴ together with the N form a 4 to 8-membered heteroaryl or heterocyclyl ring that may contain one or two further hetroatoms;
and wherein
n is 0 or 1 and p is 0, 1 or 2.

2. The use of compounds as claimed in claim 1 wherein piperidin derivatives of formula (Ia), (Ib) and (Ic): 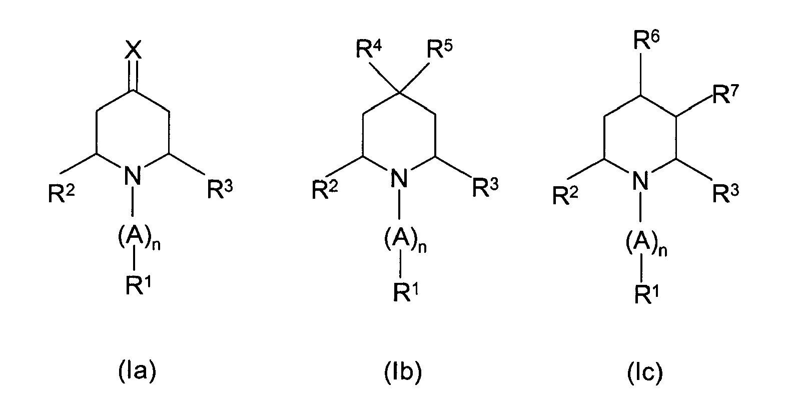 wherein:
A is an unbranched or branched (C₁-C₃)-alkylene and/or
R¹ is phenyl, unsubstituted or substituted
by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or is (C₁-C₆)-alkyl, (C₁-C₆)-haloalkyl, (C₃-C₇)-cycloalkyl, (C₃₋C₇)-cycloalkylenyl;
or is heteroaryl e.g. pyridine, pyrimidine, pyrazine and pyridazine,
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
and/or
R², R³ are each independently H, (C₁-C₃)-alkyl; wherein R² and R³ may form together a (C₁-C₃)-alkylene bridge, in particular a -C₂H₄- group so that a tropan ring system is generated;
and/or in formula (Ib)
R⁴, R⁵ are each independently H, (C₁-C₆)-alkyl, (C₁-C₆)-alkoxy; or in case R⁴ and R⁵ are (C₁-C₆)-alkyl or (C₁-C₆)-alkoxy both groups together with C-4 of the piperidine ring may form a 4-7 membered ring;
and/or in formula (Ic)
R⁶ is OH, OCOR⁸, OCOOR⁹, OCO-COO(C₁-C₄)-alkyl, COO(C₁-C₄)-alkyl , COOH or CH₂Phenyl,
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴
and/or
R⁷ is H, (C₁-C₃)-alkyl,
or wherein
R⁷ and R⁶ and the carbon atoms in position 3 (C-3) and 4 (C-4) of the piperidin ring form together a 5 to 7 -membered cycloalkyl or cycloalkenyl ring or a 6 to 14 membered aromatic ring, either unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴;
and/or in formula (Ia)
X is O, S, N-OH, N-O-(C₁-C₆)-alkyl, N-O-CO-R⁸, N-O-COOR⁹
and wherein
R⁸ is H, (C₂-C₆)-alkenyl, (C₂-C₆)-haloalkenyl, (C₂-C₆)-alkynyl, (C₂₋C₆)-haloalkynyl, (C₃-C₇)-cycloalkyl or (C₁-C₆)-alkyl which last mentioned group is unsubstituted or substituted by one or more radicals selected from the group consisting of halogen, (C₃-C₇)-cycloalkyl, (C₁-C₆)-alkoxy, (C₁-C₆)-alkylthio, OH, CN, NO₂, R¹⁰, R¹¹, S(O)ₚR¹² and NR¹³R¹⁴ ;
R⁹ is H, (C₃-C₆)-alkenyl, (C₃-C₆)-haloalkenyl, (C₃-C₆)-alkynyl, (C₃₋C₆)-haloalkynyl, (C₃-C₇)-cycloalkyl, or (C₁-C₆)-alkyl which last mentioned group is unsubstituted or substituted by one or more radicals selected from the group consisting of halogen, (C₃-C₇)-cycloalkyl, (C₁₋C₆)-alkoxy, (C₁-C₆)-alkylthio, OH, CN, NO₂, R¹⁰, R¹¹, S(O)ₚR¹² and NR¹³R¹⁴ ;
R¹⁰ is phenyl, unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
R¹¹ is a saturated, unsaturated or heteroaromatic heterocyclyl radical unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
R¹² is (C₁-C₆)-alkyl or (C₁-C₆)-haloalkyl
R¹³ and R¹⁴ are each independently H (C₁-C₆)-alkyl, (C₁-C₆)-haloalkyl, (C₂-C₆)-alkenyl, (C₂-C₆)-alkynyl, (C₃-C₇)-cycloalkyl or (C₃-C₇)-cycloalkyl-(C₁-C₆)-alkyl; or R¹³ and R¹⁴ together with the N form a 4 to 8-membered heteroaryl or heterocyclyl ring that may contain one or two further hetroatoms selected from the group consisting ot N, O, S ;
and wherein
n is 0 or 1,
p is 0, 1 or 2,
or a pesticidally acceptable salt thereof, is used for the control of pests.

3. The use of compounds as claimed in claim 1 or 2 or a pesticidally acceptable salt thereof wherein
A is a straight chain or branched (C₁-C₃)-alkylene and
R¹ is phenyl
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴ ;
or is (C₁-C₆)-alkyl, (C₁-C₆)-haloalkyl, (C₃-C₇)-cycloalkyl, (C₃₋C₇)-cycloalkylenyl;
or is pyridine, pyrimidine, pyrazine and pyridazine,
unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹², OH and oxo;
and
R² and R³ are each independently H or CH₃;
or in case R² and R³ are both CH₃ the methyl groups may be connected to form a -C₂H₄- group;
and wherein R¹², R¹³ and R¹⁴ are as defined in claim 1, and wherein n is 0 or 1 and p is 0, 1 or 2.

4. The use of a compound of formula (lb) or a pesticidally acceptable salt thereof as claimed in claim 2 or 3 wherein
R⁴ and R⁵ are each independently H, (C₁-C₆)-alkyl, (C₁-C₆)-alkoxy; or in case R⁴ and R⁵ are (C₁-C₆)-alkyl or (C₁-C₆)-alkoxy both groups together with C-4 of the piperidine ring may form a 4-7 membered ring.

5. The use of a compound of formula (Ic) or a pesticidally acceptable salt thereof as claimed in claim 2 or 3 wherein
R⁶ is OH, OCOR⁸, OCOOR⁹, COO(C₁-C₄)-alkyl ; and
R⁷ is H or
R⁷ together with R⁶ and C-3 and C-4 of the piperidin ring forms a saturated or unsaturated 6-membered ring to generate formula (IIa) and (IIb) and (IIc); 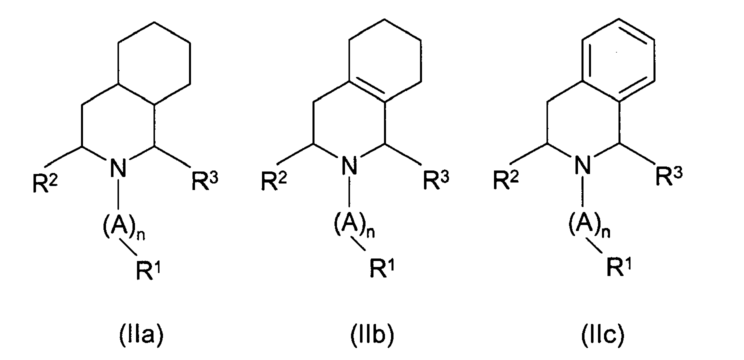 wherein said 6-membered ring is either unsubstituted or substituted by one or more radicals selected from the group consisting of halogen, (C₁-C₆)-alkyl, (C₁-C₆)-haloalkyl, (C₁-C₆)-alkoxy, (C₁-C₆)-haloalkoxy, CN, NO₂, S(O)ₚR¹² and NR¹³R¹⁴.
and wherein R¹, R², R³ and A are as defined in claim 2,
and wherein R⁸, R⁹, R¹², R¹³ and R¹⁴ are as defined in claim 1,
and wherein n is 0 or 1 and p is 0, 1 or 2

6. The use of a compound of formula (Ic) or an pesticidally acceptable salt thereof as claimed in claim 2 or 3 wherein X is O.

7. The use of substituted piperidine derivatives of formula (I) or a pesticidally acceptable salt thereof as claimed in any one of claims 1 to 6 for controlling arthropod pests and/or helminths pests.

8. The use of substituted piperidine derivatives of formula (I) or a pesticidally acceptable salt thereof as claimed in any one of claims 1 to 6 for controlling insects, arachnids and/or nematodes.

9. The use of substituted piperidine derivatives of formula (I) or a pesticidally acceptable salt thereof as claimed in any one of claims 1 to 6 as pesticides which are used as ectoparasiticides in stock animals or in domestic companion animals.

10. The use of a pesticidal composition comprising a compound of formula (I) or a pesticidally acceptable salt thereof as defined in any one of claims 1 to 6, in association with a pesticidally acceptable diluent or carrier and/or surface active agent for controlling pests.

11. The use of substituted piperidine derivatives of formula (I) as claimed in any on of claims 1 to 6 for the preparation of a veterinary medicament.

12. The use of substituted piperidine derivatives of formula (I) as claimed in claim 11 for the preparation of a veterinary medicament for controlling pests.

13. A method for the control of pests at a locus which comprises the application of at least one compound of formula (I) or a salt thereof as claimed in any of claims 1 to 6 or of a composition as claimed in claim 10 or of a veterinary medicament as claimed in claim 11 or 12 for controlling pests.
